(19) Europäisches Patentamt
European Patent Office
Office européen des brevets

(11) EP 3 357 920 A1

(12) **EUROPEAN PATENT APPLICATION**

(43) Date of publication:
**08.08.2018 Bulletin 2018/32**

(21) Application number: **17196453.9**

(22) Date of filing: **16.05.2013**

(51) Int Cl.:
*C07D 403/12* (2006.01)
*A01N 43/50* (2006.01)
*A01N 43/647* (2006.01)
*A01P 1/00* (2006.01)
*C07D 233/72* (2006.01)
*C07D 233/82* (2006.01)
*A01N 33/12* (2006.01)
*A01N 35/02* (2006.01)
*A01N 59/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2012 US 201261648167 P**
**04.03.2013 US 201361772440 P**

(62) Document number(s) of the earlier application(s) in accordance with Art. 76 EPC:
**13793373.5 / 2 850 077**

(71) Applicant: **Exigence Technologies Inc.**
**Winnipeg, Manitoba R3T 6A8 (CA)**

(72) Inventor: **LIU, Song**
**Winnipeg, Manitoba R3T 5V4 (CA)**

(74) Representative: **HGF Limited**
**8th Floor**
**140 London Wall**
**London EC2Y 5DN (GB)**

Remarks:
This application was filed on 13-10-2017 as a divisional application to the application mentioned under INID code 62.

# (54) BIOCIDAL COMPOUNDS AND METHODS FOR USING SAME

(57) Biocidally active cationic analogs of N-halamine having two biocidally active groups covalently bonded together in a single molecule and having general Formula (I). Compounds of Formula (I), and precursors thereof, can be in solution form immobilized onto a substrate via physical coating or covalent chemical bonding to functionalize surfaces or added into materials as additives so as to render them biocidal. The biocidal solutions and substrates comprising the compounds or precursors of the present invention can then be used to inactivate pathogenic microorganisms. N-halamine-L-QUAT (I) wherein: the N-halamine may be a cyclic or acyclic N-halamine; L is $C_1$-$C_6$ alkyl, cyclic aromatic or nonaromatic ring, ether, ketone or any other organic linking structures, and QUAT has general formula (II):

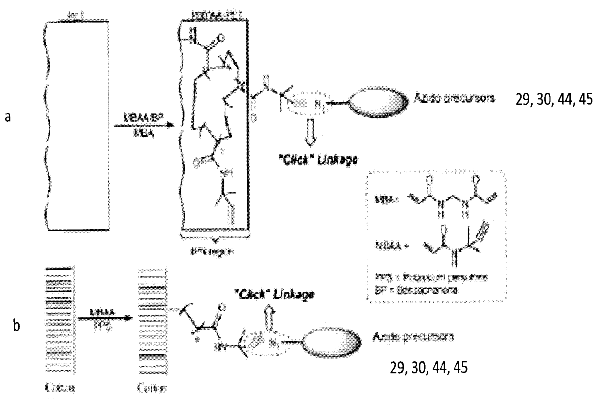


Fig. 1

EP 3 357 920 A1

## Description

### FIELD OF THE INVENTION

The present disclosure relates to the field of biocides and, in particular, to cationic analogs of N-halamine having biocidal activity. The cationic analogs of N-halamine according to the present disclosure, comprise two biocidally active groups covalently bonded together in a single molecule. The present disclosure further relates to compositions comprising the cationic analogs of N-halamine and methods for using these compounds and compositions as biocidal agents.

### BACKGROUND OF THE INVENTION

Biocidal compounds continue to be investigated in an effort to contain and control the spread of infectious pathogens in a variety of health and industrial applications. To this end, broad-spectrum biocides have been developed for use in solution form as well as to incorporate biocidal activity into materials and coatings. Two major categories of compounds that have been investigated are the quaternary ammonium compounds (QACs) and N-halamines.

N-halamines are inorganic and organic compounds in which oxidative halogen is chemically bonded to nitrogen. The nitrogen-halogen bond is formed by reaction of an amine, imine, amide, or imide with halogen, hypohalous acid, or hypochlorite. The mechanism by which these N-halamine compounds inactivate pathogenic microorganisms is through direct contact. For example, kill of bacteria by N-chloramines occurs by two mechanisms. One is based on release of free chlorine and another on direct transfer of chlorine to biological receptors. Chlorine can be transferred from polar N-Cl bond to water, generating chlorine in the "+1" oxidation state as hypochlorous acid or hypochlorite anion. In the second mode of action, chlorine is directly transferred to biological receptors to form a thermodynamically more stable species. Using a model study to explore the antibacterial mechanism of one typical N-chloramine, it has been concluded that the disinfecting action of 3-chloro-4,4-dimethyl-2-oxazolidinone against *S. aureus* actually was the result of the interaction of the whole N-chloramine molecule with the bacterium instead of the limited amount of dissociated free chlorine (Worley et al. App Environ Microbiol 54 (1988) 2583-5). As a result, the major biocidal mechanism for N-chloramine is believed to be through chlorine transfer. Once the halogen is depleted, N-halamines have the ability to be regenerated. Covalent attachment of N-halamine moieties to insoluble polymers have also been investigated to create biocidal materials and coatings.

Quaternary ammonium cations, also known as quaternary ammonium salts, quaternary ammonium compounds or "quats", are ammonium compounds in which four organic groups are linked to a nitrogen atom that produces a positively charged ion (cation) of the structure $NR_4^+$ with R being alkyl groups. Quaternary ammonium compounds have also been shown to have broad-spectrum antimicrobial activity, in particular, quaternary ammonium compounds containing at least one R group having a chain length in the range C8 to C18. The bactericidal action of quaternary compounds differs from the N-halamines. The mode of action of quaternary ammonium compounds has been attributed to inactivation of energy-producing enzymes, denaturation of proteins, and disruption of the cell membrane. Quaternary ammonium compounds have been found to be weakly biocidal. As with N-halamines, attachment of quaternary ammonium functional groups to polymers has been investigated to utilize these biocidal compounds in surface active applications.

Demands for biocidal performance have led to the combination of N-halamine and quaternary ammonium compounds into copolymers. For example, International Patent Publication No. WO2007/120173 describes a copolymer having pendant hydantoin groups and pendant quaternary ammonium groups randomly attached to a polysiloxane copolymer backbone. By attaching a specific fraction of quaternary ammonium groups to the polysiloxane backbone, it is described that the typically water insoluble polysiloxane N-halamine polymer, is rendered water soluble.

Increasing demands on biocidal performance and increasing bacterial resistance to existing biocidal compounds necessitate a continuous effort in searching for new and powerful biocides.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

Exemplary embodiments of the present disclosure pertain to biocidal compounds, compositions, and uses thereof. In accordance with one aspect, the present disclosure relates to a biocidal compound having general formula (I):

N-halamine-L-QUAT (I)

wherein:

the N-halamine may be a cyclic or acyclic N-halamine;

L is $C_1$-$C_6$ alkyl, cyclic aromatic or non-aromatic ring,

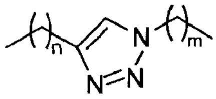
,

ether, ketone or any other organic linking structures, and

QUAT has general formula (II):

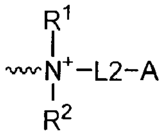
(II)

wherein:

$R^1$ and $R^2$ are each independently $C_1$-$C_6$ alkyl;
L2 is absent, $C_1$-$C_6$ alkyl or

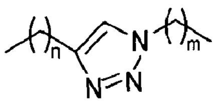
;

A is $R^3$, N-halamine or $-N^+R^4R^5R^6$;
$R^3$ is $C_1$-$C_{18}$ alkyl;
$R^4$ and $R^5$ are each independently $C_1$-$C_6$ alkyl;
$R^6$ is $C_1$-$C_{18}$ alkyl or $-(CH_2)_pB$;
B is N-halamine;
n and m are each independently 1-6, and
p is 1-6,

and wherein:

when A is $R^3$, L2 is absent, and

when A is N-halamine or $-N^+R^4R^5R^6$, L2 is $C_1$-$C_6$ alkyl or

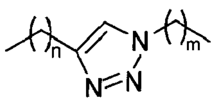
.

In accordance with another aspect, the present disclosure relates to a compound having general formula (VI):

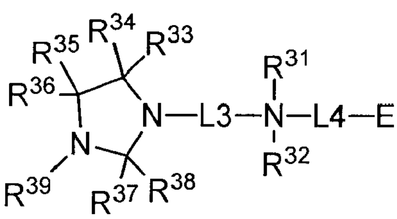
(VI)

wherein:

L3 is $C_1$-$C_6$ alkyl;
$R^{31}$ and $R^{32}$ are each independently $C_1$-$C_6$ alkyl;
L4 is absent, $C_1$-$C_6$ alkyl or

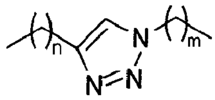

;

E is $R^{40}$, $-N^+R^{41}R^{42}R^{43}$, or N-halamine of general formula (V), wherein general formula V is:

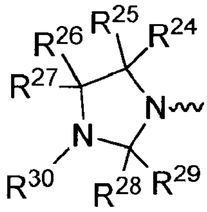

(V)

wherein:

$R^{24}$ and $R^{25}$ are each independently H, $C_1$-$C_4$ alkyl, or $C_1$-$C_4$ alkoxy, or $R^{24}$ and $R^{25}$ taken together form =O;
$R^{26}$ and $R^{27}$ are each independently H, $C_1$-$C_4$ alkyl, or $C_1$-$C_4$ alkoxy, or $R^{26}$ and $R^{27}$ taken together form =O;
$R^{28}$ and $R^{29}$ are each independently H, $C_1$-$C_4$ alkyl, or $C_1$-$C_4$ alkoxy, or
$R^{28}$ and $R^{29}$ taken together form =O, and
$R^{30}$ is halo,
and wherein:

when $R^{24}$ and $R^{25}$ taken together form =O, $R^{26}$ and $R^{27}$ are each independently H, $C_1$-$C_4$ alkyl, or $C_1$-$C_4$ alkoxy;

$R^{40}$ is $C_1$-$C_{18}$ alkyl;
$R^{41}$ and $R^{42}$ are each independently $C_1$-$C_6$ alkyl;
$R^{43}$ is $C_1$-$C_{18}$ alkyl or $-(CH_2)_pM$;
M is N-halamine of general formula (V);
n and m are each independently 1-6, and
p is 1-6,
$R^{33}$ and $R^{34}$ are each independently H, $C_1$-$C_4$ alkyl, or $C_1$-$C_4$ alkoxy, or $R^{33}$ and $R^{34}$ taken together form =O;
$R^{35}$ and $R^{36}$ are each independently H, $C_1$-$C_4$ alkyl, or $C_1$-$C_4$ alkoxy, or $R^{35}$ and $R^{36}$ taken together form =O;
$R^{37}$ and $R^{38}$ are each independently H, $C_1$-$C_4$ alkyl, or $C_1$-$C_4$ alkoxy, or $R^{37}$ and $R^{38}$ taken together form =O, and
$R^{39}$ is halogen,

wherein:

when E is $R^{40}$, L4 is absent, and
when E is N-halamine of general formula (V) or $-N^+R^{41}R^{42}R^{43}$, L4 is $C_1$-$C_6$ alkyl or

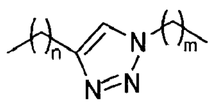

,

and wherein:
when $R^{33}$ and $R^{34}$ taken together form =O, $R^{35}$ and $R^{36}$ are each independently H, $C_1$-$C_4$ alkyl, or $C_1$-$C_4$ alkoxy.

In accordance with another aspect, the present disclosure relates to a precursor of the biocidal compound

having general Formula I, wherein each halogen substituent in each N-halamine moiety is replaced with a hydrogen substituent, and wherein halogenation of said substituent results in the biocidally activity compound.

In accordance with another aspect, the present disclosure relates to a composition comprising the compound having general Formula I or a precursor thereof.

In accordance with another aspect, the present disclosure relates to a use of a compound having general Formula I, or a precursor thereof, as a disinfectant.

## BRIEF DESCRIPTION OF THE DRAWINGS

These and other features of the invention will become more apparent in the following detailed description in which reference is made to the appended drawings.

**Figure 1** is a schematic representation of the immobilization of azido-derivatives via "click" reaction onto the surface of a substrate, (a) PET and (b) cotton, according to embodiments of the present disclosure;

**Figure 2** is an ATR spectrum of (a) PMBAA-g-cotton (percentage graft = 1.03%), (b) untreated cotton, according to embodiments of the present disclosure;

**Figure 3** is a visualization of PMBAA-g-cotton-ADNS under UV light (365 nm); (a) and (c) are control samples, (b) and (d) are "clicked" samples (magnification of images: (a,b) 40x, (c,d) 100x, according to embodiments of the present disclosure; and

**Figure 4** is a schematic representation of boosting microbiocidal function between cation and N-chloramine, according to embodiments of the present disclosure.

## DETAILED DESCRIPTION OF THE INVENTION

The present disclosure relates to cationic analogs of N-halamine having biocidal activity. The cationic analogs of N-halamine according to the present disclosure, comprise two biocidally active groups covalently bonded together in a single molecule. In this way, embodiments of the present disclosure relate to compounds exhibiting a biocidal activity resulting from the combined effect of two biocidally active groups.

The biocidally active groups comprise both structural cationic and N-halamine moieties covalently bonded together. The cationic moiety of the N-halamine analog may comprise a quaternary ammonium cation. In certain embodiments, the N-halamine moiety may comprise an acyclic N-halamine or a cyclic N-halamine. In further exemplary embodiments, the N-halamine moiety is a cyclic N-halamine comprising general formula (I). According to preferred embodiments, the cationic analogs of N-halamine are cationic analogs of halogenated hydantoin having biocidal activity.

In some embodiments, the biocidal activity of the analogs is enhanced by the covalently bonded cationic moiety. This enhanced biocidal activity may be additive in some embodiments. In other embodiments, the covalently bonded cationic and N-halamine moieties produce a synergistic biocidal activity.

The compounds, according to embodiments of the present disclosure, are water soluble and provide biocidal activity in solution form. In other embodiments, the compounds can be immobilized onto a substrate. In this way, compounds of the present disclosure offer versatility in use. In certain exemplary embodiments, the compounds of the present disclosure may be covalently bonded to a substrate to provide covalent immobilization.

According to embodiments of the present disclosure, the biocidal activity of the compounds of the present disclosure is regenerable. Biocidal activity of the compounds resulting from a halogen exchange reaction upon contact with a microorganism, according to some embodiments, results in consumption of halogens. The consumed halogens may be regenerated by halogen treatment. In this regard, compounds according to embodiments of the present disclosure are rechargeable.

The present disclosure further relates to compositions comprising the compounds of the present disclosure. Such compositions may comprise one or more cationic analogs of N-halamine having biocidal activity. In some embodiments, the compositions may be provided in solution form.

Compounds and compositions of the present disclosure can be used in a variety of biocidal treatment methods. In one embodiment, one or more compounds can be used as a surface disinfectant. In other embodiments, one or more compounds can be used for incorporation into polymers to generate regenerable antibacterial coatings or surfaces. Accordingly, it is within the scope of the present disclosure to use one or more compounds of the present disclosure for grafting onto and into various surfaces or materials to provide durable and regenerable antibacterial activity.

In some embodiments, the compounds and compositions of the present disclosure can be activated with less active halogen loadings, and can be activated using dilute halogen treatment solutions.

***Definitions***

Unless defined otherwise, all technical and scientific terms used herein have the same meaning as commonly understood by one of ordinary skill in the art to which this invention belongs.

As used herein, the term "about" refers to an approximately +/-10% variation from a given value. It is to be understood that such a variation is always included in any given value provided herein, whether or not it is specifically referred to.

The term "N-halamine" as used herein refers to a compound containing one or more nitrogen-halogen covalent bonds that is normally formed by the halogenation of imide, amide or amine groups of a compound. The presence of the halogen renders the compound biocidal. N-halamines, as referred to in the present disclosure, include both cyclic and acyclic N-halamine compounds.

The term "halo" or "halogen" by themselves or as part of another substituent, have the same meaning as commonly understood by one of ordinary skill in the art, and preferably refer to chlorine, bromine or iodine atom.

The term "quaternary ammonium cation", "quaternary ammonium compound", "quaternary ammonium salt", "QAC", and "quat" may be used interchangeably throughout the present disclosure to refer to ammonium compounds in which four organic groups are linked to a nitrogen atom that produces a positively charged ion (cation) of the structure $NR_4^+$.

The term "biocide", as used herein, means a chemical compound, a chemical composition, a chemical formulation which can kill or render harmless a microorganism exemplified by bacterium, yeast, and fungi.

As used herein, the term "activity" refers to biocidal activity.

***A. CATIONIC N-HALAMINE COMPOUNDS AND PRECURSORS***

The compounds of the present disclosure have the general formula (I):

N-halamine-L-QUAT (I)

wherein:

the N-halamine may be a cyclic or acyclic N-halamine;

L is $C_1$-$C_6$ alkyl, cyclic aromatic or non-aromatic ring,

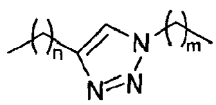
,

ether, ketone or any other organic linking structures, and

QUAT has general formula (II):

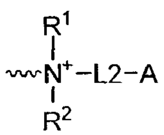
(II)

wherein:

$R^1$ and $R^2$ are each independently $C_1$-$C_6$ alkyl;
L2 is absent, $C_1$-$C_6$ alkyl or

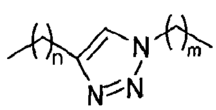
;

A is $R^3$, N-halamine or $-N^+R^4R^5R^6$;
$R^3$ is $C_1$-$C_{18}$ alkyl;
$R^4$ and $R^5$ are each independently $C_1$-$C_6$ alkyl;
$R^6$ is $C_1$-$C_{18}$ alkyl or $-(CH_2)_pB$;
B is N-halamine;
n and m are each independently 1-6, and
p is 1-6,
and wherein
when A is $R^3$, L2 is absent, and
when A is N-halamine or $-N^+R^4R^5R^6$, L2 is $C_1$-$C_6$ alkyl or

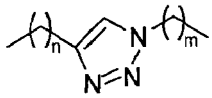

.

In certain embodiments in the compounds of general formula (I), the N-halamine is a cyclic N-halamine.

In certain embodiments in the compounds of general formula (I), each N-halamine is independently a cyclic N-halamine having general formula (III) or general formula (IV):

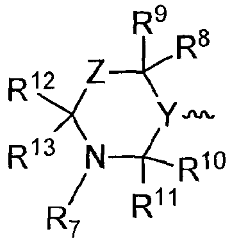

(III)

wherein:

Y is CH or N;
Z is absent, $CH_2$ or $NR^{23}$;
$R^7$ is halo;
$R^8$ and $R^9$ are each independently H, $C_1$-$C_4$ alkyl, or $C_1$-$C_4$ alkoxy, or $R^8$ and $R^9$ taken together form =O;
$R^{10}$ and $R^{11}$ are each independently H, $C_1$-$C_4$ alkyl, or $C_1$-$C_4$ alkoxy, or $R^{10}$ and $R^{11}$ taken together form =O; and
$R^{12}$ and $R^{13}$ are each independently H, $C_1$-$C_4$ alkyl, or $C_1$-$C_4$ alkoxy, or $R^{12}$ and $R^{13}$ taken together form =O, and
$R^{23}$ is H or halo,
wherein when Z is absent and $R^8$ and $R^9$ taken together form =O, $R^{12}$ and $R^{13}$ are each independently H, $C_1$-$C_4$ alkyl, or $C_1$-$C_4$ alkoxy;

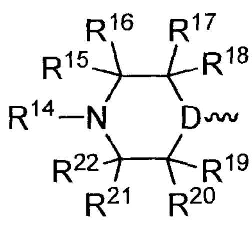

(IV)

wherein:

D is CH or N;
$R^{14}$ is halo;
$R^{15}$ and $R^{16}$ are each independently H, $C_1$-$C_4$ alkyl, or $C_1$-$C_4$ alkoxy, or $R^{15}$ and $R^{16}$ taken together form =O;
$R^{17}$ and $R^{18}$ are each independently H, $C_1$-$C_4$ alkyl, or $C_1$-$C_4$ alkoxy, or $R^{17}$ and $R^{18}$ taken together form =O;
$R^{19}$ and $R^{20}$ are each independently H, $C_1$-$C_4$ alkyl, or $C_1$-$C_4$ alkoxy, or $R^{19}$ and $R^{20}$ taken together form =O, and

$R^{21}$ and $R^{22}$ are each independently H, $C_1$-$C_4$ alkyl, or $C_1$-$C_4$ alkoxy, or $R^{21}$ and $R^{22}$ taken together form =O, wherein when $R^{15}$ and $R^{16}$ taken together form =O, $R^{17}$ and $R^{18}$ are each independently H, $C_1$-$C_4$ alkyl, or $C_1$-$C_4$ alkoxy, and
wherein when $R^{21}$ and $R^{22}$ taken together form =O, $R^{19}$ and $R^{20}$ are each independently H, $C_1$-$C_4$ alkyl, or $C_1$-$C_4$ alkoxy.

In certain embodiments, in the compounds of general formula (I), each N-halamine is a cyclic N-halamine having general formula (IV).

In certain embodiments, in the compounds of general formula (I), each N-halamine is a cyclic N-halamine having general formula (III).

In certain embodiments, in the compounds of general formula (I), each N-halamine is a cyclic N-halamine having general formula (III) wherein:

Y is N, and
Z is absent or $NR^{23}$.

In certain embodiments, in the compounds of general formula (I):

$R^1$ and $R^2$ are each $-CH_3$, and
each N-halamine is a cyclic N-halamine having general formula (III) wherein:

Y is N, and
Z is absent or $NR^{23}$.

In certain embodiments, in the compounds of general formula (I), in which each N-halamine is a cyclic N-halamine of general formula (III), each cyclic N-halamine has general formula (V):

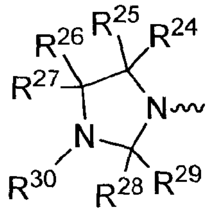
(V)

wherein:

$R^{24}$ and $R^{25}$ are each independently H, $C_1$-$C_4$ alkyl, or $C_1$-$C_4$ alkoxy, or $R^{24}$ and $R^{25}$ taken together form =O;
$R^{26}$ and $R^{27}$ are each independently H, $C_1$-$C_4$ alkyl, or $C_1$-$C_4$ alkoxy, or $R^{26}$ and $R^{27}$ taken together form =O;
$R^{28}$ and $R^{29}$ are each independently H, $C_1$-$C_4$ alkyl, or $C_1$-$C_4$ alkoxy, or $R^{28}$ and $R^{29}$ taken together form =O, and
$R^{30}$ is halo,
and wherein:

when $R^{24}$ and $R^{25}$ taken together form =O, $R^{26}$ and $R^{27}$ are each independently H, $C_1$-$C_4$ alkyl, or $C_1$-$C_4$ alkoxy.

In certain embodiments, in the compounds of general formula (I), in which each N-halamine is a cyclic N-halamine of general formula (III), each cyclic N-halamine has general formula (V):

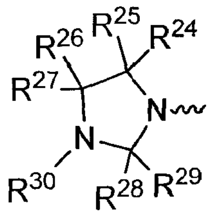
(V)

wherein:

$R^{24}$ and $R^{25}$ are each independently H, $C_1$-$C_4$ alkyl, or $C_1$-$C_4$ alkoxy, or $R^{24}$ and $R^{25}$ taken together form =O;
$R^{26}$ and $R^{27}$ are each independently H, $C_1$-$C_4$ alkyl, or $C_1$-$C_4$ alkoxy, or $R^{26}$ and $R^{27}$ taken together form =O;
$R^{28}$ and $R^{29}$ are each independently H, $C_1$-$C_4$ alkyl, or $C_1$-$C_4$ alkoxy, or $R^{28}$ and $R^{29}$ taken together form =O, and $R^{30}$ is halo,
and wherein:

when $R^{24}$ and $R^{25}$ taken together form =O, $R^{26}$ and $R^{27}$ are each independently H, $C_1$-$C_4$ alkyl, or $C_1$-$C_4$ alkoxy;

and L, in general formula I, is $C_1$-$C_6$ alkyl.

In certain embodiments, the compounds of general formula (I) have general formula (VI):

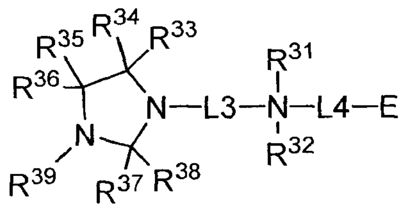

(VI)

wherein:

L3 is $C_1$-$C_6$ alkyl;
$R^{31}$ and $R^{32}$ are each independently $C_1$-$C_6$ alkyl;
L4 is absent, $C_1$-$C_6$ alkyl or

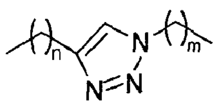

;

E is $R^{40}$, N-halamine of general formula (V) or $-N^+R^{41}R^{42}R^{43}$;
$R^{40}$ is $C_1$-$C_{18}$ alkyl;
$R^{41}$ and $R^{42}$ are each independently $C_1$-$C_6$ alkyl;
$R^{43}$ is $C_1$-$C_{18}$ alkyl or $-(CH_2)_pM$;
M is N-halamine of general formula (V);
n and m are each independently 1-6, and
p is 1-6,
$R^{33}$ and $R^{34}$ are each independently H, $C_1$-$C_4$ alkyl, or $C_1$-$C_4$ alkoxy, or $R^{33}$ and $R^{34}$ taken together form =O;
$R^{35}$ and $R^{36}$ are each independently H, $C_1$-$C_4$ alkyl, or $C_1$-$C_4$ alkoxy, or $R^{35}$ and $R^{36}$ taken together form =O;
$R^{37}$ and $R^{38}$ are each independently H, $C_1$-$C_4$ alkyl, or $C_1$-$C_4$ alkoxy, or $R^{37}$ and $R^{38}$ taken together form =O, and $R^{39}$ is halo,

wherein

when E is $R^{40}$, L4 is absent, and
when E is N-halamine of general formula (V) or $-N^+R^{41}R^{42}R^{43}$, L4 is $C_1$-$C_6$ alkyl or

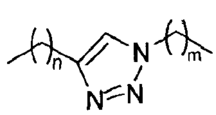

,

and wherein
when $R^{33}$ and $R^{34}$ taken together form =O, $R^{35}$ and $R^{36}$ are each independently II, $C_1$-$C_4$ alkyl, or $C_1$-$C_4$ alkoxy.

In certain embodiments, in any one of general formulae (II), (III), (IV), (V) or (VI), each halo when present is

-Cl or -Br or -I.

In certain embodiments, in any one of general formulae (II), (III), (IV), (V) or (VI), n and m are each independently 1-4.

In certain embodiments, in the compounds of general formula (VI):

$R^{33}$ and $R^{34}$ are each independently H or $C_1$-$C_4$ alkyl, or $R^{33}$ and $R^{34}$ taken together form =O;
$R^{35}$ and $R^{36}$ are each independently H or $C_1$-$C_4$ alkyl, or $R^{35}$ and $R^{36}$ taken together form =O, and
$R^{37}$ and $R^{38}$ are each independently H or $C_1$-$C_4$ alkyl, or $R^{37}$ and $R^{38}$ taken together form =O.

In certain embodiments, in the compounds of general formula (VI):

$R^{31}$ and $R^{32}$, and $R^{41}$ and $R^{42}$ when present, are each $-CH_3$.

In certain embodiments, in the compounds of general formula (VI):

$R^{31}$ and $R^{32}$, and $R^{41}$ and $R^{42}$ when present, are each $-CH_3$;
$R^{33}$ and $R^{34}$ are each independently H or $-CH_3$, or $R^{33}$ and $R^{34}$ taken together form =O;
$R^{35}$ and $R^{36}$ are each independently H or $-CH_3$, or $R^{35}$ and $R^{36}$ taken together form =O, and
$R^{37}$ and $R^{38}$ are each independently H or $-CH_3$, or $R^{37}$ and $R^{38}$ taken together form =O.

In certain embodiments, in the compounds of general formula (VI):

$R^{33}$ and $R^{34}$ taken together form =O;
$R^{35}$ and $R^{36}$ are each independently H or $C_1$-$C_4$ alkyl, and
$R^{37}$ and $R^{38}$ taken together form =O.

In certain embodiments, in the compounds of general formula (VI):

$R^{33}$ and $R^{34}$ are each independently H or $C_1$-$C_4$ alkyl;
$R^{35}$ and $R^{36}$ taken together form =O, and
$R^{37}$ and $R^{38}$ taken together form =O.

In certain embodiments, in the compounds of general formula (VI):

$R^{33}$ and $R^{34}$ are each independently H or $C_1$-$C_4$ alkyl;
$R^{35}$ and $R^{36}$ taken together form =O, and
$R^{37}$ and $R^{38}$ are each independently H or $C_1$-$C_4$ alkyl.

In certain embodiments, in the compounds of general formula (VI):

$R^{33}$ and $R^{34}$ taken together form =O;
$R^{35}$ and $R^{36}$ are each independently H or $C_1$-$C_4$ alkyl, and
$R^{37}$ and $R^{38}$ are each independently H or $C_1$-$C_4$ alkyl.

In certain embodiments, in the compounds of general formula (VI):

$R^{33}$ and $R^{34}$ are each independently H or $C_1$-$C_4$ alkyl;
$R^{35}$ and $R^{36}$ are each independently H or $C_1$-$C_4$ alkyl, and
$R^{37}$ and $R^{38}$ taken together form =O.

In certain embodiments, in any one of the preceding embodiments relating to general formula (VI):

$R^{31}$ and $R^{32}$ are each $-CH_3$.

In certain embodiments, in any one of the preceding embodiments relating to general formula (VI), each halo is -Cl or -Br.

Certain embodiments relate to precursors of the cationic N-halamine compounds defined by Formula I, which may be halogenated in order to produce the above-described cationic N-halamine compounds. Accordingly, certain

embodiments relate to precursor compounds having a structure as set forth in any one of the above-described embodiments in which in each N-halamine moiety, each halo substituent is replaced with a hydrogen substituent.

In certain embodiments, the precursors have a general formula (VII):

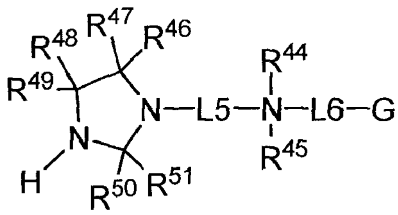

(VII)

wherein:

L5 is $C_1$-$C_6$ alkyl;
$R^{44}$ and $R^{45}$ are each independently $C_1$-$C_6$ alkyl;
L6 is absent, $C_1$-$C_6$ alkyl or

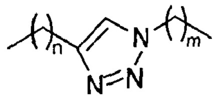

;

G is $R^{52}$, a N-halamine precursor of general formula (V) in which each halo substituent is replaced with a hydrogen substituent, or $-N^+R^{53}R^{54}R^{55}$;
$R^{52}$ is $C_1$-$C_{18}$ alkyl;
$R^{53}$ and $R^{54}$ are each independently $C_1$-$C_6$ alkyl;
$R^{55}$ is $C_1$-$C_{18}$ alkyl or $-(CH_2)_pJ$;
J is a N-halamine precursor of general formula (V) which comprises a hydrogen substituent in place of each halo substituent;
n and m are each 0-6, and
p is 1-6,
$R^{46}$ and $R^{47}$ are each independently H, $C_1$-$C_4$ alkyl, or $C_1$-$C_4$ alkoxy, or $R^{46}$ and $R^{47}$ taken together form =O;
$R^{48}$ and $R^{49}$ are each independently H, $C_1$-$C_4$ alkyl, or $C_1$-$C_4$ alkoxy, or $R^{48}$ and $R^{49}$ taken together form =O;
$R^{50}$ and $R^{51}$ are each independently H, $C_1$-$C_4$ alkyl, or $C_1$-$C_4$ alkoxy, or $R^{50}$ and $R^{51}$ taken together form =O, and

wherein

when G is $R^{52}$, L6 is absent, and
when G is a N-halamine precursor or $-N^+R^{53}R^{54}R^{55}$, L6 is $C_1$-$C_6$ alkyl or

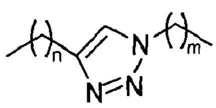

,

and wherein
when $R^{46}$ and $R^{47}$ taken together form =O, $R^{48}$ and $R^{49}$ are each independently H, $C_1$-$C_4$ alkyl, or $C_1$-$C_4$ alkoxy.

In certain embodiments, the compounds or precursors are selected from compounds having general formula (VIII), (IX) or (X):

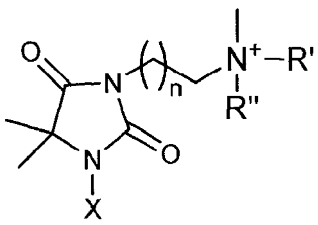

(VIII)

wherein:

X is H, Cl or Br;

n is 1 or 2;

R' is $C_1$-$C_{12}$ alkyl, and

R" is $C_1$-$C_6$ alkyl.

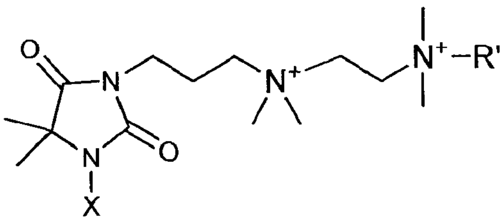

(IX)

wherein:

X is H, Cl or Br, and

R' is $C_1$-$C_{12}$ alkyl.

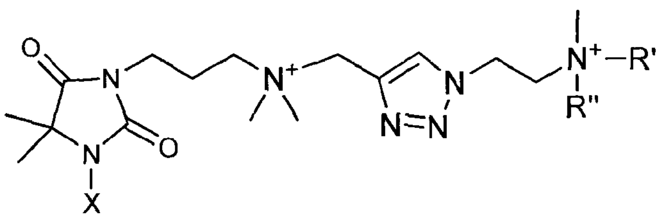

(X)

wherein:

X is H, Cl or Br;

R' is $C_1$-$C_{12}$alkyl, and

R" is $C_1$-$C_6$ alkyl.

In certain embodiments, the compounds or precursors according to any of the preceding embodiments, is derivatized to allow attachment of the compound or precursor to another compound(s), surface, substrate or polymer.

In further embodiments, the compound or precursor of the present disclsoure is derivatized to include an azide moiety or an alkynyl group to allow for attachment to another compound(s), surface, substrate or polymer through "click" chemistry.

In other embodiments, one or more of the alkyl groups attached to the quaternary ammonium centre in any of general formulae (II), (III), (IV), (V), (VI) or (VII), is derivatized to include a terminal azide or alkynyl moiety.

In certain embodiments, the compounds and precursors, or derivatives thereof, are selected from compounds 1 to 42:

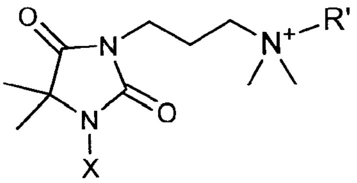

**1** X = H, R' = $CH_3$ **5** X = H, R' = $C_6H_{13}$
**2** X = Cl, R' = $CH_3$ **6** X = Cl, R' = $C_6H_{13}$
**3** X = H, R' = $C_4H_9$ **7** X = H, R' = $C_{12}H_{25}$
**4** X = Cl, R' = $C_4H_9$ **8** X = Cl, R' = $C_{12}H_{25}$

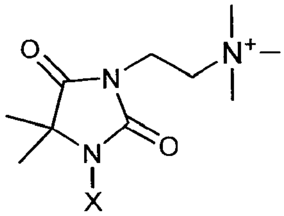

**9** X = H
**10** X = Cl

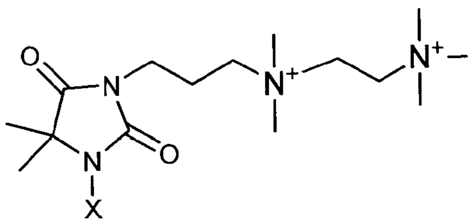

**11** X = H
**12** X = Cl

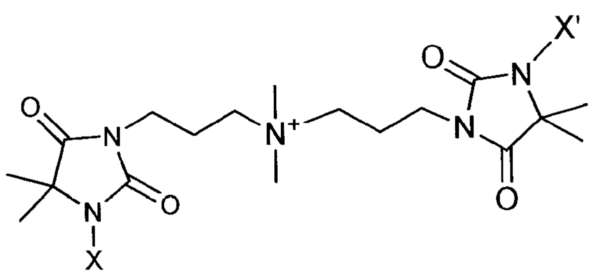

**13** X, X' = H
**14** X, X' = Cl

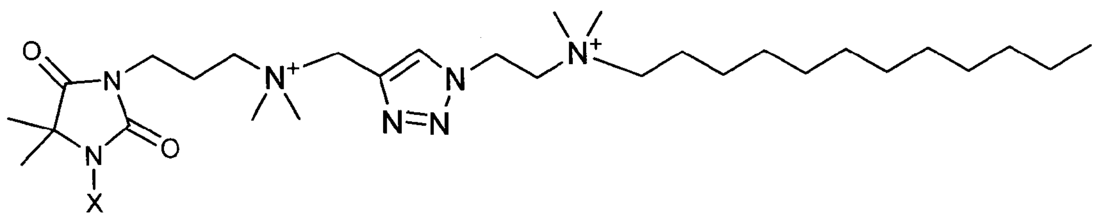

**15** X = H
**16** X = Cl

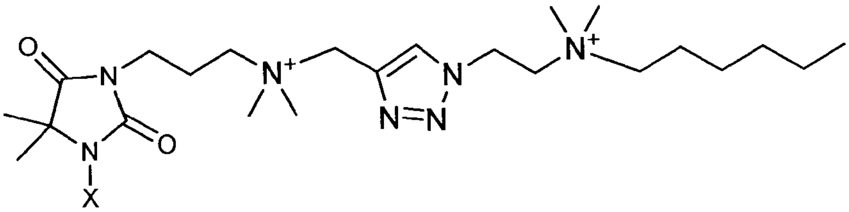

**17** X = H
**18** X = Cl

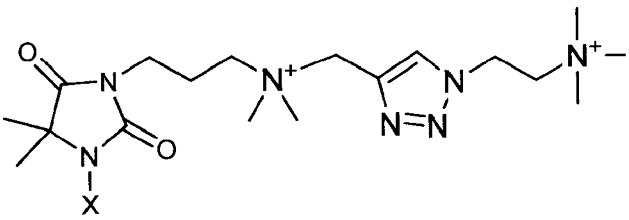

**19** X = H
**20** X = Cl

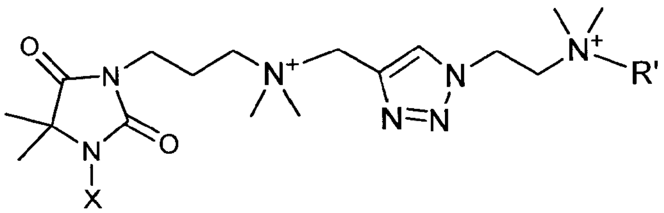

**21** X = H, R' = $C_2H_5$
**22** X = Cl, R' = $C_2H_5$
**23** X = H, R' = $C_4H_9$
**24** X = Cl, R' = $C_4H_9$
**25** X = H, R' = $C_8H_{17}$
**26** X = Cl, R' = $C_8H_{17}$
**37** X = H, R' = $C_6H_{13}$
**38** X = Cl, R' = $C_6H_{13}$

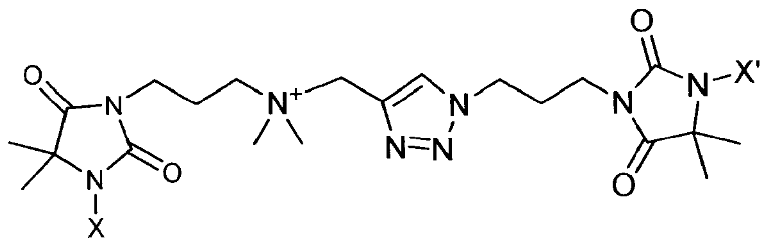

**27** X, X' = H
**28** X, X' = Cl

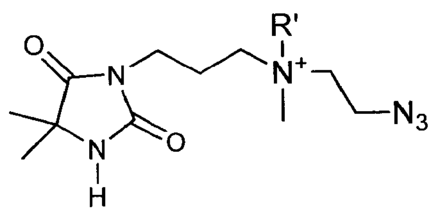

**29** R' = $CH_3$
**30** R' = $C_{12}H_{25}$

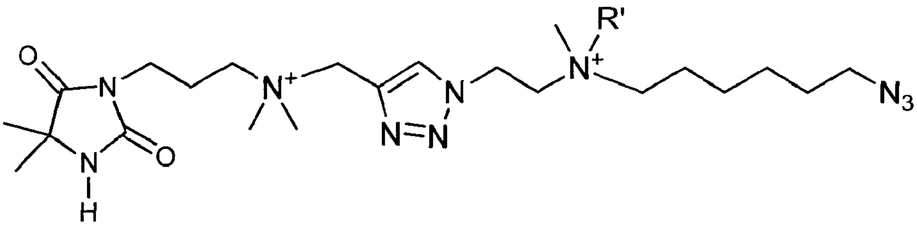

**31** R' = $CH_3$ **36** R' = $C_6H_{13}$

**32** R' = $C_2H_5$ **35** R' = $C_8H_{17}$

**33** R' = $C_4H_9$ **36** R' = $C_{12}H_{25}$

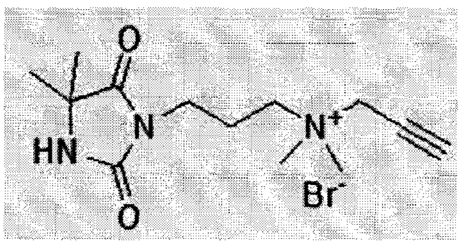

**39**

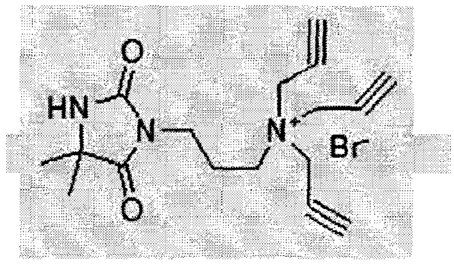

**40**

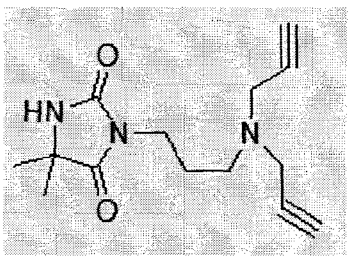

**41**

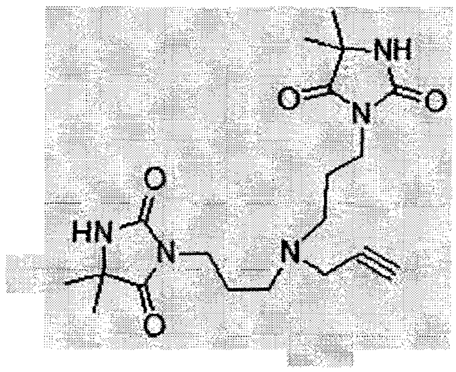

**42**

In certain embodiments, the cationic N-halamine compounds or precursors are in the of form pharmaceutically acceptable salts. The term "pharmaceutically acceptable salt" as used herein, refers to a salt of a compound described herein, which is substantially non-toxic to living organisms. Typical pharmaceutically acceptable salts include those salts prepared by reaction of the compound of the present invention with a pharmaceutically acceptable mineral or organic acid or an organic or inorganic base. Such salts are known as acid addition and base addition salts.

One skilled in the art will understand that the particular counterion forming a part of a pharmaceutically acceptable salt is usually not of a critical nature, so long as the salt as a whole is pharmacologically acceptable and as long as the counterion does not contribute undesired qualities to the salt as a whole. In certain embodiments, the counterion is a halogen ion, for example, $Cl^-$ or $Br^-$.

***B. PREPARATION OF CATIONIC N-HALAMINE COMPOUNDS AND PRECURSORS***

The cationic N-halamine compounds and precursors of the present disclosure can be synthesized by standard techniques known in the art as exemplified in the Examples provided herein. In certain embodiments the synthetic pathways include one or more click chemistry steps.

In certain embodiments, cationic N-chloramine compounds and precursors of the present disclosure can be prepared by reaction of an N-chloramine precursor with a substituted tertiary amine according to the following general synthetic scheme:

a)

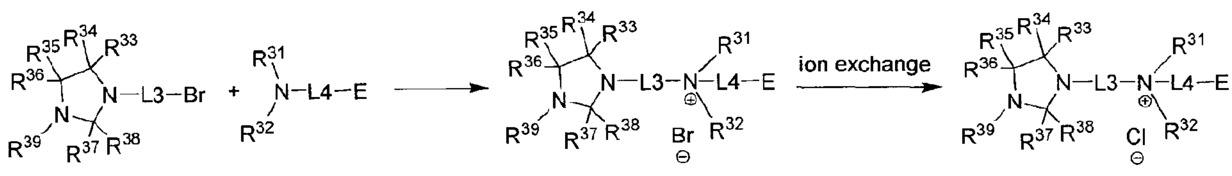


b)

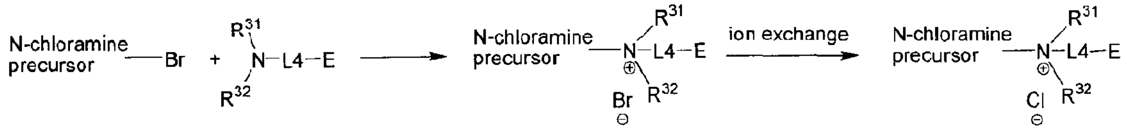


### C. *TESTING BIOCIDAL ACTIVITY OF CATIONIC N-HALAMINE COMPOUNDS*

#### *Biocidal Activity*

As described herein, compounds of Formula I contemplated for use as antimicrobial agents (or biocides), are biocidally active against microorganisms. In addition, in certain embodiments of the present disclosure, the compounds of Formula I may exhibit an enhanced biocidal activity when compared to the biocidal activity of each functional group, i.e., the N-halamine and QUAT, respectively. In further embodiments of the present disclosure, the compounds of Formula I may exhibit an enhanced biocidal activity that is additive of the biocidal activities of each functional group, i.e., the N-halamine and QUAT, respectively. In other embodiments of the present disclosure, the compounds of Formula I may exhibit a synergistic biocidal activity between the covalently bonded functional groups, i.e., the N-halamine and QUAT, respectively.

In further embodiments, the compounds of Formula I may exhibit an improved biodical activity compared to non-ionic or anionic N-halamine-based biocides.

The biocidal activity of a compound of Formula I can be tested using standard techniques known in the art. Similarly, an enhanced biocidal activity the compounds of Formula I can be tested using standard techniques. Exemplary methods of testing compounds of Formula I are provided in the examples included herein. One skilled in the art will understand that other methods of testing the compounds are known in the art and are also suitable for testing compounds of the present disclosure.

Generally, the testing methods comprise exposing a suspension of a selected bacterial strain to the compound or composition for a chosen period of time (for example, between about 1 and 90 mins.) and determining percentage bacterial reduction using standard plating techniques.

All microorganisms susceptible to disinfection by free halogen, e.g., free chlorine, or combined halogen, e.g., N-haloimidazolidinones, N-halohydantoins, N-halooxazolidinones, N-haloisocyanurates, etc., will also be susceptible to disinfection by the biocidal compounds of the present disclosure. Such microorganisms include, for example, bacteria, protozoa, fungi, viruses, and algae. For example, the cationic N-halamine compounds of the present disclosure may be biocidally active against such as the bacteria genera Staphylococcus, Pseudomonas, Escherichia, Salmonella, Shigella, Legionella, Methylobacterium, Klebsiella, and Bacillus; the fungi genera Candida, Rhodoturula, and molds such as mildew; the protozoa genera Giardia, Entamoeba, and Cryptosporidium; the viruses poliovirus, rotavirus, HIV, and herpesvirus; and the algae genera Anabaena, Oscillatoria, and Chlorella. In certain embodiments, the biocidal compounds of the present disclosure may be biocidally active against antibiotic resistent strains of microorganisms.

#### *Efficiency of Halogenation/Activation*

As described herein, cationic N-halamine compounds of the present disclosure become biocidally ineffective due to inactivation of the N-halamine functional group. According to embodiments of the present disclosure, the N-halamine functional group can be recharged or regenerated by treatment with a halogen solution. In other embodiments, the present disclosure contemplates the use of the cationic N-halamine compounds within compositions. In particular, embodiments of the present disclosure include immobilizing inactive precursors of the cationic N-halamine compounds onto the surface of a substrate to be activated with a halogen treatment solution.

In some applications, it may be desirable to be able to activate biocidal compounds with a low concentration of halogen in order to minimize any environmental or toxic effects that may result from the halogenation treatment. In

certain embodiments, the biocidal activity of the compounds of Formula I can be activated using dilute halogenating solutions. In other embodiments, the biocidal activity of the compounds of Formula I can be activated using halogenating solutions with relatively low available chlorine concentration. In certain embodiments, the concentration of available chlorine can be from about 10 ppm to about 300 ppm.

In accordance with some embodiments, a higher amount of active chlorine loading can be achieved on surfaces immobilized with the compounds of Formula I than with similar nonionic N-halamine compounds that have been activated using a dilute halogenating solution (i.e., having relatively low available halogen concentrations, for example, about 10 to 300 ppm available halogen). In further embodiments, the biocidal activity of the compounds of Formula I can be activated at a lower active halogen loading than similar nonionic N-halamine compounds. In other words, in certain embodiments, surfaces immobilized with the compounds of Formula I can exhibit more potent antimicrobial activity than surfaces immobilized with similar nonionic N-halamine compounds having the same active halogen loading level. In other embodiments, the rate of halogenation and activation of the compounds of Formula I can be faster than similar nonionic or anionic N-halamine compounds.

The efficiency of halogenation activation can be tested using standard techniques known in the art. Exemplary methods of testing the efficiency of halogenation are provided in the examples included herein. One skilled in the art will understand that other methods of testing the compounds are known in the art and are also suitable for testing compounds of the present disclosure.

### *D. USES OF CATIONIC N-HALAMINE COMPOUNDS AND PRECURSORS*

The cationic N-halamine compounds and precursors according to the present disclosure can be used as a biocide in a variety of applications. For example, in water treatment applications, food applications, medicine and healthcare, and the like.

In some embodiments, the cationic N-halamine compounds and precursors can be used in solution form as a surface disinfectant. In other embodiments, the cationic N-halamine compounds and/or precursors of the present disclosure can be used as a biocidal treatment in disinfectant applications. In further embodiments, the cationic N-halamine compounds and precursors can be attached or inserted onto a polymer backbone for use as antimicrobial polymers. In this way, the cationic N-halamine compounds and precursors of the present disclosure can be used to biofunctionalize a substrate, thereby, inhibiting or reducing the ability for a microorganism to grow on the surface of the substrate. In some embodiments, the cationic N-halamine compounds and precursors of the present disclosure can be immobilized onto a substrate via physical coating or covalent chemical bonding to functionalize surfaces, or added into materials as additives so as to render them biocidal.

In one embodiment, for example, precursor biocides of the present disclosure can be incorporated into the shell or core of thermoplastic fibers (such as polypropylene and polyester) that are spun using fiber spinning techniques known in the art. The precursor biocides that are incorporated in the shell or core fibers can then be chlorinated to activate the antibacterial activity on the surfaces of the so-formed fibers.

In certain embodiments, the biocidal activity of the cationic N-halamine compounds and/or precursors of the present disclosure, is reversible by the reversible chlorination and de-chlorination of the compounds and/or precursors. In this way, certain embodiments include the use of the cationic N-halamine compounds and/or precursors of the present disclosure to generate a regenerable antibacterial surface.

Exemplary substrates, to which the cationic N-halamine compounds and/or precursors of the present disclosure may be immobilized to, include protective coverings and materials such as fabrics, films, foams, and the like. In one embodiment, the cationic N-halamine compounds and/or precursors of the present disclosure can be immobilized onto a woven or knit fabric. The woven fabric may comprise naturally occurring fibers exemplified by cotton, hemp, flax, and the like, and mixtures thereof. Alternatively, the woven fabric may comprise synthetic fibers exemplified by polymers comprising PET (polyethylene terephthalate), NOMEX® (NOMEX is a registered trademark of Dr. Pychlau GmbH, Freiburg, Fed. Rep. Germany, KEVLAR® (KEVLAR is a registered trademark of E. I. du Pont de Nemours & Co., Wilmington, DE, USA), and the like, and mixtures thereof. Alternatively, the woven fabric may comprise mixtures of naturally occurring fibers and synthetic fibers.

### *Derivatives of Cationic N-Halamine Compounds and Precursors*

The cationic N-halamine compounds and/or precursors of the present disclosure can be incorporated into a polymeric substrate by chemical grafting techniques known in the art that covalently link the cationic N-halamine compounds and/or precursors to the substrate. One strategy for immobilizing cationic N-halamine compounds and/or precursors of the present disclosure onto the surface of a chemically inert polymeric substrate is by using "click" chemistry in which azide molecules can be "clicked" onto alkynyl-presenting ("clickable") handles on the polymeric substrate to introduce biofunctionality (see, for example, Li et al., Polymer 53 (2012) 67-78).

In a similar way, compounds and/or precursors of the present disclosure can be attached to other compounds by using "click" chemistry to create further analogs. In one embodiment, compounds and/or precursors of the present disclosure can be "clicked" onto one or more compounds to create branched analogs (see for example, Example 23).

Certain embodiments relate to cationic N-halamine compounds or precursors as described above that have been derivatized to allow attachment of the cationic N-halamine compound or precursor to another compound, surface, substrate or polymer. In accordance with one embodiment, the cationic N-halamine compounds or precursors are modified to introduce one or more azido groups to allow attachment of the cationic N-halamine compound or precursor to another compound(s), surface, substrate or polymer.

In some embodiments the cationic N-halamine compounds or precursors are derivatized to include one or more azide moieties or one or more alkynyl groups to allow for attachment to one or more compound, surface, substrate or polymer through "click" chemistry. In this way, the cationic N-halamine compounds or precursors of the present disclosure can be made "clickable" onto the surface of a substrate or 'clickable" to one or more compounds. Accordingly, in any of general formulae (II), (III), (IV), (V), (VI), (VII), (VIII), (IX), or (X) above, one or more of the alkyl groups attached to the quaternary ammonium centre may be derivatized to include a terminal azide or alkynyl moiety by standard techniques known in the art. In one embodiment, one or more of the alkyl groups attached to the quaternary ammonium centre, in a cationic N-halamine compound or precursor having the general formulae (II), (III), (IV), (V), (VI), (VII), (VIII), (IX), or (X) above, is derivatized to include a terminal azide moiety. In other embodiments, one or more of the alkyl groups attached to the quaternary ammonium centre, in a cationic N-halamine compound or precursor having the general formulae (II), (III), (IV), (V), (VI), (VII), (VIII), (IX), or (X) above, is derivatized to include a terminal alkynyl moiety.

In certain embodiments, derivitives of the cationic N-halamine compounds and precursors of the present disclosure are selected from:

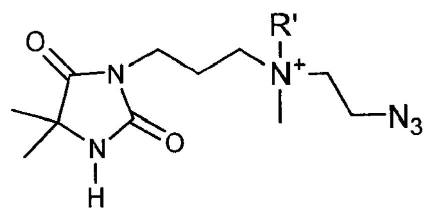

**29** R' = $CH_3$
**30** R' = $C_{12}H_{25}$

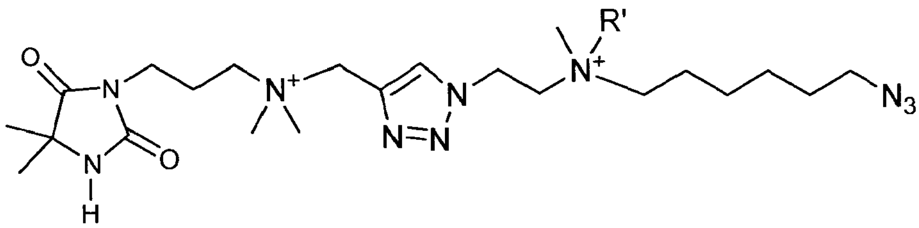

**31** R' = $CH_3$    **36** R' = $C_6H_{13}$
**32** R' = $C_2H_5$    **35** R' = $C_8H_{17}$
**33** R' = $C_4H_9$    **36** R' = $C_{12}H_{25}$

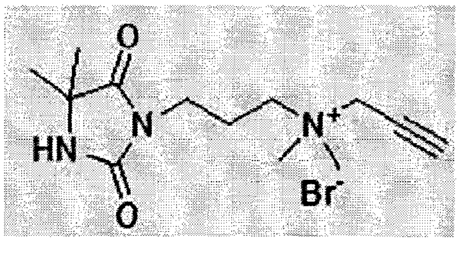

**39**

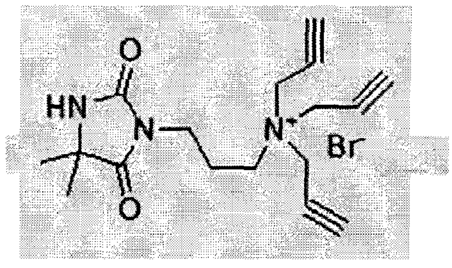

**40**

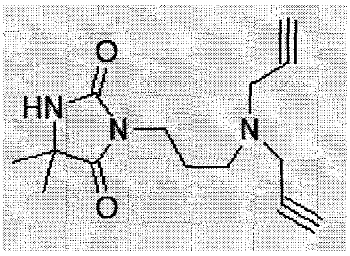

41

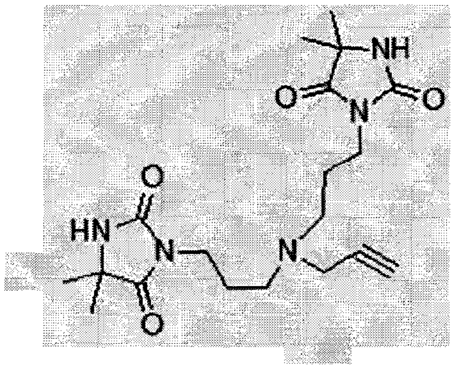

42

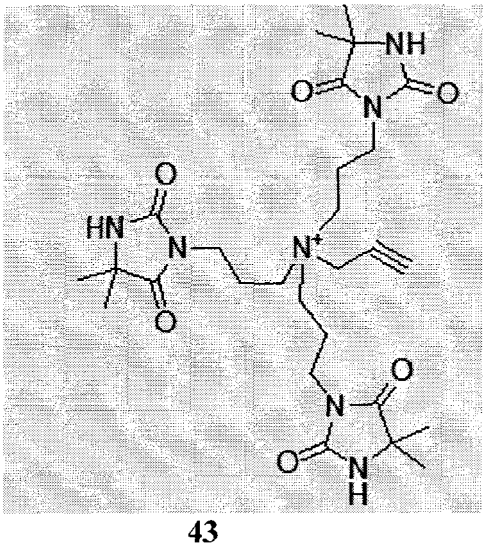

43

***Preparation of Derivatives of Cationic N-Halamine Compounds and Precursors***

Chemical modification of the cationic N-halamine compounds or precursors of the present disclosure to introduce an azido or alkynyl group can be achieved by several general synthetic methods known in the art.

In some embodiments, the N-halamine or unhalogenated precursor thereof is a terminal moiety of the azido-derivitive. In a further embodiment, the cationic centre bridges the two terminal functional groups of the azido-derivative, i.e., the N-halamine, or unhalogenated precursor thereof, and the azide group.

In other embodiments, the N-halamine or unhalogenated precursor thereof is a terminal moiety of the alkynyl-derivitive. In a further embodiment, the cationic centre bridges the two terminal functional groups of the alkynyl-derivative, i.e., the N-halamine, or unhalogenated precursor thereof, and the alkynyl group.

***Immobilization of Derivatives onto Substrates***

The derivatives of the present disclosure are attachable to a substrate surface. In some embodiments, the derivatives comprise an azido or an alkynyl group that undergoes a "click" linkage reaction with a corresponding alkynyl or azido handle presented on the substrate surface. In such embodiments, the substrate surface may be modified using methods known in the art (see, for example, Li et al., Polymer 53 (2012) 67-78) to create a substrate platform comprising alkynyl or azido -presenting ("clickable") handles. In one embodiment, the substrate platform may be modified to comprise alkynyl-presenting handles.

As is known in the art, a substrate platform comprising alykynyl-presenting handles may be created by forming an interpenetrating network on the surface of the substrate. For example, the substrate may be a semicrystalline thermoplastic polymeric substrate, such as PET, or a natural fiber, such as cotton. According to known methods, the monomer N-(2-methylbut-3-yn-2-yl)acrylamide (MBAA) can be co-polymerized with N,N'-methyl-enebisacrylamide (MBA, crosslinker) in the swollen surface of PET, or the surface of cotton, to form the surface interpentrating network (IPN), leading to a PET substrate bearing alkynyl groups (PMBAA-PET) (**Fig. 1**).

According to embodiments of the present disclosure, the derivitized cationic N-halamine compounds or precursors of the present disclosure can be attached onto the surface of a substrate platform comprising alkynyl or azido -presenting handles. Specifically, according to one embodiment, an azido-derivative of cationic N-halamine compounds or precursors of the present disclosure can be "click" reacted with an alkynyl-presenting substrate to immobilize the cationic N-halamine compounds or precursors thereof to the surface of the substrate (**Fig. 1**).

In some embodiments, an unhalogenated (unactivated) precursor of the present disclosure is attached to the substrate surface and then activated by halogenation of the precursors. Once immobilized onto a surface, therefore, a rechargeable self-disinfecting property can result as the halogenation (biocidal activity) and de-halogenation (bacterial killing) is reversible. Halogenating the immobilized precursors of the present disclosure can be achieved by treatment methods known in the art. For example, by spraying, soaking, immersing, washing, with a halogen solution. In one embodiment, the immobilized precursors can be activated by chlorination, bromination, or iodination. In a further embodiment, biocidal function is activitated by chlorination.

In certain embodiments, immobilized precursors of the present disclosure can be activated using dilute halogenating solutions. For example, a NaClO chlorinating solution may be used to activate precursors of N-chloramine containing compounds of the present disclosure. Suitable concentrations of the halogenating solutions used for activating the immobilized precursors will depend on the treatment time, particular substrate being treated, and the particular precursor. In certain embodiments, the halogenating solution has an available halogen concentration of at least about 2 ppm, 5 ppm, 10 ppm, 25 ppm, 30 ppm, 35 ppm, 40 ppm, 45 ppm, 50 ppm, 75 ppm, 100 ppm, 150 ppm, 200 ppm, 250 ppm, 300 ppm, 350 ppm, 400 ppm, 450 ppm, 500 ppm, 750 ppm, 1000 ppm, 1250 ppm, 1500 ppm, 1750 ppm, 2000 ppm, 2250 ppm, or 2500 ppm.

In certain embodiments, the halogenating solution is an NaClO chlorinating solution having at least about 2 ppm available chlorine, 5 ppm available chlorine, 10 ppm available chlorine, 25 ppm available chlorine, 30 ppm available chlorine, 35 ppm available chlorine, 40 ppm available chlorine, 45 ppm available chlorine, 50 ppm, 500 ppm, 1000 ppm, 1500 pm or 2500 ppm available chlorine.

In order to activate the precursors, the halogenating solutions used must convert the precursor to its activated halogenated form to give sufficient active halogen loading on the surface within a short period of time. In some embodiments, the precursors of the present disclosure can be activated within about 1 min., about 5 mins., about 10 mins., about 15 mins., about 20 mins., about 25 mins., or about 30 mins.

In certain embodiments, the halogenating solution results in an active halogen loading of the precursor-immobilized substrate at relatively low available halogen concentrations. In some embodiments, active halogen loading can be achieved at available halogen concentrations of about10 ppm, 20 ppm, 30 ppm, 40 ppm, 50 ppm, 100 ppm, 75 ppm, 100 ppm, 150 ppm, or 200 ppm.

In one embodiment, the precursor-immobilized substrate can be loaded with active chlorine in the range of about 35 ppm to about 76 ppm using a halogenating solution, for example a NaClO chlorinating solution, having a low available chlorine concentration of about 10 ppm, 25 ppm, 40 ppm, 50 ppm, 100 ppm, 75 ppm, 100 ppm, 150 ppm, or 200 ppm.

It is contemplated that any embodiment discussed herein can be implemented with respect to any method or composition of the invention, and vice versa. Furthermore, compositions and kits of the invention can be used to achieve methods of the invention.

To gain a better understanding of the invention described herein, the following examples are set forth. It will be understood that these examples are intended to describe illustrative embodiments of the invention and are not intended to limit the scope of the invention in any way.

To gain a better understanding of the invention described herein, the following examples are set forth. It will be understood that these examples are intended to describe illustrative embodiments of the invention and are not intended to limit the scope of the invention in any way.

## EXAMPLES

## PREPARATION OF COMPOUNDS:

Exemplary compounds of Formula I have been prepared according to a general scheme exemplified by the synthetic scheme shown below wherein a hydantoin amine is reacted with trimethyl amine:

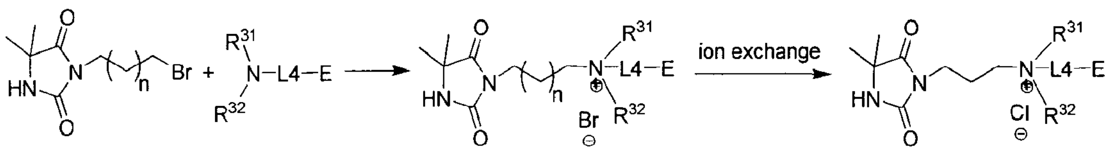

**EXAMPLE 1: PREPARATION OF PRECURSOR 1**

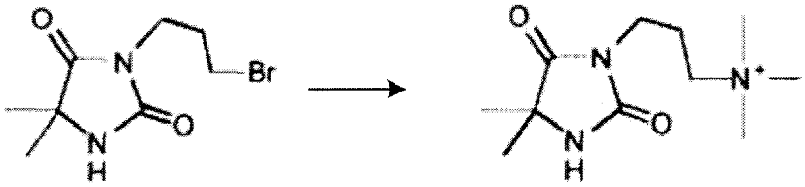

A 1

To the solution of bromide **A** (1.0 g, 4.0 mmol) in EtOH (5mL) was added aqueous dimethylamine (2.2 mL, 24 wt%, 8.0 mmol) at room temperature. The resulting solution was heated to reflux overnight under vacuum. Removal of solvent and excess dimethylamine afforded the bromo-quaternary ammonium salt, which was dissolved in a minimum amount of water and slowly passed through an anion-exchange resin (Amberlite RIRA-900,Cl-) to give **1** as a white solid (Cl- form, 0.94g, 90%).

1: $^1$H NMR ($D_2O$, 300 MHz, $\delta$) 3.61 (t, $J$ = 6.9 Hz, 2 H; --$CH_2CH_2CH_2N^+$), 3.38 (t, $J$ = 8.4 Hz, 2H; -$CH_2CH_2CH_2N^+$), 3.14 (s, 9 H; -$N^+CH_3)_3$), 2.10-2.20 (m, 2H; - $CH_2CH_2CH_2N^+$), 1.44 (s, 6H; $(CH_3)_2C$-); $^{13}$C NMR ($D_2O$, 75 MHz, $\delta$) 185.6 (1'-C=O), 162.1 (3'-C=O), 68.8 (-$CH_2CH_2CH_2N^+$), 64.2 ($CH_3C$-), 57.9 ($N^+CH_3$), 40.4 (-$CH_2CH_2CH_2N^+$), 28.4 ($CH_3$-C), 26.7 (-$CH_2CH_2CH_2N^+$); HRMS (MALDI-TOF) m/z: $[M-Cl]^+$ calcd for $C_{11}H_{22}N_3O_2$, 228.1707; found: 228.1704.

**EXAMPLE 2: PREPARATION OF COMPOUND 2**

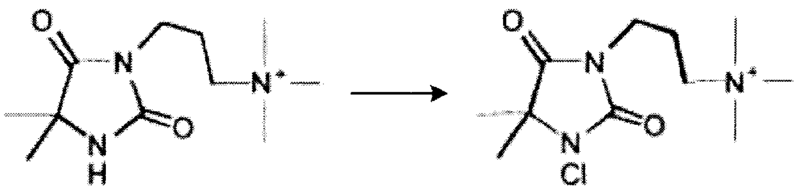

1 2

Precursor **1** was suspended in *t*-BuOH (8 mL) and $H_2O$ (2mL) was subsequently added to make clear solution. Afterwards, excess *t*-butyl hypochlorite (3~4 equiv.) was added to the solution and the mixture was continuously stirred overnight. Removal of excess *t*-butyl hypochlorite and solvent under vacuum afforded the final chlorinated 2 as white solid quantitively.

**2:** $^1$H NMR ($D_2O$, 300 MHz, $\delta$) 3.69 (t, $J$ = 6.9 Hz, 2 H; -$CH_2CH_2CH_2N^+$), 3.43-3.38 (m, 2 H; -$CH_2CH_2CH_2N^+$), 3.15 (s, 9 H; -$N^+CH_3$), 2.22-2.12 (m, 2 H; - $CH_2CH_2CH_2N^+$), 1.51 (s, 6H; $(CH_3)_2C$); $^{13}$C NMR ($CDCl_3$, 75 MHz, $\delta$) 181.8 (1'-C=O), 160.4 (3'-C=O), 71.3 (-$CH_2$ $CH_2CH_2N^+$), 68.7 ($CH_3C$), 58.0 ($N^+CH_3$), 41.6 (-$CH_2CH_2CH_2N^+$), 26.6 ($CH_3$-C), 25.9 (-$CH_2CH_2CH_2N^+$); HRMS (MALDI-TOF) m/z: $[M-2NH_4+H]^+$ cald for $C_8H_{16}N_2O_5P$, 251.0791; found: 251.0789.

**EXAMPLE 3: PREPARATION OF DERIVATIVE 29**

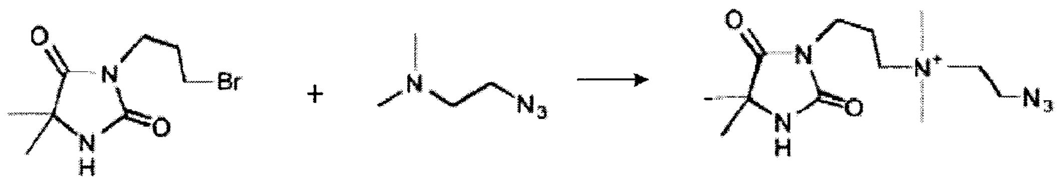

A B 29

To the solution of bromide **A** (1.48 g, 5.9 mmol) in MeCN (15mL) was added **B** (0.71 g, 6.2 mmol), and the resulting solution was heated to reflux for 14 h. Removal of solvent and excess **B** under vacuum afforded the crude **29** ($Br^-$ form), which was dissolved in minimum volume water and passed through ion-exchange resin (Amberlite R IRA-900, $Cl^-$) to give **29** as white solid ($Cl^-$ form, 1.87g, 99%).

**29:** $^1H$ NMR (DMSO-$d^6$, 300 MHz, δ) 3.79 (t, *J* = 4.8 Hz, 2 H; - $CH_2CH_2CH_2N^+$), 3.39 (t, *J* = 5.3 Hz, 2 H; -$N^+CH_2CH_2N_3$), 3.27 (t, *J* = 6.6 Hz, 2 H; - $N^+CH2CH_2N_3$), 3.19 (t, *J* = 8.1 Hz, 2 H; -$CH_2CH_2CH_2N^+$), 2.93 (s, 6 H; -$N(CH_3)_2$), 1.77-1.86 (m, 2H; -$CH_2CH_2CH_2N^+$), 1.17 (s, 6H; $C(CH_3)_2$); $^{13}C$ NMR (DMSO-$d^6$, 75 MHz, δ) 177.4 (1'-C=O), 155.0 (3'-C=O), 61.4 ($N^+CH_2CH_2N_3$), 61.2 ($CH_3C$), 57.8 ($N^+CH_3$), 50.5 (-$CH_2CH_2CH_2N^+$), 44.0 ($N^+CH_2CH_2N_3$), 34.8 (-$CH_2CH_2CH_2N^+$), 24.5 ($CH_3C$-), 21.3 (-$CH_2CH_2CH_2N^+$); HRMS (MALDI-TOF) m/z: $[M-Cl]^+$ calcd for $C_{12}H_{23}N_6O_2$, 283.1877; found: 283.1865.

**EXAMPLE 4: PREPARATION OF DERIVATIVE 30**

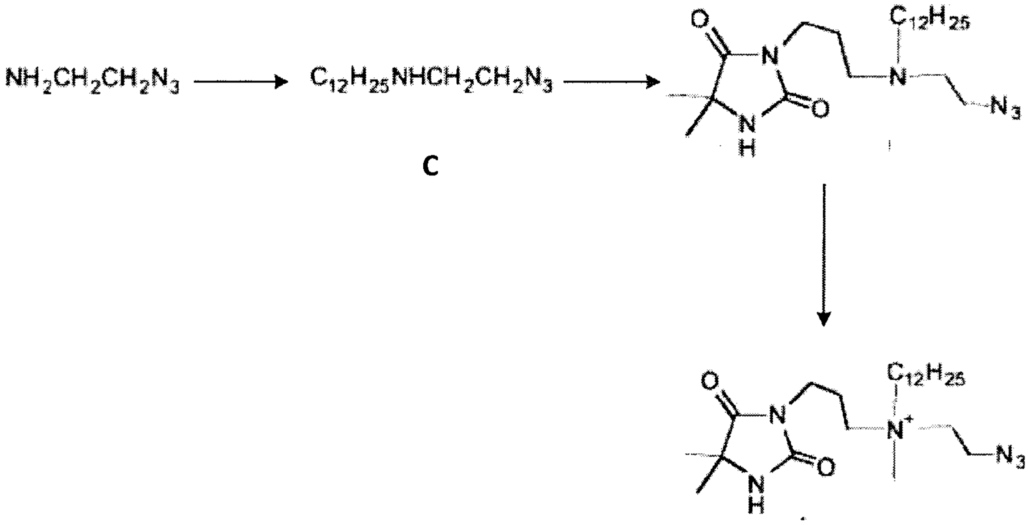

C D 30

To the lauryl bromide (1.49 g, 6.0 mmol) solution in DMF (15 mL) was added 2-azidoethylamine (0.54 g, 6.27 mmol) and anhydrous $K_2CO_3$ (2.5 g, 18 mmol) at room temperature. The suspension was maintained at 70 °C with stirring for 14 h before removing solvent under vacuum. The residue was partitioned between EtOAc and $H_2O$, and concentration of the organic layer produced the crude compound which was further purified by column chromatography (EtOAc/Hexanes =1:1) to afford C as colorless oil (0.92 g, 60%).

C: $^1H$ NMR ($CDCl_3$, 300 MHz, δ) 3.44 (t, *J* = 6.0 Hz, 2 H; -$NHCH_2CH_2N_3$), 2.81 (t, *J* = 6.0 Hz, 2 H; -$NHCH_2CH_2N_3$), 2.63 (t, *J* = 7.2 Hz, 2 H; - $CH_2CH_2NHCH_2CH_2N_3$), 1.52-1.48 (m, 2 H; --$CH_2CH_2NHCH_2CH_2N_3$), 1.30-1.27 (m, 18 H; lauryl chain), 0.90 (t, *J* = 6.6 Hz, 2 H; $CH_3CH_2CH_2$-); $^{13}C$ NMR ($CDCl_3$, 75 MHz) δ 51.5 (-$NHCH_2CH_2N_3$), 49.7 (-$NHCH_2CH_2N_3$), 48.6, (-$CH_2CH_2NHCH_2CH_2N_3$) 31.9(-$CH_2CH_2NHCH_2CH_2N_3$), 30.1, 29.7, 29.6, 29.5, 27.3, 22.7 (30.1 to 22.7 belong to carbon of lauryl chain), 14.1 (-$CH_3CH_2CH_2$-); HRMS (MALDI-TOF) m/z: $[M+H]^+$ cald for $C_{14}H_{31}N_4$, 255.2548; found: 255.2540.

To the bromide **A** (0.97 g, 3.9 mmol) solution in DMF (10 mL) was added **1** (1.0 g, 3.9 mmol) and anhydrous $K_2CO_3$ (1.6 g, 12 mmol) at room temperature. The suspension was maintained at 70 °C with stirring for 14 h before DMF

was removed and $H_2O$ (30 mL) and EtOAc (30 mL) was added. The organic layer was concentrated to give the crude compound which was further purified by column chromatography eluting with MeOH/$CHCl_3$ (1:20) to afford **D** as slight yellow oil (1.2 g, 72%). Compound **D** was directly mixed with excess MeI (0.6 mL, 9.6 mmol) in 20 mL $CH_3CN$ at room temperature. The resulting solution was continuously stirred for 10 h before removing the solvent under vacuum to afford the crude compound, which was purified on column chromatography eluting with MeOH/$CHCl_3$ (1:4) to give final ammonium salt **30** (1.4 g, 88%)

30: $^1H$ NMR ($CDCl_3$, 300 MHz, δ) 7.11 (s, 1 H; -NH), 4.13 (t, $J$ = 4.8 Hz, 2 H; $N^+CH_2CH_2N_3$), 3.88 (t, $J$ = 4.8 Hz, 2 H; $N^+CH_2CH_2N_3$), 3.71-3.67 (m, 4 H; $NCH_2CH_2CH_2N^+$ and $CH_2CH_2CH_2N^+$), 3.51-3.46 (m, 2 H; $-CH_2CH_2CH_2N^+$), 3.40 (s, 3 H; $-N^+(CH_3)_2$), 2.26 (t, $J$ = 7.0 Hz, 2 H; $N^+CH_2CH_2CH_2$-), 1.76-1.48 (m, 2 H; - $CH_2CH_2CH_2N^+$), 1.30-1.27 (m, 18 H; lauryl chain), 0.90 (t, $J$ = 6.6 Hz, 2 H; $CH_3CH_2CH_2$-); $^{13}C$ NMR ($CDCl_3$, 75 MHz, δ) 177.2 (1'-C=O), 156.0 (3'-C=O), 61.2 ($-CH_2CH_2CH_2N^+$), 61.0 ($CH_3C$), 51.5 ($N^+CH_3C_{11}H_{23}$), 49.7 ($-N^+CH_2CH_2N_3$), 48.6 ($N^+CH_2CH_2N_3$) 34.9 ($NCH_2CH_2CH_2N^+$), 30.1, 29.7, 29.6, 29.5, 27.3, 22.7 (from 30.1 to 22.7, $CH_2$ of the lauryl chain), 14.1 ($CH_3$ of the lauryl chain); HRMS (MALDI-TOF) m/z: $[M-I]^+$ cald for $C_{23}H_{45}N_6O_2$, 437.3600; found 437.3651.

**EXAMPLE 5: PREPARATION OF PRECURSOR 19**

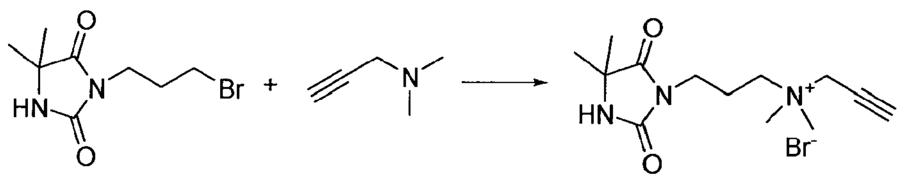

A E

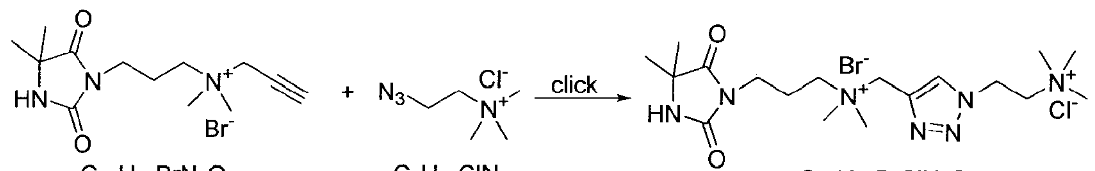


$C_{13}H_{22}BrN_3O_2$
Exact Mass: 331.09
Mol. Wt.: 332.24

$C_5H_{13}ClN_4$
Exact Mass: 164.08
Mol. Wt.: 164.64

$C_{18}H_{35}BrClN_7O_2$
Exact Mass: 495.17
Mol. Wt.: 496.87

E 19

To N-(3-(4,4-dimethyl-2,5-dioxoimidazolidin-1-yl)propyl)-N,N-dimethylprop-2-yn-1-aminium bromide (E, 1.90 g, 5.7 mmol) solution in $CH_3OH$ (30 mL, containing 3 mL $H_2O$) was added another azido precusor 2-azido-N,N,N-trimethylethanaminium chloride (0.94 g, 5.7 mmol) at room temperature. Catalyst $CuSO_4$ (1M, 0.57mL) and copper powder (2.55 g, 40 mmol) was added to initiate the click reaction. The suspension was maintained at room temperature with stirring for 24 h before solid was filtered. The filtrate was applied on a flash silica gel column to purify the product **19.** Product (1.7 g, 60%) was obtained when 80~90% MeOH in DCM was used as eluting solvent. This compound was transformed into its Cl- form before chlorination.

**E:** $^1H$ NMR ($D_2O$, 300 MHz, δ) 4.29 (s, 2 H), 3.64 (t, $J$= 5.6 Hz, 2 H), 3.47-3.53 (m, 2 H), 3.21 (s, 6 H), 2.14-2.21 (m, 2 H), 1.46 (s, 6H); $^{13}C$ NMR ($D_2O$, 75 MHz, δ) 180.6, 157.7, 70.3, 61.1, 59.2, 54.1, 50.7, 48.9, 35.2, 23.4, 21.4; HRMS (MALDI-TOF) m/z: not measured yet

**19:** $^1H$ NMR ($D_2O$, 300 MHz, δ) 8.53 (s, 1 H), 5.15 (t, $J$ = 6.1 Hz, 2H), 4.74 (m, 2H), 4.10 (t, $J$ = 6.2 Hz, 2 H), 3.63 (t, $J$ = 6.3 Hz, 2 H), 3.22-3.33 (m, 2 H), 3.27 (s, 9 H), 3.16 (s, 6 H), 2.24-2.29 (m, 2 H), 1.45 (s, 6H) $^{13}C$ NMR ($D_2O$, 75 MHz, δ) and HRMS (MALDI-TOF) m/z: not measured yet.

**EXAMPLE 6: PREPARATION OF PRECURSOR 15**

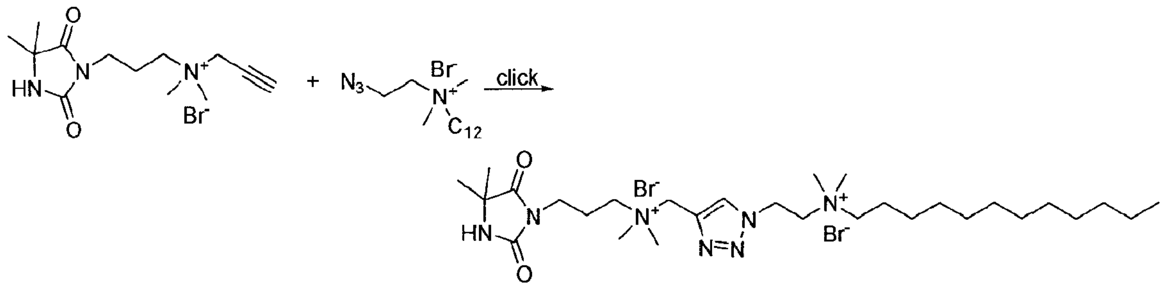


E 15

The above click reaction was performed using $Cu^{2+}$/Cu powder (9:1 MeOH/$H_2O$) catalysis system.(project 121208)

**15:** $^1$H NMR ($D_2O$, 300 MHz, δ) 8.59 (s, 1 H), 5.15 (t, *J* = 6.3 Hz, 2H), 4.76 (m, 2H), 4.09 (t, *J* = 6.3 Hz, 2 H), 3.63 (t, *J* = 6.3 Hz, 2 H), 3.49-3.54 (m, 2 H), 3.22-3.34 (m, 2 H), 3.26 (s, 6 H), 3.18 (s, 6 H), 2.26-2.31 (m, 2 H), 1.81 (m, 2H), 1.46 (s, 6H), 1.30-1.37 (m, 18H), 0.90 (t, *J* = 6.3 Hz, 3H); $^{13}$C NMR ($D_2O$, 75 MHz, δ) 180.2, 157.0, 135.7, 129.6, 65.3, 59.1, 51.2, 50.7, 48.9, 44.1, 35.3, 31.6, 29.2, 29.1, 28.9, 28.6, 25.7, 23.6, 22.3, 22.2, 21.6, 13.7; HRMS (MALDI-TOF) m/z: not measured yet.

**EXAMPLE 7: PREPARATION OF PRECURSOR 5 & COMPOUND 6**

1 5

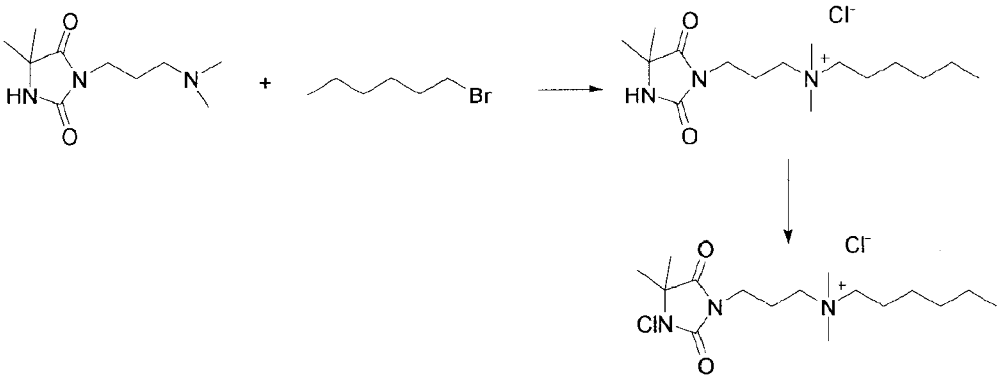

6

1.68 g (7.89 mmol) of compound **1** was mixed with 1.95 g bromohexane (1.5 equiv.) and dissolved in 40 ml of $CH_3CN$. The resulting solution was heated with stirring to gentle reflux for 24 hours. After the reaction was completed, the solvent was removed by rotary evaporator and the residue was purified by column chromatography (MeOH/$CH_2Cl_2$, 1:3) to afford the bromo-quaternary ammonium salt, which was dissolved in a minimum amount of water and slowly passed through an anion-exchange resin (Amberlite R IRA-900, $Cl^-$) to afford **5** as white solid.

**5:** $^1$H NMR ($D_2O$, 300 MHz, δ) 3.62 (t, *J* = 6.6 Hz, 2 H), 3.28-3.37 (m, 4 H), 3.09 (s, 6 H), 2.09-2.17 (m, 2 H), 1.70-1.75 (m, 2H), 1.45 (s, 6 H), 1.35-1.40 (m, 6 H), 0.90 (t, *J* = 6.4 Hz, 3 H); $^{13}$C NMR ($D_2O$, 75 MHz, δ) 180.6, 157.1, 64.1, 60.7, 59.2, 50.9, 35.4, 30.4, 25.0, 23.5, 21.8, 21.6, 21.2, 13.2;

To the *t*-BuOH and water solution (*t*-BuOH:$H_2O$, 4:1, v/v) was added the non-chlorinated precursor **5.** The resulting solution was subsequently added excess *t*-butyl hypochlorite (3 to 4 equiv.) and allowed to stir overnight. Excess *t*-butyl hypochlorite and solvent were removed under vacuum and yielded the corresponding chlorinated compound **6** as white or yellow solid.

6: $^1$H NMR ($D_2O$, 300 MHz, δ) 3.71 (t, *J* = 6.4 Hz, 2 H), 3.29-3.38 (m, 4 H), 3.09 (s, 6 H), 2.09-2.18 (m, 2 H), 1.71-1.76 (m, 2H), 1.53 (s, 6 H), 1.35-1.41 (m, 6 H), 0.91 (t, *J* = 6.5 Hz, 3 H); $^{13}$C NMR ($D_2O$, 75 MHz, δ)176.8, 155.4, 66.3, 64.2, 60.6, 50.8, 36.6, 30.4, 29.6, 25.0, 21.7, 21.1, 21.0, 13.2;

**EXAMPLE 8: PREPARATION OF PRECURSOR 37**

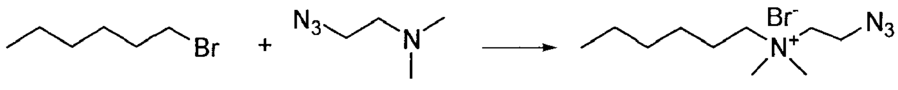

F

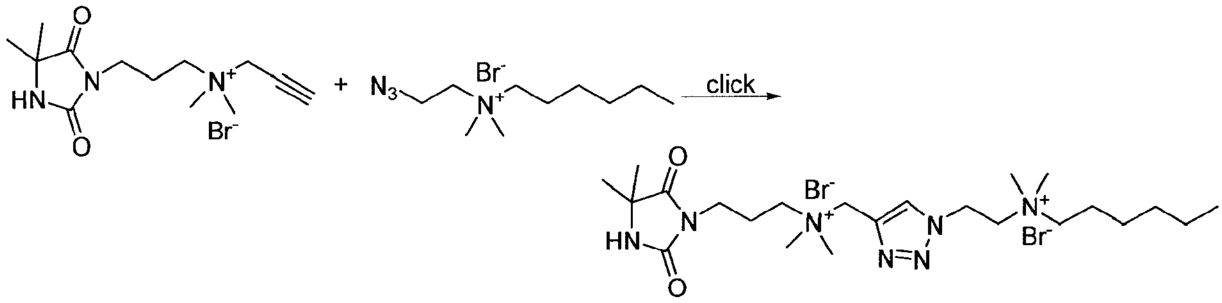


E F 37

To **E** (1.61 g, 4.8 mmol) solution in $CH_3OH$ (30 mL, containing 3 mL $H_2O$) was added **F** (1.30 g, 4.8 mmol) at room temperature. Click catalyst $CuSO_4$ (1M, 0.48mL) and copper powder (2.15 g, 33 mmol) was added to initiate the connection reaction. The suspension was maintained at room temperature with stirring for 24 h before solid was filtered. The filtrate was applied on flash silica gel column to purify the product. Product 37 (1.75 g, 60%) was obtained when 60~70% MeOH in DCM was used as eluting solvent. This compound was transformed into its $Cl^-$ form before chlorination.
**F:** $^1$H NMR ($D_2O$, 300 MHz, δ) 3.95 (t, *J* = 5.0 Hz, 2 H), 3.57 (t, *J* = 5.6 Hz, 2 H), 3.38 (t, *J*= 7.9 Hz, 2 H), 3.14 (s, 6 H), 1.77-1.82 (m, 2 H), 1.33-1.36 (m, 6 H), 0.90 (t, *J* = 6.5 Hz, 3 H); $^{13}$C NMR ($D_2O$, 75 MHz, δ) 65.4, 61.8, 51.1, 44.5, 30.4, 25.1, 21.8, 21.7, 13.2; HRMS (MALDI-TOF) m/z: not measured yet
**37:** $^1$H NMR ($D_2O$, 300 MHz, δ) 8.55 (s, 1 H), 5.13 (t, *J* = 6.3 Hz, 2H), 4.74 (m, 2H), 4.05 (t, *J* = 6.5 Hz, 2 H), 3.63 (t, *J* = 6.2 Hz, 2 H), 3.32-3.45 (m, 4 H), 3.22 (s, 6 H), 3.16 (s, 6 H), 2.24-2.30 (m, 2 H), 1.75 (m, 2H), 1.45 (s, 6H), 1.33 (m, 6H), 0.89(t, *J* = 6.0 Hz, 3H); $^{13}$C NMR ($D_2O$, 75 MHz, δ) 180.6, 157.1, 135.7, 129.5, 65.3, 61.3, 59.2, 51.2, 50.6, 48.9, 44.1, 35.3, 30.4, 25.0, 23.5, 21.9, 21.7, 21.5, 13.2 ; HRMS (MALDI-TOF) m/z: not measured yet.

**EXAMPLE 9: PREPARATION OF DERIVATIVE 39**

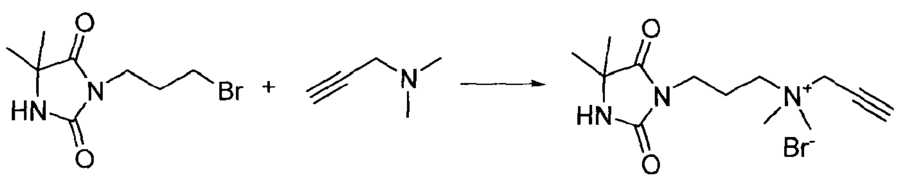

A 39

To the solution of bromide **A** (1.48 g, 5.9 mmol) in MeCN (15mL) was added N,N-dimethylprop-2-yn-1-amine (0.49 g, 5.9 mmol), and the resulting solution was heated to reflux for 14 h. Removal of solvent under vacuum afforded the product **39** ($Br^-$ form, >98%), which could be further purified by flash chromatography or used directly for next steps.
**39:** $^1$H NMR ($D_2O$, 300 MHz, δ) 4.29 (s, 2 H), 3.64 (t, *J* = 5.6 Hz, 2 H), 3.47-3.53 (m, 2 H), 3.21 (s, 6 H), 2.14-2.21 (m, 2 H), 1.46 (s, 6H); $^{13}$C NMR ($D_2O$, 75 MHz, δ) 180.6, 157.7, 70.3, 61.1, 59.2, 54.1, 50.7, 48.9, 35.2, 23.4, 21.4; HRMS (MALDI-TOF) m/z: not measured yet

**EXAMPLE 10: PREPARATION OF PRECURSOR 7 & COMPOUND 8**

1 7

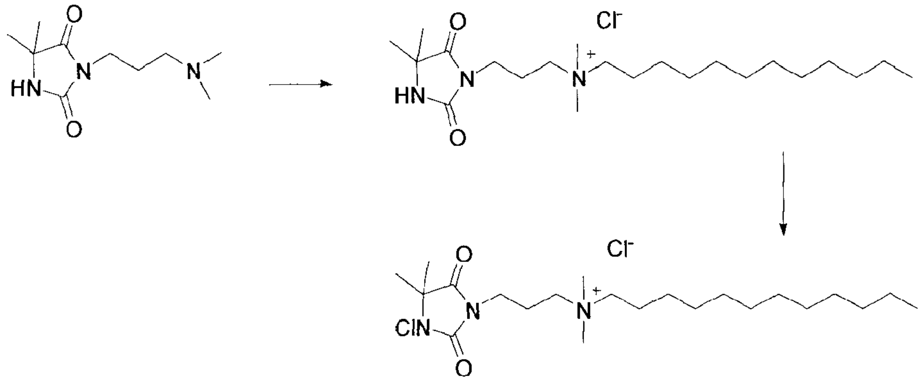

8

1.5 g (7.0 mmol) of compound **1** was mixed with 1.95 g bromododecane (2 equiv.) and dissolved in 40 ml of $CH_3CN$. The resulting solution was heated with stirring to gentle reflux for 24 hours. After the reaction was completed, the solvent was removed by rotary evaporator and the residue was purified by column chromatography ($MeOH/CH_2Cl_2$, 1:3, v/v) to afford the bromo-quaternary ammonium salt, which was dissolved in a minimum amount of water and slowly passed through an anion-exchange resin (Amberlite R IRA-900, $Cl^-$) to afford **7** as white solid.

**7:** $^1H$ NMR ($D_2O$, 300 MHz, δ) 3.62 (t, *J* = 6.2 Hz, 2 H), 3.41-3.43 (m, 4 H), 3.18 (s, 6 H), 2.14-2.17 (m, 2 H), 1.76-1.77 (m, 2H), 1.47 (s, 6 H), 1.32-1.40 (m, 18 H), 0.92 (t, *J* = 6.3 Hz, 3 H); $^{13}C$ NMR ($D_2O$, 75 MHz, δ) 179.7, 156.8, 63.8, 60.7, 58.9, 51.3, 35.5, 31.9, 29.7, 29.6, 29.4, 29.0, 26.0, 23.9, 22.6, 22.3, 21.5, 18.9;

To the *t*-BuOH and water solution (*t*-BuOH:$H_2O$, 4:1, v/v) was added the non-chlorinated precursor **7.** The resulting solution was subsequently added excess *t*-butyl hypochlorite (3 to 4 equiv.) and allowed to stir overnight. Excess *t*-butyl hypochlorite and solvent were removed under vacuum and yielded the corresponding chlorinated compound **8** as white or yellow solid.

**8:** $^1H$ NMR ($D_2O$, 300 MHz, δ) 3.74 (t, *J* = 6.0 Hz, 2 H), 3.33-3.37 (m, 4 H), 3.16 (s, 6 H), 2.15-2.17 (m, 2 H), 1.76-1.77 (m, 2H), 1.52 (s, 6 H), 1.32-1.38 (m, 18 H), 0.92 (t, *J* = 6.0 Hz, 3 H); $^{13}C$ NMR ($D_2O$, 75 MHz, δ) 175.7, 155.0, 66.1, 60.7, 59.9, 51.7, 36.7, 31.9, 29.7, 29.6, 29.4, 29.3, 25.8, 22.6, 22.2, 21.5, 21.3, 13.9;

**EXAMPLE 11: PREPARATION OF PRECURSOR 9 & COMPOUND 10**

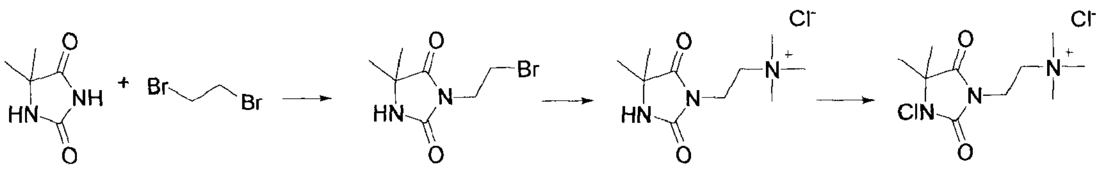

J A 9 10

3.2 g (25.4 mmol) of compound **J** was mixed with 7.2 g potassium carbonate (3 equiv.) and then dissolved in 160 ml of acetone and reflux for 30 minutes before 6.6 ml (1.3 equiv.) of 1,2-dibromoethane was added followed by continuous reflux for 6 hours. After the reaction was finished, the extra salts were filtered off by passing through Celite then air dried. The residues were purified by column chromatography (Ethyl acetate/hexane, 3:2-4:1, v/v) to afford **A** as white solid.

**A:** $^1H$ NMR ($CDCl_3$, 300 MHz, δ) 6.15 (broad, 1H), 3.92 (t, *J* = 6.2 Hz, 2 H), 3.61 (t, *J* = 6.2 Hz, 2 H), 1.48 (s, 6 H); $^{13}C$

NMR ($CDCl_3$, 75 MHz, δ) 177.1, 156.1, 59.0, 39.7, 28.1, 25.1.

1.85 g (7.87 mmol) of compound **A** and 5 ml(2.2 equiv.) of trimethylamine was dissolved in 25 ml 95% ethanol and then reflux for 24 hours. Solvent was removed by rotary evaporator and column chromatography ($MeOH/CH_2Cl_2$, 1:3-2:3, v/v) purification afforded the bromo-quaternary ammonium salt, which was dissolved in a minimum amount of water and slowly passed through an anion-exchange resin (Amberlite R IRA-900, $Cl^-$) to afford **9** as white solid.

**9:** $^1H$ NMR ($D_2O$, 300 MHz, δ) 4.02 (t, *J* = 6.7 Hz, 2 H), 3.65 (t, *J*= 6.8 Hz, 2 H), 3.25 (s, 6 H), 1.45 (s, 6 H); $^{13}C$ NMR ($D_2O$, 75 MHz, δ) 179.0, 156.3, 62.5, 59.4, 53.4, 32.6, 23.4.

To the *t*-BuOH and water solution (*t*-BuOH:$H_2O$, 4:1, v/v) was added the non-chlorinated precursor **9.** The resulting solution was subsequently added excess *t*-butyl hypochlorite (3 to 4 equiv.) and allowed to stir overnight. Excess *t*-butyl hypochlorite and solvent were removed under vacuum and yielded the corresponding chlorinated compound **10** as white or yellow solid.

**10:** $^1H$ NMR ($D_2O$, 300 MHz, δ) 4.12 (t, *J* = 6.8 Hz, 2 H), 3.69 (t, *J* = 6.7 Hz, 2 H), 3.27 (s, 6 H), 1.53 (s, 6 H); $^{13}C$ NMR ($D_2O$, 75 MHz, δ) 176.1, 154.6, 66.6, 62.2, 53.4, 35.5, 20.9;

**EXAMPLE 12: PREPARATION OF PRECURSOR 11**

A H I

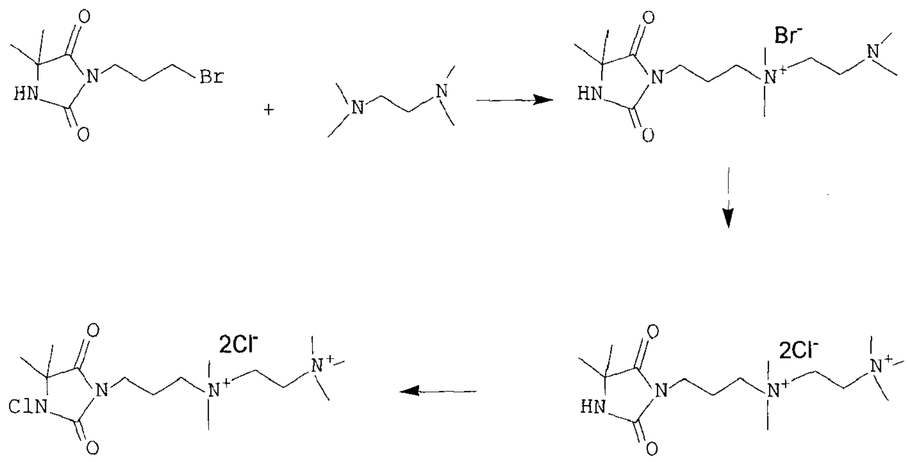

12 11

1.5g (6.02 mmol) of bromide **A** was dissolved in 25 ml $CH_3CN$, followed by addition of 4.5 ml (5 equiv) of N,N,N',N'-Tetramethylethylenediamine **H.** The resulting solution was heated with stirring to gentle reflux for 18 hours. Yellowish solution was then air blow to dry and the residue was purified by column chromatography ($MeOH/CH_2Cl_2$, 1:3, v/v) to yield **I** as yellowish oil (1.3g, 76%).

**I:** $^1H$ NMR ($D_2O$, 300 MHz, δ) 3.61 (t, *J* = 6.0 Hz, 2 H), 3.49 (t, *J* = 7.5 Hz, 2 H), 3.41 (t, *J* = 6 Hz, 2H),3.15 (s, 6 H), 2.83(t, J=7.5Hz, 2H), 2.30(s, 6H)2.09-2.18 (m, 2H), 1.45(s,6H;);

$^{13}C$ NMR($CDCl_3$, 75 MHz)δ[ppm]: 180.57, 157.04, 61.8, 60.7, 59.2, 53.5, 44.4, 43.7, 35.4, 23.6, 21.4

0.9 g of synthesized compound **I** (3.15 mmol) was dissolved in solution of $CH_3CN$ and $CH_3OH$ ($CH_3CN$:$CH_3OH$=2:1, v/v) for a total of 30 ml. 2 ml of methyl iodide (10 equiv.) was added and the resulting solution was continuously stirred at room temperature for 22 hours. Solvent and excess of methyl iodide were removed by air blow followed by vacuum. The resulting yellowish oil was dissolved in MeOH, concentrated and purified by column chromatography ($MeOH/CH_2Cl_2$, 1:3-1:2, v/v) to yield lodo-quaternary ammonium salts as yellow solid. Then the yellow solid was dissolved in minimum amount of water and slowly passed through an anion-exchange resin (Amberlite R IRA-900, $Cl^-$) to afford **11** as white solid.

**11:** $^1H$ NMR ($D_2O$, 300 MHz, δ) 4.03(s,4H), 3.63 (t,*J*=7.5Hz,2H), 3.54(t, *J*=7.5Hz, 2H), 3.32(s, 15H), 2.21(m, 2H), 1.46(s, 6H); $^{13}C$ NMR($CDCl_3$, 75 MHz)δ[ppm]: 180.7, 156.8, 63.1, 59.3, 56.3, 57.5, 53.8, 35.2, 23.4, 21.4.

**EXAMPLE 13: PREPARATION OF COMPOUND 12**

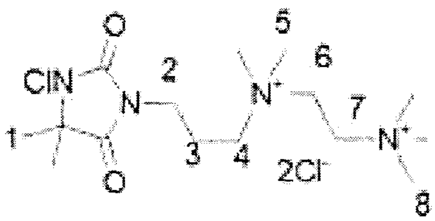

**12**

To the *t*-BuOH and water solution (*t*-BuOH:$H_2O$, 4:1, v/v) was added the non-chlorinated precursort **11.** The resulting solution was subsequently added excess *t*-butyl hypochlorite (3 to 4 equiv.) and allowed to stir overnight. Excess *t*-butyl hypochlorite and solvent were removed under vacuum and yielded the corresponding chlorinated compound **12** as white or yellow solid.

**12:** $^1$H NMR ($D_2O$, 300 MHz, δ) 4.03 (m, 4 H), 3.72 (t, *J* = 6.8 Hz 2 H), 3.56 (t, *J* = 7.4 Hz, 2 H), 3.32 (s, 9 H), 3.26 (s, 6 H), 2.21-2.26 (m, 2 H), 1.49 (s, 6H); $^{13}$C NMR ($D_2O$, 75 MHz, δ) 176.9, 155.6, 63.3, 59.3, 57.9, 56.5, 53.5, 51.2, 35.3, 23.4, 21.2;

**EXAMPLE 14: PREPARATION OF PRECURSOR 13**

**J** **A** **1**

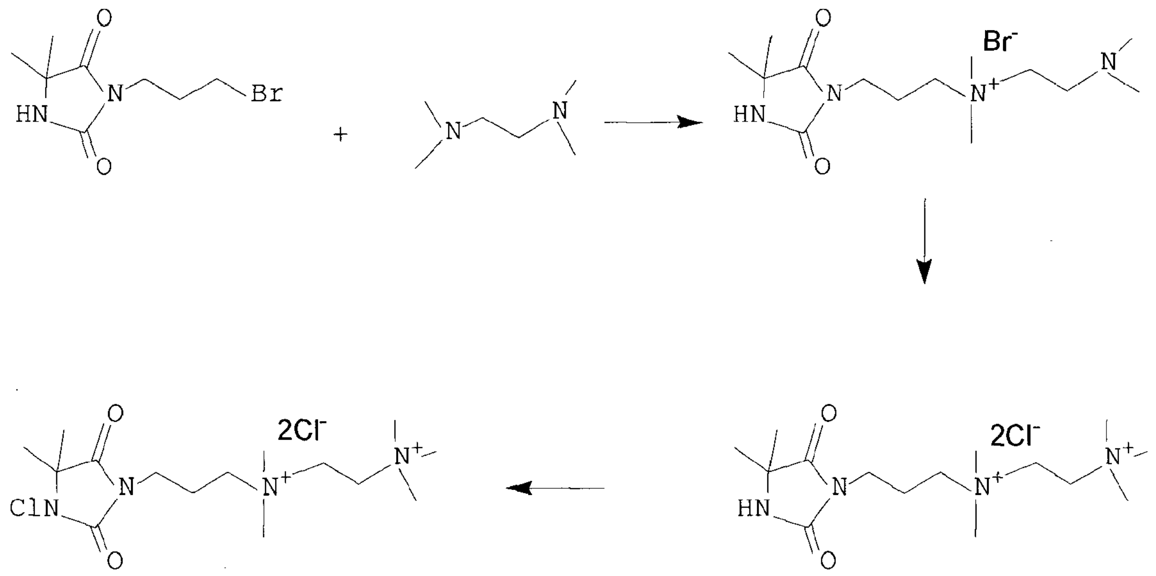

**14** **13**

3.28g (26 mmol) of 5,5-dimethyl hydantoin **J** were mixed with 7.2 g (52 mmol, 2 equiv.) $K_2CO_3$ and dissolved in 150 ml acetone. The resulting suspension was heated to reflux for 20 minutes before 8.0 ml of 1,3-Dibromopropane (3 equiv) was added. Reflux was allowed to continue for a total of 4 hours. Acetone was removed by air dry and the residue was partitioned between ethyl acetate and water. Organic layer was obtained and washed twice more. The concentrated organic layer was purified by column chromatography (Ethyl acetate/hexane, 1:2, v/v) to obtain **14** as white solid (5.2g, 80%)

1.2g (4.8 mmol) of bromide **A** was dissolved in EtOH solution (30 ml EtOH + 3 ml $H_2O$), to which 1.6 g (24 mmol, 5 equvi.) of aqueous dimethylamine was added followed by 5 equivalence of NaOH. The resulting solution was heated to reflux overnight under vacuum. Removal of solvent and excess dimethylamine by air dry and the residue was purified by column chromatography eluting with MeOH/$CH_2Cl_2$ (1:5, v/v) to afford **1** as white solid (0.7g, 51%).

**1:** $^1$H NMR ($D_2O$, 300 MHz, δ) 3.55 (t, *J*=7.5 Hz, 2H), 2.65 (t, *J*=7.5 Hz, 2H;), 2.46 (s, 6H; $N(CH_3)_2$), 1.88(m, 2H;), 1.44(s,

6H); $^{13}C$ NMR($D_2O$, 75 MHz) δ[ppm]: 181.0, 157.3, 58.8, 55.6, 43.6, 36.0, 24.3, 23.7

0.25 g (1.17 mmol) of compound **1** was dissolved in 10 ml $CH_3CN$ followed by addition of 0.32 g (1.1 equiv.) bromide **A.** Suspended white solid was formed initially but eventually disappeared while it was heated to reflux. The clear solution was allowed to undergo reflux under vacuum for 24 hours. Removal of solvent followed by purification with column chromatography (MeOH/$CH_2Cl_2$, 1:3, v/v) to give bromo-quaternary ammonium salts, which was dissolved in a minimum amount of water and slowly passed through an anion-exchange resin (Amberlite R IRA-900, $Cl^-$) to afford **13** as white solid (0.46g, 94%)

**13:** $^1H$ NMR ($D_2O$, 300 MHz, δ) 3.6 (t, *J*=6Hz, 2H), 3.37 (t, *J*=7.5Hz,2H), 3.12 (s, 3H), 2.10, (m,2H),1.45(s,6H) ; $^{13}C$ NMR($D_2O$, 75 MHz) δ[ppm]: 180.7, 157.1, 61.3,59.2, 50.8, 35.2, 23.6, 21.2

**EXAMPLE 15: PREPARATION OF COMPOUND 14**

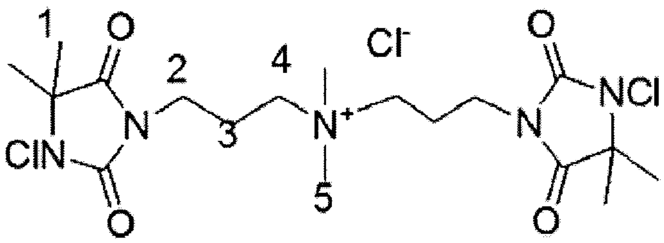

**14**

To the *t*-BuOH and water solution (*t*-BuOH:$H_2O$, 4:1, v/v) was added the non-chlorinated precursor **13.** The resulting solution was subsequently added excess *t*-butyl hypochlorite (3 to 4 equiv.) and allowed to stir overnight. Excess *t*-butyl hypochlorite and solvent were removed under vacuum and yielded the corresponding chlorinated compound **14** as white or yellow solid.

5: $^1H$ NMR ($D_2O$, 300 MHz) δ[ppm]: 3.7 (t, J=7.5Hz, 2H), 3.37 (t, J=4.5Hz,2H), 3.13 (s, 3H), 2.13, (m,2H;),1.53(s,6H); $^{13}C$ NMR($D_2O$, 75 MHz) δ[ppm]: 176.7 , 155.4, 66.5 61.3, 50.9, 36.5, 21.3, 20.9 .

**EXAMPLE 16: PREPARATION OF PRECURSOR 27**

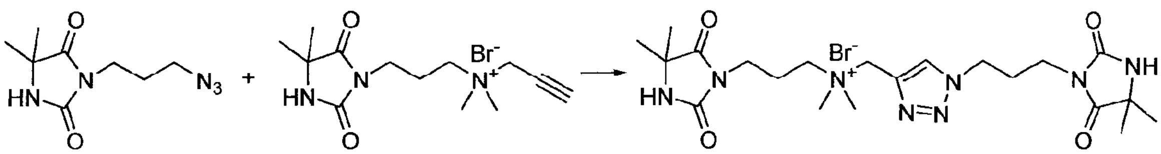

**E** **27**

To E (1.40 g, 4.2 mmol) solution in $CH_3OH$ (30 mL, containing 3 mL $H_2O$) was added azido-DMH precusor 3-(3-azidopropyl)-5,5-dimethylimidazolidine-2,4-dione (1.06 g, 5.0 mmol) at room temperature. Click catalyst $CuSO_4$ (1M, 0.42 mL) and copper powder (1.88 g, 29 mmol) was added to initiate the connection reaction. The suspension was maintained at room temperature with stirring for 24 h before solid was filtered. The filtrate was applied on a flash silica gel column to afford product **27** (1.8 g, 80%) when 30% MeOH in DCM was used as eluting solvent. This compound was transformed into it Cl- form before chlorination.

**27:** $^1H$ NMR ($D_2O$, 300 MHz, δ) 8.40 (s, 1H), 4.70 (s, 2H), 4.56 (t, *J* = 6.2 Hz, 2 H), 3.62 (t, *J* = 5.9 Hz, 4 H), 3.55 (t, *J* = 6.3 Hz, 4 H), 3.28-3.33 (m, 2H), 3.16 (s, 6 H), 2.37-2.24 (m, 4 H), 1.43 (s, 6H), 1.41 (s, 6H); $^{13}C$ NMR ($D_2O$, 75 MHz, δ) not measured yet.; (MALDI-TOF) m/z: not measured yet.

## EXAMPLE 17: ANTIBACTERIAL ACTIVITY OF CATIONIC ANALOGS OF N-HALAMINE - COMPOUND 2

***Test Compounds:***

To test the antibacterial activity of compounds comprising structural cationic and N-halamine moieties covalently bonded together, Precursor **1,** a hydantoin derivative with cationic charge, was synthesized and converted to its N-chloramine counterpart (Compound **2**). A hydantoin derivative with anionic charge (Anionic Precursor **42**), was also synthesized and converted to N-chloramine for comparison (Anionic Compound **43**).

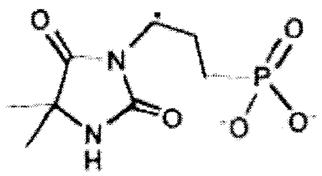

**42**

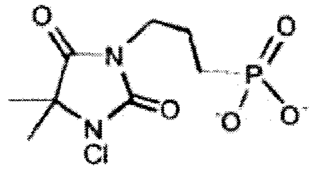

**43**

Both compounds **1** and **42** were used to serve as controls.

***Test Cultures:***

Strains of *Escherichia coli* (*E.coli*) a typical Gram-negative bacterium and *Staphylococcus aureus* a typical Gram-positive bacterium were studied. A clinical isolate of healthcare-associated MRSA (HA-MRSA) isolate #77090, community-associated MRSA (HA-MRSA) #70527, and those of multi-drug-resistant *E.coli* (MDR-*E.coli*) isolate #70094 and # 95882 were obtained from the CANWARD (Canadian Ward Surveillance) study assessing antimicrobial resistance in Canadian hospitals, www.canr.ca. *E.coli* ATCC 25922 and MRSA ATCC 33592 were obtained from the American Type Culture Collection (ATCC) **(Manassas, VA).**

***Methods:***

In the model study we investigated the bactericidal performance of small molecules **2** and **43** against three strains for each bacterium at the concentration of 15 ppm.

Tryptone Soya Agar (TSA) was used for bacterial culture. After sub-cultured from stocks, bacteria were allowed to grow at 37 °C for 18-20 hours to obtain logarithmic-phase cultures. Biocidal activity of **2** and **43** were completed as followed. To 20 mL bacterial suspension ($10^6$-$10^7$ colony forming units (CFU)/mL) in a centrifuge tube was added 30 $\mu$L **2** or **43** solutions (0.28 M stock solution) respectively to achieve final 15 ppm $[Cl^+]$). Timing of the exposure to the disinfectant was started immediately with the addition of the synthetic compound **2** or **43.** After the contact for 5 min, 10 min, and 20 min respectively, 1.0 mL aliquots were withdrawn and added to an equal volume of 0.02 N sodium thiosulfate in PBS (0.05 M, pH 7.0). The quenched suspension was serially diluted and 100 $\mu$L of each resulting dilutions were placed onto nutrient agar plates. The same procedure was also applied to compounds **1** and **42** as controls. After being incubated at 37 °C for 24 hours, viable bacterial colonies on the plates were counted. Bacterial reduction was reported according to the following equation.

$$\text{Percentage reduction of bacteria (\%)} = (A\text{-}B)/A \times 100$$

$$\text{Log reduction} = \text{Log } (A/B)$$

Where A is the number of bacteria retrieved from controls (CFU/mL), and B is the number of bacteria retrieved from **2** or **43** (CFU/mL).

***Results:***

As shown in Table 1, Compound **2** demonstrated a total kill of all six bacterial strains within 5 min whereas no significant reduction was observed for **43** at the same time frame. For **43,** total kill or >3 log reduction was only achieved at the contact time of 20 min except for MRSA #77090. It indicated that as compared with negative charge, positive charge contributed to a faster bacterial killing of the N-chloramine compound. The fact that > 3 log reduction or total kill (except MRSA # 77090) can still be achieved by **43** after extending the contact time to 20 min led us to a conclusion that the negative charge just impedes the killing kinetic without compromising the overall antibacterial capacity of **43.**

**Table 1.** Antibacterial efficacy of **2** and **43** against 3 *E.coli* and 3 MRSA strains

| Bacteria[a] | | Synthetic compounds [b] | Bacteria reduction at various contact times (min) | | | | | |
|---|---|---|---|---|---|---|---|---|
| | | | 5 | | 10 | | 20 | |
| | | | % | $Log_{10}$ | % | $Log_{10}$ | % | $Log_{10}$ |
| Gram-negative | *E.coli* ATCC 25922 | **2** | 100 | 6.63 | 100 | 6.63 | 100 | 6.63 |
| | | **43** | 28.5±3.4 | 0.15 | 99.96±0.00 | 3.40 | 100 | 6.63 |
| | MDR-*E.coli* (#70094) | **2** | 100 | 6.17 | 100 | 6.17 | 100 | 6.17 |
| | | **43** | 35.6±1.9 | 0.19 | 66.8±0.5 | 0.48 | 100 | 6.17 |
| | MDR-*E.coli* (#95882) | **2** | 100 | 6.67 | 100 | 6.67 | 100 | 6.67 |
| | | **43** | 4.6±1.2 | 0.02 | 99.75±0.02 | 2.59 | 99.94±0.03 | 3.24 |
| Gram-positive | MRSA ATCC33592 | **2** | 100 | 6.60 | 100 | 6.60 | 100 | 6.60 |
| | | **43** | 6.2±0.9 | 0.028 | 98.83±0.12 | 1.94 | 99.94±0.01 | 3.19 |
| | MRSA (#70527) | **2** | 100 | 6.76 | 100 | 6.76 | 100 | 6.76 |
| | | **43** | 32.5±3.5 | 0.17 | 99.78±0.00 | 2.97 | 100 | 6.76 |
| | MRSA (#77090) | **2** | 100 | 6.16 | 100 | 6.16 | 100 | 6.16 |
| | | **43** | 37.1±10.6 | 0.2 | 52.8±4.5 | 0.33 | 74.2±0.5 | 0.59 |

a. Inoculum concentration: 1.46-5.87 × $10^6$CFU/mL
b. compounds **1** and **42** were used as controls.

**EXAMPLE 18: ANTIBACTERIAL ACTIVITY OF CATIONIC ANALOGS OF N-HALAMINE - COMPOUNDS 2, 12, 14, 15, AND 16**

The antibacterial activity of Compounds 2, 12, 14, 15, and 16 was similarly tested.

***Test Cultures:***

Logarithmic-phase cultures of *P. aeruginosa* were prepared by initially suspending several colonies in cation-supplemented Mueller-Hinton broth (Oxoid, Nepean, Ontario, Canada) at a density equivalent to a 0.5 McFarland standard ($1\times10^8$ cfu/mL). This suspension was then diluted 1:100 and 20 $\mu$L of the diluted suspension was further diluted in 60 mL of cation-supplemented Mueller-Hinton broth. Following overnight growth at 37°C, suspensions were diluted 1:10 or 1:00 to get inoculums of approximately $1\times10^6$ or $1\times10^5$ cfu/mL.

Logarithmic-phase cultures of MRSA were prepared using similar way except TSA broth was used instead.

***Test Compounds:***

Compounds **2, 12, 14, 15,** and **16** were tested using the methodology described below.

***Methods:***

Biocidal activity of synthetic compounds was completed as followed. To 20 mL of bacterial suspension ($10^5$ or $10^6$ cfu/mL) in a centrifuge tube was added 30 $\mu$L solution of synthetic compounds (0.282 M stock solution) to achieve a final [$Cl^+$] of 15 ppm. Timing of the exposure to the disinfectant was started immediately with the addition of the synthetic compound. After predetermined contact time, 1.0 mL aliquots were withdrawn and added to an equal volume of 0.02 N sodium thiosulfate in PBS (0.1 M, pH 7.4). The quenched suspension was serially diluted and 100 $\mu$L of each resulting dilution was placed onto nutrient agar plates. After being incubated at 37°C for 24 hours, the viable bacterial colonies on the plates were counted. Bacterial reduction was reported according to:

$$\text{Percentage reduction of bacteria (\%)} = (A - B) / A \times 100$$

$$\text{Log reduction} = \text{Log}\,(A/B)\ (4)$$

where A is the number of bacteria in the starting inoculum (cfu/mL), and B is the number of bacteria retrieved from synthetic compounds (cfu/mL).

***Results:***

Compounds **2, 12,** and **14** were challenged with CA-MRSA 40065 and *Pseudomonas aeruginosa* 73104. It appears that Compounds **2, 12**, and **14** cannot bring any significant reduction of $10^6$ cfu/mL *P. aeruginosa* within 60 min of contact.

The results of inactivation efficacy of Compounds **2, 12,** and **14** against CA-MRSA 40065 are presented in Table 2.

**Table 2: Antibacterial efficacy of Compounds 2, 12, and 14 against CA-MRSA 40065**

| Bacteria | Synthetic compounds[b] | Bacteria reduction at various contact times (min) | | | | | | | | | |
|---|---|---|---|---|---|---|---|---|---|---|---|
| | | 1 | | 3 | | 5 | | 10 | | 60 | |
| | | % | $Log_{10}$ | % | $Log_{10}$ | % | $Log_{10}$ | % | $Log_{10}$ | % | $Log_{10}$ |
| CA-MRSA 40065 | **2** | | | 92.8 | 1.14 | 93.8 | 1.20 | 90.3 | 1.01 | 99.6 | 2.36 |
| | **12** | | | 88.0 | 0.92 | 923 | 0.92 | 94.2 | 1.24 | 99.5 | 2.31 |
| | **14** | 85.8 | 0.85 | 79.1 | 0.68 | 643 | 0.45 | 91.9 | 1.09 | 99.5 | 2.32 |

Note: Inoculum concentration: 1.57 - 1.75 $\times$ 10 CFU/mL; all compounds were prepared at the concentration equivalent to of 15 ppm [$Cl^+$]

It appears that compounds **2, 12,** and **14** are all very similar in their potency versus CA-MRSA. At 10 min all achieve >90% inhibition and at 60 min all achieve > 99% inhibition. Compounds **2, 12,** and **14** were then challenged with $10^5$ CFU/mL *P. aeruginosa* and data are presented in Table 3.

**Table 3: Antibacterial efficacy of Compounds 2, 12, and 14 against *P. aeruginosa 73104***

| Synthetic compounds | Bacteria reduction at various contact times (min) | | | | | | | |
|---|---|---|---|---|---|---|---|---|
| | 3 | 5 | 10 | | 60 | | 90 | |
| | % | % | % | $Log_{10}$ | % | $Log_{10}$ | % | $Log_{11}$ |
| **2** | 37.6 ± 1.9 | 26.6 ± 12.5 | 61.5 ± 1.9 | 0.41 ± 0.02 | 100 | 5.35 | 100 | 5.35 |
| **12** | 39.8 ± 10.0 | 44.7 ± 1.9 | 62.8 ± 13 | 0.43 ± 0.01 | 100 | 5.35 | 100 | 5.35 |
| **14** | 22.6 ± 4.4 | 24.8 ± 10.0 | 26.6 ± 12.5 | 0.14 ± 0.07 | 100 | 5.35 | 100 | 5.35 |

Note: Inoculum concentration: $2.26 \times 10^5$ CFU/mL; all compounds were prepared at the concentration equivalent to of 15 ppm [Cl+]

Both compounds 2 and 12 gave around 62% reduction after 10 min of contact whereas only 26.6% reduction was achieved in the case of compound 14. It seems compound 14 does show a slower kill than compounds 2 and 12. Since 60 min of contact is long enough for all three compounds to generate a total kill of *P. aeruginosa* (5 log), more contact durations were tested. The antibacterial dynamics of compounds 2, 12, 14, and 15, 16 are presented in Table 4 and Graph 1.

**Table 4: Antibacterial efficacy of Compounds 2, 12, 14, and 15, 16 against *P. aeruginosa* 73104**

| Cpds | Bacteria reduction at various contact times (min) | | | | | | | | | | | | | |
|---|---|---|---|---|---|---|---|---|---|---|---|---|---|---|
| | 3 | | 5 | | 10 | | 20 | | 30 | | 45 | | 60 | |
| | % | Log | % | Log | % | Log | % | Log | % | Log | % | Log | % | Log |
| **2** | 37.6± 1.9 | 0.2 | 26.6± 12.5 | 0.14 | 61.5± 1.9 | 0.41 | 99.65 | 2.46 | 99.99 | 4.02 | 99.99 | 4.67 | 100 | 5.47 |
| **12** | 39.8± 10.0 | 0.22 | 44.7± 1.9 | 026 | 62.8± 1.3 | 0.43 | 99.70± 0.1 | 2.50 | 100 | 4.82 | 100 | 5.47 | 100 | 5.47 |
| **14** | 22.6 ±4.4 | 0.11 | 24.8± 10.0 | 0.13 | 26.6± 12.5 | 0.14 | 70.8± 5.2 | 0.54 | 99.96± 0.02 | 3.39 | 100 | 5.47 | 100 | 5.47 |
| **15** | 75.7 ±22.6 | 0.74 | 98.4± 1.4 | 1.88 | 99.9± 0.05 | 3.02 | 99.97± 0.05 | 4.32 | 100 | 5.47 | 100 | 5.47 | 100 | 5.47 |
| **16** | 99.6 ±0.4 | 2.62 | 99.97±0.05 | 432 | 100 | 5.47 | 100 | 5.47 | 100 | 5.47 | 100 | 5.47 | 100 | 5.47 |

Note: Inoculum concentration: 2.26-2.98 × $10^5$ CFU/mL; all compounds were prepared at the concentration equivalent to of 15 ppm [Cl+]

It can be clearly seen that compound 14 shows the slowest kill profile among all the tested compounds: <1 log reduction with 20 min of contact. It seems diffusion of all the biocides through the aqueous solution onto the cell surface is not a rate limiting step in the inactivation process. So, the charge density in the molecule might not play a critical role in the killing dynamics. Instead, the size of the molecules for compounds 2, 12, and 14 is important in their interaction with a Gram-negative bacterium like *P. aeruginosa* (the smaller the better to get through the outer membrane). However, the size of molecules might not be a factor versus a Gram-positive organism with no outer membrane. That is why no obvious difference was observed for compounds 2, 12, and 14 in their killing dynamics against MRSA. Surprisingly, the bulk molecule 15 kills *P. aeruginosa* faster than all N-chloramine compounds 2, 12, and 14. The long alkyl chain quaternary ammonium cation can punch holes in cell membranes to cause leach of cytoplasm and at the same time allow the N-chloramine component to exert oxidative stress inside the cell.

**Graph 1: Percent of bacterial reduction vs contact time with compounds 2, 12, 14, and 15, 16**

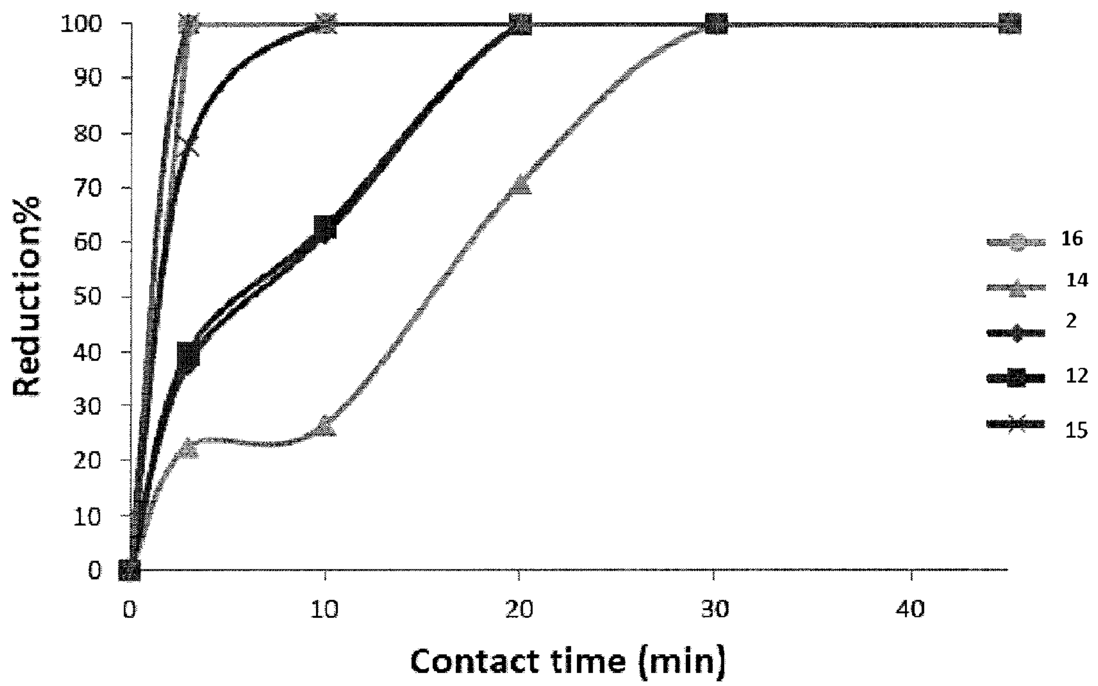


Compounds 15 and 16 were also challenged with MRSA and the results are listed in Table 5.

**Table 5. Antibacterial efficacy of 15 and 16 against CA-MRSA 40065**

| Molecules | Bacteria reduction at various contact times (min) | | | | | | | | | | | | |
|---|---|---|---|---|---|---|---|---|---|---|---|---|---|
| | 3 | | 5 | 10 | | 20 | | 30 | | 45 | | 60 | |
| | % | % | Log | % | Log | % | Log | % | Log | % | Log | % | Log |
| **15** | 86.9 | 79.5 | 0.69 | 85.1 | 0.83 | 67.8 | 0.49 | 79.8 | 0.70 | 92.6 | 1.13 | 87.3 | 0.89 |
| **16** | 84.4 | 93.4 | 1.18 | 99.8 | 2.77 | 100 | 6.45 | 100 | 6.45 | 100 | 6.45 | 100 | 6.45 |

Note: Inoculum concentration: 2.83 × $10^6$ cfu/mL; all compounds were prepared at the concentration equivalent to of 15 ppm [$Cl^+$]

Compound 16 had a kill profile of >1 log reduction within 5 minutes and compound 15 of around 80% reduction independent of contact duration (3 - 60 minutes). Compound 15 doesn't kill as fast as compounds 2, 12, and 14 probably because its long alkyl chain is trapped in one bacterial cell and can not exert further kill on other bacterial cells. So the reduction doesn't progress with the extension of contact duration. In other words, the kill capacity of compound 15 is overwhelmed by the large amount of bacteria in the solution (2.83 × $10^5$ cfu/mL x 20 mL). Compound 16 still possesses a faster killing dynamics than compound 2, 12, and 14 implying a possible synergistic bactericidal activitiy between N-chloramine and long alkyl chain quaternary ammonium cation.

Compound 16 has better antibacterial efficacy than both compounds 15 and 12. N-chloramine and long alkyl-chain quaternary ammonium salt might exert synergistic bactericidal action in solution.

**EXAMPLE 19: ANTIBACTERIAL ACTIVITY OF CATIONIC ANALOGS OF N-HALAMINE - COMPOUNDS 5, 6, 7, 8, AND 10**

The antibacterial activity of Compounds 5, 6, 7, 8, and 10 was tested.

***Test Cultures:***

*Pseudomonas aeruginosa* (*P. aeruginosa*) (#73104, Gram-negative) and *Staphylococcus aureus* (MRSA) (# 40065, Gram-positive) were used as the model microorganism to challenge the antibacterial function of the compounds. It should be noticed that both *P. aeruginosa* and MRSA are biosafety level 2 microorganisms and potentially biohazardous, therefore the following antibacterial assessments were carried out in a Biological Safety Level 2 cabinet and safety precautions were strictly followed.

***Test Compounds:***

Compounds 5, 6, 7, 8, and 10 were tested using the methodology described below.

***Methods:***

Tryptone Soya agar plates were used as platforms for bacterial cell growth and were prepared following the instructions on the bottle (CM 0131, OXOID). The prepared agar was kept at 65°C after being autoclaved and the resulting agar plates were stored in fridge at 3-4°C. All glassware and related materials were subjected to autoclave or disinfection with 70% ethanol prior to use.

Several colonies of each bacteria type were suspended in broth solutions (cation-supplemented Mueller-Hinton broth for *P. aeruginosa* and tryptic soy broth for MRSA) whose concentrations were equivalent to a 0.5 McFarland standard ($1\times10^8$ cfu/ml). This suspension was then diluted 1:100 and 20 μL of the diluted suspension was further diluted in 60 ml of cation-supplemented Mueller-Hinton broth or tryptic soy broth. Then the prepared bacteria inoculums were incubated overnight at 37°C to obtain logarithmic-phase cultures. For each microbial study, 0.2ml (0.02 ml for *P.aeruginosa*) of the cell suspension was diluted in 19.8 mL (19.98 ml for *P.aeruginosa*) of phosphate-buffered Saline (PBS, 0.1 M Sodium phosphate monobasic, 0.1 M Sodium phosphate dibasic, pH 7.4) to give a cell concentration of $10^6$-$10^7$ cfu/mL($10^5$ cfu/ml for *P.aeruginosa*). 30 μL of each synthesized compound solution (0.28 N stock solution) were added into the cell suspension to achieve a [$Cl^+$] of 15 ppm and start timing instantly. The mixture was vortexed several times during the reaction. After contact for the desired time intervals, 1.0 ml cell suspension was withdrawn and added to 1.0 ml of 0.02 N sodium thiosulfate and/or Letheen (1% lecithin, 10% peptone and 0.5% tween 80 dissolved in PBS at pH 7.4) to quench the bactericidal effect. The quenched suspension was then serially diluted (10 times less concentrated than the previous one) and 100 μL of each dilution was placed onto agar plates. The same procedure was applied to the blanks as controls with the same matrices but with no synthesized compounds added. Bacterial colonies on the agar plates were enumerated after being incubated at 37°C for 22 hours.

$$\text{Percentage reduction of bacteria (\%)} = (A\text{-}B)/A \times 100$$

$$\text{Log (reduction)} = \log(A/B)$$

Where A is the number of bacterial colonies in the control (cfu/mL), and B is the number of bacteria colonies under the effect of the synthesized compounds.

***Results:***

We further investigated the effect of length of alkyl chain associated with the cationic QAC center on the antibacterial action. The antibacterial efficacy of dodecyl and hexyl alkyl chain QAC without DMH moieties on polymer substrates or silica nanoparticles have been studied by other research groups with both exhibited antibacterial efficacy. Dodecyl and hexyl alkyl chain QAC DMH analogues were both synthesized in this study and are referred to as compound

**7** and **10,** respectively. Unlike other DMH analogues prior to chlorination, compound **7** demonstrated antibacterial activity to some extent against MRSA but excellent potency against *P.aeruginosa* probably due to the difference in the starting concentrations (Table 10).

Hexyl associated QAC has shown no bactericidal effect at all before chlorination and poor activity after chlorination compared to dodecyl associated QAC. The difference in killing kinetics between the hexyl and dodecyl alkyl chain was due to the different inactivation mechanisms. The mode of action of dodecyl involved membrane damage which is a faster process compared to that of hexyl which acted predominantly through inhibition of DNA functions. There is no difference in the killing kinetics before and after the chlorination for compound **7** against *P. aeruginosa* and no difference was seen neither after we increased the starting concentration from $10^5$ cfu/ml to $10^6$ cfu/ml. However, a significant change in the killing kinetics after chlorination for compound **7** against MRSA is evident. The results indicate that gram-negative *P.aeruginosa* is more sensitive towards the dodecyl alkyl chain than the gram-positive MRSA which might attribute to the thicker peptidoglycan layer outside of the cell membrane in the gram-positive bacteria cells.

**Table 10. Antibacterial efficacy of compounds 5, 6, 7, 8, and 10 against *P. aeruginosa* (73104) and methicillin resistant *Staphylococcus aureus* (MRSA 40065) at various contact times.**

| Bacteria | Synthetic Compound | Bacteria Reduction at Various Contact Time (min) | | | | | | | | | |
|---|---|---|---|---|---|---|---|---|---|---|---|
| | | 1 | | 3 | | 5 | | 10 | | 60 | |
| | | % | $log_{10}$ | % | $log_{10}$ | % | $log_{10}$ | % | $log_{10}$ | % | $log_{10}$ |
| Gram-positive MRSA | 5 | No bactericidal effect | | | | | | | | | |
| | 6 | 78.3 | 0.66 | 78.9 | 0.67 | 82.8 | 0.76 | 89.3 | 1.00 | 99.2 | 2.11 |
| | 7 | 0 | 0 | 0 | 0 | 85.6. | 0.85 | 99.7 | 2.59 | 100 | 6.26 |
| | 8 | 26.1 | 0.13 | 100 | 6.26 | 100 | 6.26 | 100 | 6.26 | 100 | 6.26 |
| | 10 | 81.7 | 0.74 | 76.7 | 0.63 | 68.2 | 0.50 | 84.5 | 0.81 | 95.3 | 1.33 |
| Gram-negative *P.aeruginosa* | | 3 | | 10 | | 20 | | 30 | | 60 | |
| | 5 | No bactericidal effect | | | | | | | | | |
| | 6 | 42.7 | 0.24 | 49.3 | 0.30 | 68.6 | 0.50 | 97.0 | 1.52 | 100 | 5.80 |
| | 7 | 100 | 5.80 | 100 | 5.80 | 100 | 5.80 | 100 | 5.80 | 100 | 5.80 |
| | 8 | 100 | 5.80 | 100 | 5.80 | 100 | 5.80 | 21.4 | 5.80 | 100 | 5.80 |
| | 10 | N/A | N/A | 55.1 | 0.35 | 100 | 5.27 | 100 | 5.27 | 100 | 5.27 |

Note: Compounds had concentrations equivalent to $[Cl^+]$ of 15 ppm; inoculum of MRSA was $10^6$ cfu/ml; inoculum of *P.aeruginosa* was $10^5$ cfu/ml. N/A denotes data was not available.

It was also found that a synergistic effect might exist against gram-positive MRSA by combining the dodecyl QAC and the chlorinated DMH if the bactericidal activity of compounds **2, 7, 8** and the dodecyl QAC by itself were compared. The log reduction of MRSA within 3 minutes given by compounds **2** and **7** are 1.14 (see Table 2) and 0, respectively and the sum of which give much less potency than that displayed by compound **8** alone. Even though no difference in the killing kinetics was observed between the non-chlorinated **(7)** and chlorinated forms **(8)** against *P. aeruginosa* at the bacterial concentration of $10^5$ or $10^6$ cfu/mL because *P.aeruginosa* is more vulnerable to the quaternary ammonium salt **7,** the difference might be detected if the starting inoculum concentration of *P.aeruginosa* increases further to $10^7$ - $10^8$ cfu/mL.

The proposed antibacterial mechanism of the possible synergistic effect is thought to proceed in three steps (Scheme 1). The first step is hole formations caused by the long alkyl chain then followed by the penetration of the whole molecules into the bacteria cells; the accumulation of QACs and transfer of oxidative chlorine to the biological receptors might confer compound **8** an enhanced antibacterial effect.

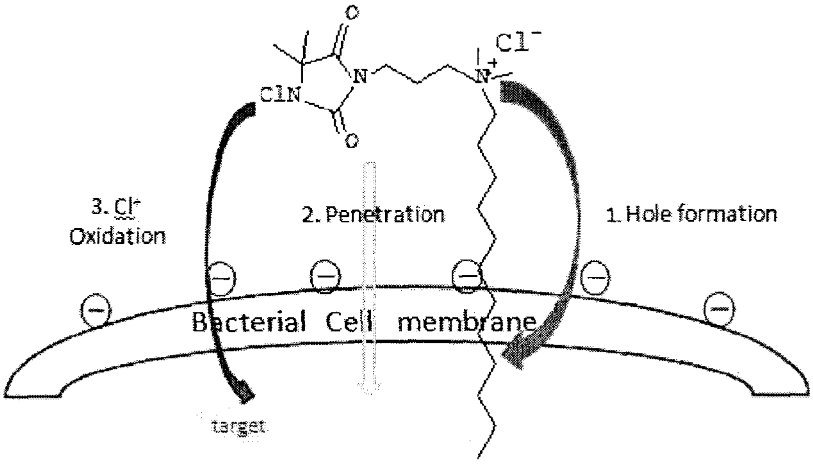


Proposed mode of action of possible synergistic effect exerted by dodecyl QAC linked DMH analogues.

***Observations***

The antibacterial activity of the synthesized compounds against gram-positive MRSA and gram-negative *P.aeruginosa* as a function of the quantity ratio of QAC to N-chloramine (DMH) was observed. It has been shown that compound with a ratio of 0.5 displayed the slowest killing kinetics but no significant difference was observed between the ratios of 1 and 2. The antibacterial activity was greatly enhanced by the attachment of a dodecyl QAC to the N-chloramine whereas hexyl QAC linked DMH exhibited no noticeable increased activity. A synergistic effect might exist by linking one dodecyl QAC to the N-chloramine.

**EXAMPLE 20: IMMOBILIZATION OF "CLICKABLE" DERIVATIVES ON PET AND COTTON - DERIVATIVES 29 AND 30**

Clickable derivatives were grafted onto PET and cotton.

***Preparation of Substrate (PMBAA-PET and PMBAA-g-cotton)***

Attachment of Derivatives 29 and 30 on a PET surface was completed by forming an interpenetrating network (IPN) of poly(MBAA) ((PMBAA), **Fig. 1)** on PET surface (named as PMBAA-PET) (Li et al., Polymer 53 (2012) 67-78).

To bind the synthetic azido derivatives on cotton fabrics, PMBAA was first grafted onto cotton (termed as PMBAA-g-cotton) via potassium persulfate (PPS) initiated radical grafting polymerization to present surface alkynyl groups **(Fig. 1).**

To the solution of monomer MBAA (1.92 g, 14 mmol) in mixed solvent (acetone 8 mL + DI water 32 mL) was added initiator potassium persulfate (PPS, 0.43 g, 1.6 mmol). After the initiator was dissolved completely, a piece of cotton fabric (10 × 10 cm) was dipped in the resulting solution and padded twice at a required expression (150% wet pcikup). The padded fabric was dried at 60°C for 10 min, cured at 105°C for 30 min, and then washed with copious amounts of water. The fabric was then extracted with MeOH in a Soxhlet-extractor for 24 h to remove ungrafted monomer and homopolymer. Afterwards, the fabric was air dried and stored in desiccator for 24 h to reach a constant weight. The resultant modified fabric was referred to as "PMBAA-*grafted*-cotton" (PMBAA-g-cotton). Percentage graft was calculated according to the following equation:

$$\text{Graft Percentage (\%)} = (W_2 - W_1)/ W_1$$

where $W_1$ and $W_2$ are the weights of the original and grafted fabrics, respectively.

In the attenuated total reflectance (ATR) spectrum of PMBAA-g-cotton **(Fig. 2(a))**, a new peak appeared at 1647 $cm^{-1}$ characteristic of carbonyl stretch C=O of amide in PMBAA. N-H stretching gave rise to a broad peak centered at 3421 $cm^{-1}$ in the spectrum of PMBAA-g-cotton **(Fig. 2(a)).** The ATR results implied successful grafting of PMBAA onto cotton. To visualize the distribution of PMBAA on cotton, we attached 2-azidoethyl 5-(dimethylamino)naphthalene-1-sulfonate (ADNS), an azido fluorescent dye previously synthesized in our research group, onto PMBAA-g-cotton using the "click" chemistry method shown below (denoted as PMBAA-g-cotton-ADNS).

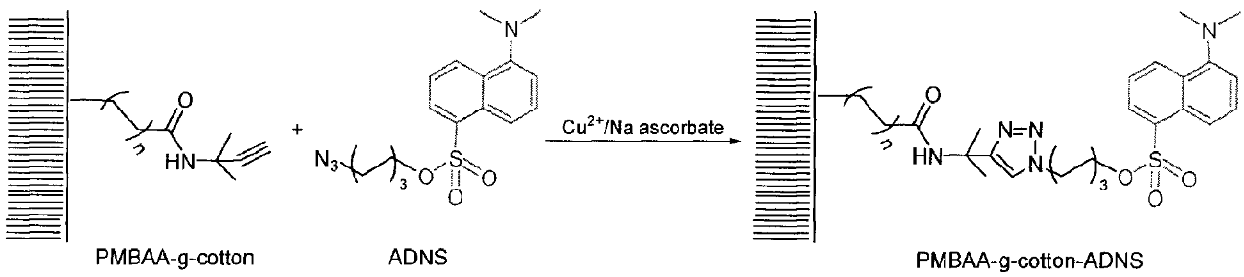


PMBAA-g-cotton ADNS PMBAA-g-cotton-ADNS

Attachment of dansyl-azide (ADNS) using "click" reaction

The protocol for the click reaction was the same as that for immobilization of 44, 29, 30, and 45. The untreated cotton was also submitted to this reaction process for 1 h serving as control. After "click" reaction, both PMBAA-g-cotton and untreated cotton were rinsed thoroughly until no green fluorescence was observed on the control fabric.

As shown in **Fig. 3,** uniform green fluorescence was observed on PMBAA-*g*-cotton-ADNS whereas only blue auto-fluorescence of cotton appeared on the control sample, which indicated the surface PPS induced grafting polymerization was successful and the alkynyl groups were uniformly distributed on cotton surface.

***Attachment ("Click" Linkage) Between Derivatives and PMBAA-PET and PMBAA-g-cotton***

Once the PMBAA modified substrate (PMBAA-PET or PMBAA-g-cotton) was obtained, "click" reaction between synthetic precursors and PMBAA-PET were performed following a previously reported protocol (Li et al., Polymer 53 (2012) 67-78), and the synthetic azide derivatives were covalently bonded onto PMBAA-g-cotton in a similar way.

PMBAA-g-cotton fabric (1.2 g, grafting percentage = 1.1%) was first immersed in 20 mL mixed solvent solvent (*t*-BuOH/$H_2O$ = 1:1) containing equiv. amount of synthetic azide derivatives (calculated based on totally PMBAA on grafted cotton). Then Na ascorbte (40% mol) and $Cu^{2+}$ (10% mol) were added to initiate the click reaction. After 1 h shaking, the cotton fabric was taken out and washed thoroughly with DI water and MeOH. The rinsed cotton was then air dried overnight and stored in desiccators until use. The obtained fabrics that endowed with specific precursors were named as PMBAA-PET-(**44, 29, 30, 45**) or PMBAA-*g*-cotton-(**44**, **29**, **30, 45**). Where Derviatives 44 and 45 are as follows:

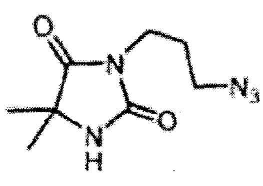

**44**

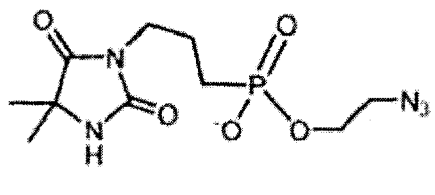

**45**

**EXAMPLE 21: ACTIVATION OF MODIFIED PET AND COTTON - DERIVATIVES 29 AND 30**

After the covalent immobilization, all "click" modified PET and cotton fabric were chlorinated to convert the clicked derivatives into corresponding N-chloramines, thereby activating their biocidal function. Both "click" modified PET and cotton fabric were chlorinated with sodium hypochlorite solution in a solid/liquid ratio of 1:50 (w/w). The concentration of the chlorinating solution varied from 15 ppm to 1500 ppm as needed. After continuous shaking for 30 min, the samples were thoroughly rinsed with DI water and then air dried overnight for titration analyses or antibacterial tests.

Virtually similar level of active chlorine on modified PET was obtained by adjusting available chlorine of the chlorinating NaClO solution. However, since cotton fabric is hydrophilic, a small change of available chlorine in the chlorination solution can result significant variation of active chlorine on the modified cotton samples.

Therefore, we studied the chlorination kinetics of those "click"-modified cotton fabrics.

Based on the previous study (Li et al., Ind. Eng. Chem. Res. 48 (2009) 613), the chlorination reaction could be regarded as in first-order relationship with the amide concentration according to equation 1:

$$v = -d[\text{amide}]/dt = k\,[\text{NaClO}][\text{amide}] \qquad (1)$$

where *v* is the chlorination reaction rate, *k* is the rate constant and t is the reaction duration.

Since NaClO for chlorination is in excess, *k*[NaClO] can be regarded as constant *k'*. Integration of equation 1 gives equation 2:

$$\ln\{[\text{amide}]_t/[\text{amide}]_0\} = -k't \qquad (2)$$

where $[\text{amide}]_t$ is amide concentration at the reaction time of t, $[\text{amide}]_0$ is the total amide of hydantoin on cotton (which can be caculated from the graft percentage 1.1%) and *k'* = *k* [NaClO]. The yield of click linkage reaction was regarded as 100%, and t was 1800 s. Therefore, based on the obtained active chlorine levels when the available chlorine ([NaClO]) was between 500 ppm and 2400 ppm (Graph 2), the *k'* in the equation 2 could be calculated as shown in Table 6.

**Graph 2: Chlorination progress versus available chlorine in NaClO solution. Reaction duration: 30 min.**

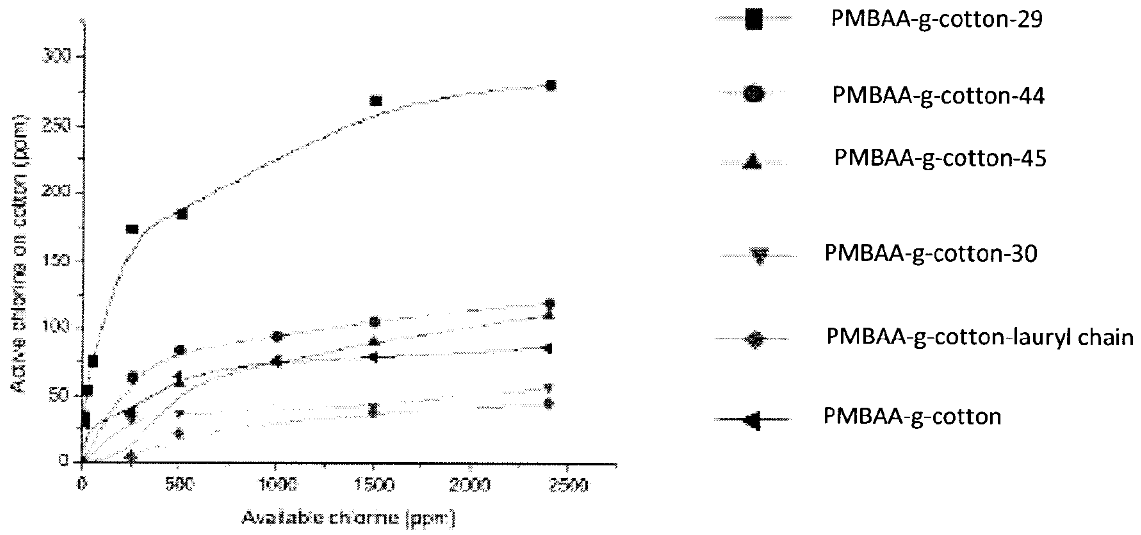


**Table 6. Rate constant (*k*) for the chlorination of modified cotton samples.**

| Modified cotton | PMBAA-g-cotton | PMBAA-g-cotton-44 | PMBAA-g-cotton-29 | PMBAA-g-cotton-30 | PMBAA-g-cotton-45 |
|---|---|---|---|---|---|
| Rate constant (*k*, $L.mol^{-1}.s^{-1}$) | $7\times10^{-5}$ | $1\times10^{-4}$ | $4\times10^{-4}$ | $7\times10^{-5}$ | $2\times10^{-4}$ |

*k* of PMBAA-g-cotton-29 (k(29)) was the highest among all the samples. The chlorination of PMBAA-g-cotton-29 proceeded at a much higher rate due to the attraction between the positive charge in 29 and the negatively charged chlorination species $ClO^-$. However, the similarly positively charged PMBAA-g-cotton-30 had only a comparable *k* and even lower active chlorine loadings than PMBAA-g-cotton amide bond of which could also be converted to N-chloramine (as shown in Graph 2). It was probably due to increased hydrophobicity of PMBAA-g-cotton-30 and that the steric

hindrance of the introduced dodecyl chain impedes the formation of hydrogen bond between amide hydrogen and hypochlorite oxygen which has been proposed to be the transition state of the chlorination of amides. To test this hypothesis, we subsequently prepared lauryl azide and attached the long chain azide onto PMBAA-g-cotton via the "click" chemistry method. The active chlorine loading on the obtained cotton sample, termed as PMBAA-g-cotton-lauryl chain, was also plotted as a function of the available chlorine of the sodium hypochlorite solution (Graph 2). The active chlorine loadings on PMBAA-g-cotton-lauryl chain were lower than both PMBAA-g-cotton and PMBAA-g-cotton-30 over the full range of available chlorine (250-2500 ppm). This confirmed that the long alkyl chains retard the chlorination of either acyclic amide of PMBAA or the cyclic amide of DMH. In addition, it is notable that total active chlorine loadings on PMBAA-g-cotton-29 were more than double of that of all other modified cotton fabrics when the available chlorine was greater 500 ppm, meaning that the positive charge center contributed to not only faster chlorination but also higher equilibrium active chlorine loading.

Interestingly, the cationic charged center was found to positively contribute to both chlorination kinetics and equilibrium active chlorine loading on modified cotton samples. These findings provide foundational guidelines for the design and synthesis of novel biocides with more potent broad-spectrum antibacterial activity.

This work also presents clinical application importance since better antibacterial efficacy could result from cotton and PET fabrics with less active chlorine loadings, minimizing the concern of such adverse effects as skin irritation when the fabrics are used in healthcare settings for decreasing cross-infection. Equally important, the ability of the modified cotton sample (PMBAA-g-cotton-29) in picking up positive chlorine atoms from very diluted sodium hypochlorite (10 ppm) will lessen the enviromental burden from using chlorine bleach for the activation of the biocidal property, hence allow the wider use of N-chloramine based biocides for battling infectious bacteria.

**EXAMPLE 22: ANTIBACTERIAL ASSESSMENT OF MODIFIED PET AND COTTON SAMPLES - DERIVATIVES 29 AND 30**

***Test Cultures:***

Antibacterial test for chlorinated PMBAA-PET-(**44, 29, 30, 45**) was carried out against a clinical isolate of MDR-*E. coli* (#70094) according to our previous report (Townsend et al., Med. J. Australia 2 (1983) 310). Antibacterial properties of chlorinated PMBAA-g-cotton-(**44, 29, 30, 45**) were examined against clinical isolates of MDR-*E. coli* (#70094) and HA-MRSA (#77090, healthcare-associated) respectively.

***Methods:***

The click-modified fabrics PMBAA-*g*-cotton-(**44, 29, 30, 45**) were first cut into four small pieces (diameter = 4.8 cm), two of which were put together in a sterilized container. Then 0.5 mL bacterial suspension ($10^6$-$10^7$ CFU/mL) was placed onto these two fabric surfaces, and sandwiched by another two portions of the identical fabrics. Immediately another 0.5 mL of bacterial suspension was dispensed on the entire fabric set. After the predetermined contact time, 100 mL of 0.03% sodium thiosulfate aqueous solution was added to the container to neutralize any active chlorine. The mixture was then vigorously shaken for 2 min followed by ultrasonic treatment for 5 min. An aliquot of the solution was removed from the mixture and then serially diluted and 100 $\mu$L of each dilution was placed onto a nutrient agar plate. The same procedure was also applied to the bleached untreated cotton and bleached PMBAA-g-cotton. Viable bacterial colonies on the agar plates were counted after incubation at 37 °C for 24 h. Bacterial reduction is reported according to the equation:

$$\text{Percentage reduction of bacteria (\%)} = (A - B)/A \times 100$$

$$\text{Log reduction} = \text{Log}(A/B)$$

Where A is the number of bacteria counted from bleached untreated cotton, and B is the number of bacteria counted from modified cotton fabrics.

In the case of PET, fabrics were cut into two smaller pieces (diameter = 2.4 cm). One of the pieces was put in a sterilized container and 60 $\mu$L of an aqueous suspension containing $10^7$ CFU/mL of *MDR-E.coli* was placed onto the surfaces of the fabric. The fabric was then "sandwiched" using another piece of identical fabric. A sterilized 50 mL beaker was placed onto the top of these two fabrics to ensure sufficient contact. After the contact for 5 min, the entire "sandwich" was placed into 10 mL of 1.0% sodium thiosulfate aqueous solution to quench the active chlorine on the fabrics. The resultant mixture was then vigorously shaken for 2 min before an aliquot (100 $\mu$L) of the solution was removed and then

serially diluted. 100 μL of each dilution were placed onto a nutrient agar plate. The same procedure was also applied to chlorinated untreated PET as control. Viable bacterial colonies on the agar plates were counted after incubation at 37 °C for 24 h. Bacterial reduction is reported according to the above equation.

Non-contact killing test was carried out by the following protocol. Chlorinated cotton and chlorinated PMBAA-g-cotton-29 were cut into small pieces and sealed in a nylon bag respectively. The bags containing cotton fabrics were immersed in 10 mL PBS (0.05 M, pH 7.0) and continuously shaken by vortex. At the predetermined time of 5 min and 10 min, 2.0 mL aliquots were taken out by a syringe equipped with a nylon filter membrane (0.45 μm, Fisher) and mixed with 0.5 mL bacterial suspension ($10^5$-$10^6$ CFU/mL). The mixture was left stand for 5 min before 12.5 mL 0.03% sodium thiosulfate aqueous solution was added to quench the "released" active chlorine. Afterwards, the bacterial suspension was serially diluted and 100 μL of each resulting dilutions were placed onto nutrient agar plates. After being incubated at 37 °C for 24 hours, viable bacterial colonies on the plates were counted.

***Results:***

***Chlorinated PMBAA-PET-(44, 29, 30, 45)***

We chose one MDR-*E.coli* strain (#70094) to challenge modified PET samples. Table 7 outlines the antibacterial results of "click"-modified PMBAA-PET against MDR-*E.coli* (#70094).

**Table 7. Antibacterial efficacy of PMBAA-PET after "click" linkage modification**

| Modified PET Sample | Active chlorine (ppm) | Reduction of MDR-*E.coli* (#70094)[a] | Contact angle (After chlorination) |
|---|---|---|---|
| PET | 0 | 0 | 122±8.1 |
| PMBAA-PET | 132±23 | 0 | 106.4±7.3 |
| PMBAA-PET-**44** | 427±19 | 46.4±0.5% | 90.8±5.6 |
| PMBAA-PET-**29** | 433±23 | 99.8±0.1% | UD[b] |
| PMBAA-PET-**30** | 434±25 | 23.2±2.5% | 107.1±4.1 |
| PMBAA-PET-**45** | 423±31 | 43.7±2.9% | 78.1±10.1 |

[a] Inoculum concentration was $1.02 \times 10^7$CFU/mL, % reduction after a contact time of 5 min.
[b] Undetectable (too hydrophilic to be detectable)

PMBAA-PET samples clicked with various hydantoin derivatives (***44, 29, 30,** and **45***) were loaded with similar amount of active chlorine (around 430 ppm). PMBAA-PET-29 showed the best antibacterial efficacy which might be due to the cationic charge in 29. However, only 23.2% bacterial reduction, the worst efficacy among all clicked samples, was achieved on PMBAA-PET-30 which possesses both N-chloramine and long chain QAC moieties. This was unexpected and intrigued us to conduct contact angle measurements. PMBAA-PET-30 is still quite hydrophobic with a contact angle of 107.1±4.1 degree, similar to PMBAA-PET. The surface energy of PMBAA-PET-30 sample is not high enough to cause the bacterial suspension to spread on its surface. In the antibacterial test, even a sterilized beaker was loaded on the top of the fabric assembly between which a bacterial suspension was sanwiched to help create an intimate contact, minute beads of the bacterial suspension might still exist on the hydrophobic surface hindering the contact killing process. For more hydrophilic samples such as PMBAA-PET-29, however, the bacterial suspension could spread over the surface immediately after being dispensed so that a sufficient contact with the immobilized biocides was ensured. Therefore, differences in the biocidal efficacies of all the samples are confounded by their differences in hydrophilicity and surface charges (negative, neutral, and positive). The sequence of bactericidal strength: PMBAA-PET-29 > PMBAA-PET-44 > PMBAA-PET-30 corresponds to their hydrophilicities as denoted by contact angles (undetectable, 90.8±5.6 and 78.1±10.1). Although we can clearly see that PMBAA-PET-29 demonstrates the most potent biocidal efficacy among all the samples, no convincing conclusion can be drawn about the effect of the cation center of PMBAA-PET-29 on its biocidal efficacy. To eliminate the effect of substrate hydrophobicity on the antibacterial efficacy, derivatives 44, 29, 30, and 45 grafted onto hydrophilic cotton substrate were tested.

***PMBAA-g-cotton-(44, 29, 30, 45)***

***Gram-negative Activity***

Given enough contact time (120-180 mins), the cotton fabrics with active chlorine as low as 48 ppm resulted

in a 5 log reduction of k-12 *E. coli* (Li et al., Ind. Eng. Chem. Res. 48 (2009) 613). Differences in the antibacterial efficacies of the cotton samples may not be distinguishable if long time contact was allowed. Also, according to the model study, cationic charge center majorly contributes to a rapid kill of bacteria. Thus, short time contact (i.e. 5 min) was adopted in the antibacterial test. Only negligible percent reduction of MDR-*E.coli* (#70094) was observed on PMBAA-g-cotton sample within 5 mins of contact (Table 8).

**Table 8. Antibacterial efficacy of modified cotton fabrics against *MDR-E.coli* #700094**

| Modified cotton Sample | Active chlorine (ppm) | Reduction of MDR-*E. coli* (#70094)[a] | |
|---|---|---|---|
| | | Percentage reduction | $Log_{10}$ reduction |
| Cotton | 0 | 0 | 0 |
| PMBAA-g-cotton | 51±5 | 5.1±0.8% | 0.02 |
| PMBAA-g-cotton-**44** | 120±8 | 22.2±3.3% | 0.11 |
| PMBAA-g-cotton-**29** | 152±12 | 89.7±3.3% | 1 |
| PMBAA-g-cotton-**29** | 35±3 | 37.8±5.3% | 0.21 |
| PMBAA-g-cotton-**45** | 107±2 | 18.9±3.3% | 0.09 |
| PMBAA-g-cotton-**30** | 55±6 | 28.3±3.4% | 0.14 |

[a] Inoculum concentration was $2.12 \times 10^6$ CFU/mL and contact time was 5 min.

This finding accords with previous findings that t-Butyl acrylamide grafted cotton could neither be easily chlorinated nor demonstrate effective biocidal efficacy (Li et al, Ind. Eng. Chem. Res. 48 (2009) 613). It is because that the methyl substitution adjacent to N-Cl structure impedes effective chlorine transfer from N-Cl biocide to biological receptors on bacteria. As shown in Table 8, the biocidal efficacy of PMBAA-g-cotton-44 was around half of that of PMBAA-g-cotton-29 (with 35±3 ppm active chlorine) even when the latter's active chlorine was much lower (120 vs. 35 ppm). This confirmed the boosting effect of cationic charge center on the biocidal efficacy of N-chloramine. PMBAA-g-cotton-45 only gave comparable efficacy as PMBAA-g-cotton-44, indicating negligible or no contribution to the biocidal effect from negative charge.

Considering the significantly enhanced bactericidal activity of chlorinated PMBAA-g-cotton-29, we proposed the possible boosting mechanism as depicted in **Fig. 4.** *E.coli* cells are covered with a lipopolysaccharide layer of 1-3 μm thickness and hence negatively charged. The negatively charged shell can be arrested by the cation in 29 through electrostatic interaction to facilitate the oxidative chlorine transfer from N-chloramine to cell biological receptors leading to bacterial death. Even at half of the active chlorine on PMBAA-g-cotton-44 or 45, PMBAA-g-cotton-30 showed comparable biocidal efficacy, if not better, within 5 mins of contact. However, compared with PMBAA-g-cotton-29, the boosting effect is less significant. It is deduced that the long alkyl chain shields the electrostatic interaction between the cationic center on N-chloramine structure and negatively charged *E. coli* cells. The contact time was too short for the QAC moiety to complete the "bubble bursting" action. Under the experimental condition, no synergist bacterial killing is found between the antibacterial QAC and the N-chloramine even when they are covalently bonded with each other.

The finding that a cationic charge center can boost the biocidal efficacy of N-chloramine is of application importance. Even though previous research has shown that dimethyloldimethyl hydantoin-treated cotton fabrics with an active chlorine loading of 1100 ppm did not generate any erythema or edema on the bare skin of 8-week-old New Zealand male rabbits after 4-hour skin contact, more evidence is needed about the safety and tolerability of N-chloramine modified fabrics before they can be used in close contact with skin. In this context, it is desirable to present more potent antibacterial activity with lower active chlorine loading as in the case of PMBAA-g-cotton-29. It is noteworthy that 33 ppm active chlorine on PMBAA-g-cotton-29 was achieved using a NaClO chlorinating solution with only 10 ppm available chlorine, which is of the similar level as in public swimming pools (2-5 ppm). It implies that the biocidal function of PMBAA-g-cotton-29 can be easily activated to become self-disinfecting and useful in such settings as surgical gown, nurse uniform and hospital privacy curtain etc.

### *Confirmation of Killing Mechanism*

As the dissociation constant of amide N-chloramine is less than $10^{-9}$, (Qian et al., J. Appl. Polym. Sci. 89 (2003) 2418) its biocidal function is believed to proceed in a direct contact manner (Williams et al., Appl. Environ. Microbiol. 54 (1988) 2583). As for the N-Cl form of 29, either free or after immobilization, the nature of N-Cl is identical to that of 44. To further confirm the on-contact killing mechanism as depicted in **Fig. 4,** we designed a non-contact killing test.

Chlorinated cotton and chlorinated PMBAA-g-cotton-29 were first suspended in PBS (0.05 M, pH 7.0) under vortex conditions for 5 and 10 minutes. Then the extration buffer was filtered through a syringe filter membrane and

added to a bacterial suspension. Viable bacterial colonies were counted to obtain almost constant bacterial concentrations as shown below.

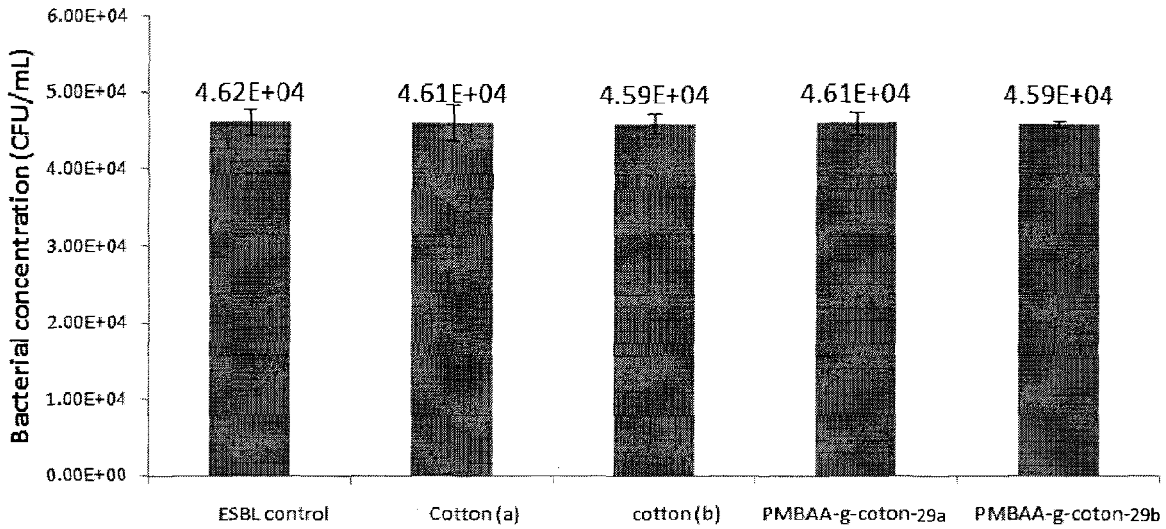


MDR-E.coli bacterial concentration after contact with soaking solution of corresponding cotton samples. (a) Cotton fabric was shaken in PBS for 5 min; (b) cotton fabric was shaken in PBS for 10 min.

No bacterial kill was observed when PMBAA-g-cotton-29 was not in direct contact with the bacterial suspension. It indicated that contact between N-chloramines and bacteria is indispensable for the microorganism inactivation, lending support to the proposed mechanism (**Fig. 4**), of enhanced bacterial kill of PMBAA-g-cotton-29.

***Gram-positive Activity***

These modified cotton fabrics were also challenged with a Gram-positive bacterium healthcare associated (HA)-MRSA #77090. As shown in Table 9, PMBAA-g-cotton-44 and PMBAA-g-cotton-45 gave similar percent reductions of the tested bacterium: 75.0% and 82.3%. Again, it indicated negligible contribution from the negative charge to N-chloramine's biocidal function. 6.3 log reduction was achieved by PMBAA-g-cotton-29 with 141±8 ppm active chlorine. The 76.5 % bacterial reduction of PMBAA-g-cotton-29 with the active chlorine loading of 33±5 ppm was comparable to that of PMBAA-g-cotton-44 and PMBAA-g-cotton-45, which possessed a little over twice active chlorine concentration (80±14 ppm and 84±1 ppm respectively).

**Table 9. Antibacterial efficacy of grafted cotton fabrics against MRSA #77090**

| Modified cotton Sample | Active chlorine (ppm) | Reduction of MRSA(#77090)[a] | |
|---|---|---|---|
| | | Percentage reduction | $Log_{10}$ reduction |
| cotton | 0 | 0.0% | 0 |
| PMBAA-g-cotton | 66±3 | 27.2±2.8% | 0.02 |
| PMBAA-g-cotton-44 | 80±14 | 75.0±1.7% | 0.6 |
| PMBAA-g-cotton-29 | 141±8 | 100.0% | 6.3 |
| PMBAA-g-cotton-29 | 33±5 | 76.5±3.7% | 0.63 |
| PMBAA-g-cotton-45 | 84±1 | 82.3±1.0% | 0.75 |
| PMBAA-g-cotton-30 | 59±4 | 26.0±3.7% | 0.11 |

[a] Inoculum concentration was $2.0 \times 10^6$ CFU/mL and contact time was 5 min.

Sonohara and co-workers (Sonohara et al., Biophys. Chem. 55 (1995) 273) studied the electrophoretic mobility of *E.coli* and *S.aureus* in mediums with a range of pHs and ionic strengths. Based on the mobility formula derived for biological cells by Ohshima and Kondo (Ohshima et al., J. Colloid Interface Sci. 130 (1989) 281), Sonohara extracted two parameters from the electrophoretic mobility results: charge density on the bacterial surface and resistance to liquid flow in the surface layer. Compared with *S.aureus,* the surfaces of *E.coli* cells are more negatively charged and more rigid, i.e. higher resistance to liquid flow in the surface layer. Since the number density of negative charges on *S.aureus*

cells (0.025 $m^{-3}$ at pH = 7) is much less than *E. coli* cells (0.145 $m^{-3}$ at pH = 7) (Sonohara et al., Biophys. Chem. 55 (1995) 273), the contribution of positive charge in the killing of *S. aureus* was not as obvious as in the case of *E. Coli.* The same reason accounts for less effective antibacterial performance of PMBAA-g-cotton-30. When the contribution of positive charge diminished, the negative impact of hydrophobic alkyl chain magnified its effect. So unlike the case of *E. coli* reduction, PMBAA-g-cotton-30 appeared even less effective than PMBAA-g-cotton-44 and -45 in inactivating MRSA.

Based on the antibacterial studies against MDR-*E.coli* and HA-MRSA, the same conclusion could be drawn that the cation in 29 contributed greatly to bacterial kill while anion in 45 did not, and no synergistic effect between antibacterial QAC and N-chloramine was found. The long alkyl chain in QAC, on the contrary, contributed negatively to the antibacterial efficacy.

The mechanism for the enhanced antibacterial activity was proposed as follows: through an electrostatic attraction of opposite charges, the cation in PMBAA-g-cotton-29 helps arrest negatively charged bacterial cells and hence facilitates the oxidative chlorine transfer from N-chlorohydantoin to cell biological receptors causing bacterial death (**Fig. 4**). Based on this hypothesis, it is possible that the antibacterial activity might be further enhanced if more than one cations are introduced to molecule 29. We believe that such new products together with 29 are good candidates to challenge biofilms, a prominent form of microbial life that may cause many chronic infections and environmental contamination.

**EXAMPLE 23: SYNTHESIS OF BRANCHED ANALOGS USING "CLICKABLE" DERIVATIVES - DERIVATIVES 40, 41, 42 AND 43**

a)

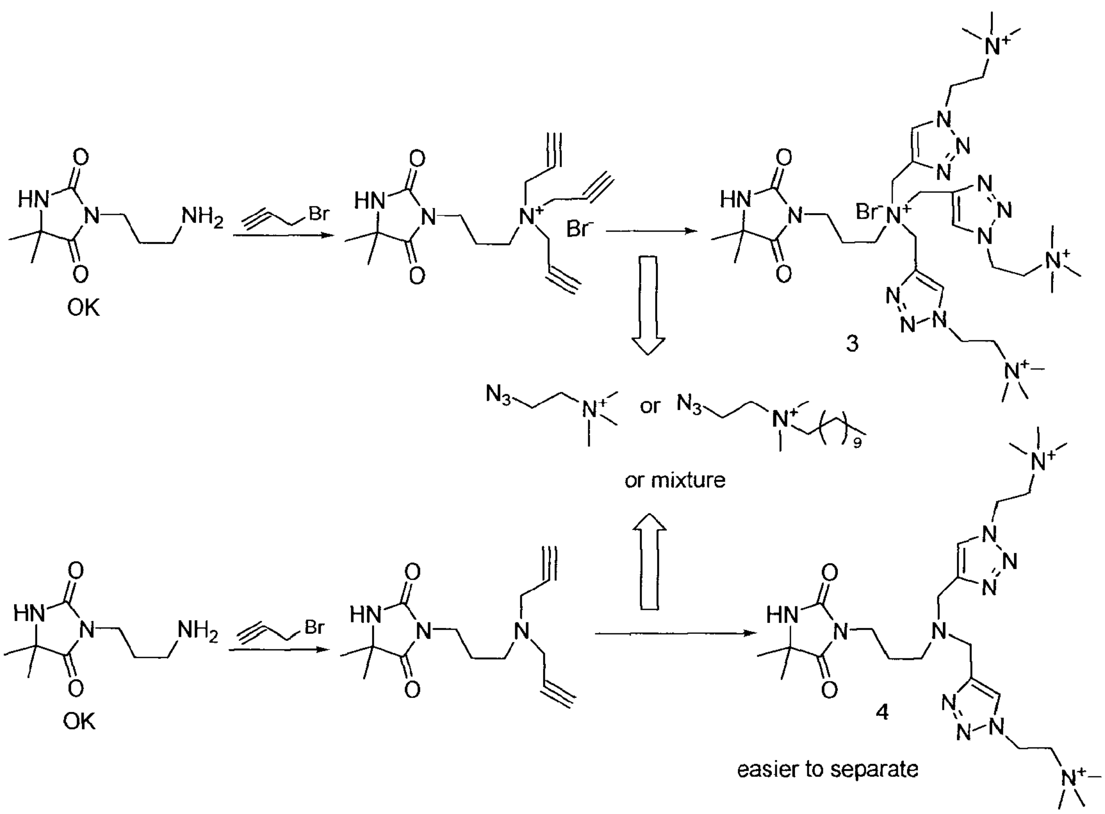

b)

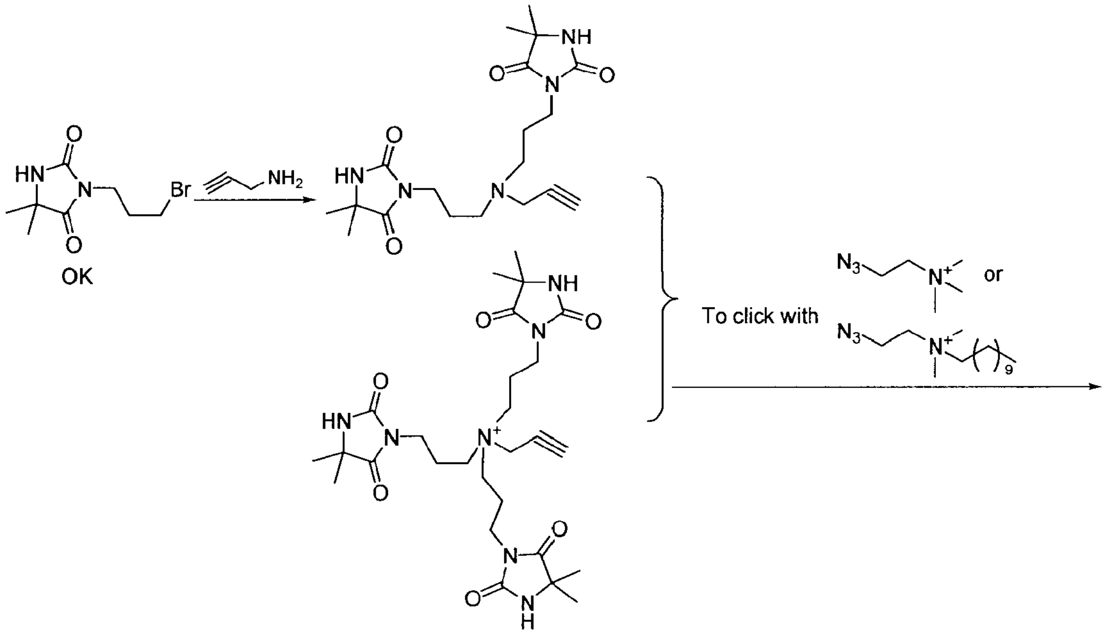


c)

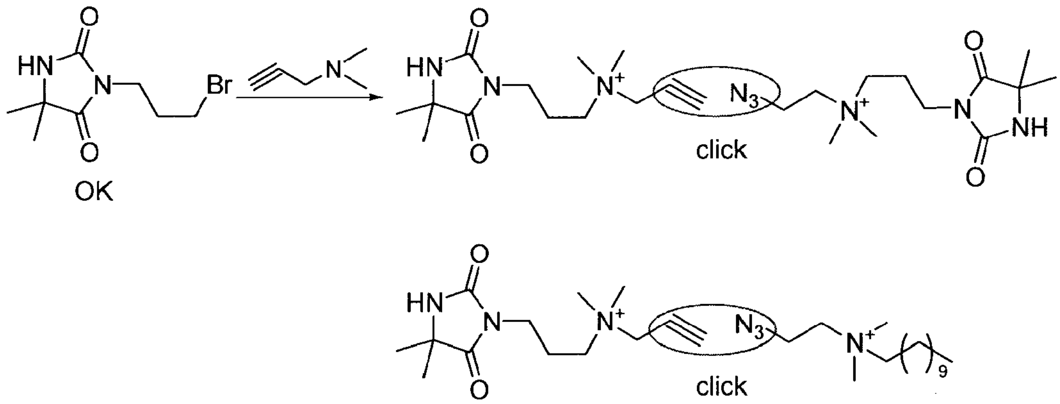

d)

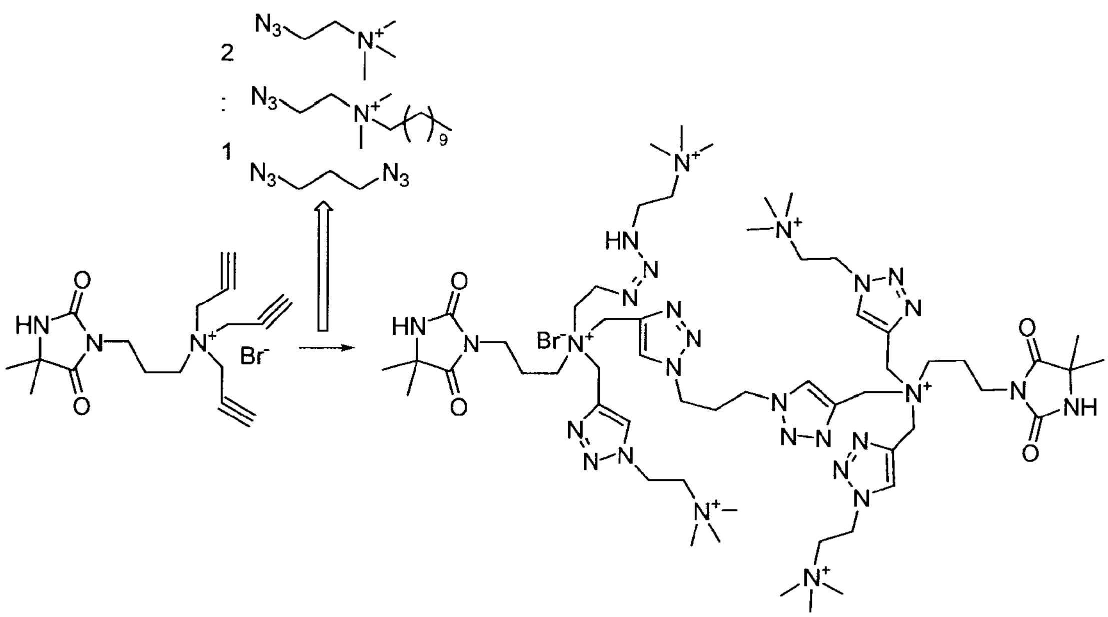

The disclosures of all patents, patent applications, publications and database entries referenced in this specification are hereby specifically incorporated by reference in their entirety to the same extent as if each such individual patent, patent application, publication and database entry were specifically and individually indicated to be incorporated by reference.

Although the invention has been described with reference to certain specific embodiments, various modifications thereof will be apparent to those skilled in the art without departing from the spirit and scope of the invention. All such modifications as would be apparent to one skilled in the art are intended to be included within the scope of the following claims.

The following numbered paragraphs also describe the invention. These paragraphs are not the claims. The claims follow on after the paragraphs.

1. A biocidal compound having general formula (I):

N-halamine-L-QUAT (I)

wherein:

the N-halamine may be a cyclic or acyclic N-halamine;

L is $C_1$-$C_6$ alkyl, cyclic aromatic or non-aromatic ring,

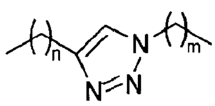

,

ether, ketone or any other organic linking structures, and QUAT has general formula (II):

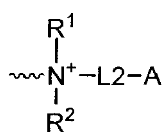

(II)

wherein:

$R^1$ and $R^2$ are each independently $C_1$-$C_6$ alkyl;
L2 is absent, $C_1$-$C_6$ alkyl or

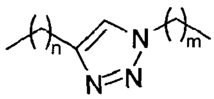 ;

A is $R^3$, N-halamine or $-N^+R^4R^5R^6$;
$R^3$ is $C_1$-$C_{18}$ alkyl;
$R^4$ and $R^5$ are each independently $C_1$-$C_6$ alkyl;
$R^6$ is $C_1$-$C_{18}$ alkyl or $-(CH_2)_pB$;
B is N-halamine;
n and m are each independently 1-6, and
p is 1-6,

and wherein

when A is $R^3$, L2 is absent, and

when A is N-halamine or $-N^+R^4R^5R^6$, L2 is $C_1$-$C_6$ alkyl or

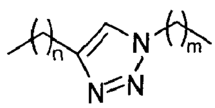

2. The compound according to paragraph 1, wherein the N-halamine is a cyclic N-halamine.

3. The compound according to paragraph 1, wherein each N-halamine is independently a cyclic N-halamine having general formula (III) or general formula (IV):

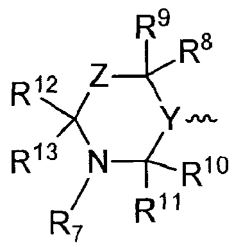

(III)

wherein:

Y is CH or N;
Z is absent, $CH_2$ or $NR^{23}$;
$R^7$ is halo;
$R^8$ and $R^9$ are each independently H, $C_1$-$C_4$ alkyl, or $C_1$-$C_4$ alkoxy, or $R^8$ and $R^9$ taken together form =O;
$R^{10}$ and $R^{11}$ are each independently H, $C_1$-$C_4$ alkyl, or $C_1$-$C_4$ alkoxy, or $R^{10}$ and $R^{11}$ taken together form =O; and
$R^{12}$ and $R^{13}$ are each independently H, $C_1$-$C_4$ alkyl, or $C_1$-$C_4$ alkoxy, or $R^{12}$ and $R^{13}$ taken together form =O, and
$R^{23}$ is H or halo,
wherein when Z is absent and $R^8$ and $R^9$ taken together form =O, $R^{12}$ and $R^{13}$ are each independently H, $C_1$-$C_4$ alkyl, or $C_1$-$C_4$ alkoxy;

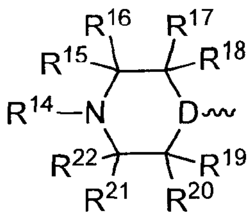

(IV)

wherein:

D is CH or N;
$R^{14}$ is halo;
$R^{15}$ and $R^{16}$ are each independently H, $C_1$-$C_4$ alkyl, or $C_1$-$C_4$ alkoxy, or $R^{15}$ and $R^{16}$ taken together form =O;
$R^{17}$ and $R^{18}$ are each independently H, $C_1$-$C_4$ alkyl, or $C_1$-$C_4$ alkoxy, or $R^{17}$ and $R^{18}$ taken together form =O;
$R^{19}$ and $R^{20}$ are each independently H, $C_1$-$C_4$ alkyl, or $C_1$-$C_4$ alkoxy, or $R^{19}$ and $R^{20}$ taken together form =O, and
$R^{21}$ and $R^{22}$ are each independently H, $C_1$-$C_4$ alkyl, or $C_1$-$C_4$ alkoxy, or $R^{21}$ and $R^{22}$ taken together form =O,
wherein when $R^{15}$ and $R^{16}$ taken together form =O, $R^{17}$ and $R^{18}$ are each independently H, $C_1$-$C_4$ alkyl, or $C_1$-$C_4$ alkoxy, and
wherein when $R^{21}$ and $R^{22}$ taken together form =O, $R^{19}$ and $R^{20}$ are each independently H, $C_1$-$C_4$ alkyl, or $C_1$-$C_4$ alkoxy.

4. The compound according to paragraph 3, wherein each N-halamine is a cyclic N-halamine having general formula (IV).

5. The compould according to paragraph 3, wherein each N-halamine is a cyclic N-halamine having general formula (III).

6. The compound according to paragraph 5, wherein:

Y is N, and
Z is absent or $NR^{23}$.

7. The compound according to paragraph 5, wherein
$R^1$ and $R^2$ are each $-CH_3$,
Y is N, and
Z is absent or $NR^{23}$.

8. The compound according to paragraph 5, wherein each cyclic N-halamine has general formula (V):

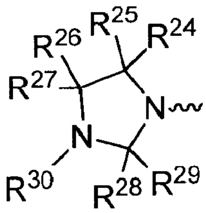

(V)

wherein:

$R^{24}$ and $R^{25}$ are each independently H, $C_1$-$C_4$ alkyl, or $C_1$-$C_4$ alkoxy, or $R^{24}$ and $R^{25}$ taken together form =O;
$R^{26}$ and $R^{27}$ are each independently H, $C_1$-$C_4$ alkyl, or $C_1$-$C_4$ alkoxy, or $R^{26}$ and $R^{27}$ taken together form =O;
$R^{28}$ and $R^{29}$ are each independently H, $C_1$-$C_4$ alkyl, or $C_1$-$C_4$ alkoxy, or $R^{28}$ and $R^{29}$ taken together form =O, and
$R^{30}$ is halo,

and wherein:

when $R^{24}$ and $R^{25}$ taken together form =O, $R^{26}$ and $R^{27}$ are each independently H, $C_1$-$C_4$ alkyl, or $C_1$-$C_4$ alkoxy.

9. The compound according to paragraph 8, wherein L is $C_1$-$C_6$alkyl.

10. A compound having general formula (VI):

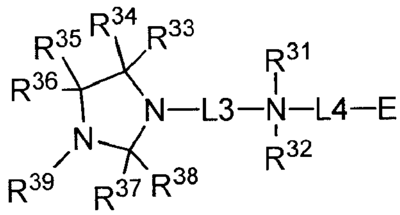

(VI)

wherein:

L3 is $C_1$-$C_6$ alkyl, cyclic aromatic or non-aromatic ring,

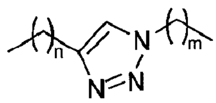

,

ether, ketone or any other organic linking structures,;
$R^{31}$ and $R^{32}$ are each independently $C_1$-$C_6$ alkyl;
L4 is absent, $C_1$-$C_6$ alkyl or

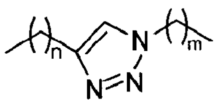

;

E is $R^{40}$, N-halamine of general formula (V) or $-N^+R^{41}R^{42}R^{43}$;
$R^{40}$ is $C_1$-$C_{18}$ alkyl;
$R^{41}$ and $R^{42}$ are each independently $C_1$-$C_6$ alkyl;
$R^{43}$ is $C_1$-$C_{18}$ alkyl or $-(CH_2)_pM$;
M is N-halamine of general formula (V);
n and m are each independently 1-6, and
p is 1-6,
$R^{33}$ and $R^{34}$ are each independently H, $C_1$-$C_4$ alkyl, or $C_1$-$C_4$ alkoxy, or $R^{33}$ and $R^{34}$ taken together form =O;
$R^{35}$ and $R^{36}$ are each independently H, $C_1$-$C_4$ alkyl, or $C_1$-$C_4$ alkoxy, or $R^{35}$ and $R^{36}$ taken together form =O;
$R^{37}$ and $R^{38}$ are each independently H, $C_1$-$C_4$ alkyl, or $C_1$-$C_4$ alkoxy, or $R^{37}$ and $R^{38}$ taken together form =O, and
$R^{39}$ is halo,

wherein

when E is $R^{40}$, L4 is absent, and
when E is N-halamine of general formula (V) or $-N^+R^{41}R^{42}R^{43}$, L4 is $C_1$-$C_6$ alkyl or

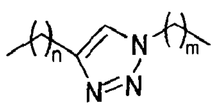

,

and wherein
when $R^{33}$ and $R^{34}$ taken together form =O, $R^{35}$ and $R^{36}$ are each independently H, $C_1$-$C_4$ alkyl, or $C_1$-$C_4$ alkoxy.

11. The compound according to any of paragraphs 1 to 10, wherein in any one of general formulae (II), (III), (IV), (V) or (VI), each halogen when present is -Cl, or -Br, or -I.

12. The compound according to any of paragraphs 1 to 10, wherein in any one of general formulae (II), (III), (IV), (V) or (VI), n and m are each independently 1-4.

13. The compound according to paragraph 10, wherein:

$R^{33}$ and $R^{34}$ are each independently H or $C_1$-$C_4$ alkyl, or $R^{33}$ and $R^{34}$ taken together form =O;
$R^{35}$ and $R^{36}$ are each independently H or $C_1$-$C_4$ alkyl, or $R^{35}$ and $R^{36}$ taken together form =O, and
$R^{37}$ and $R^{38}$ are each independently H or $C_1$-$C_4$ alkyl, or $R^{37}$ and $R^{38}$ taken together form =O.

14. The compound according to paragraph 10, wherein:

$R^{31}$ and $R^{32}$, and $R^{41}$ and $R^{42}$ when present, are each $-CH_3$.

15. The compound according to paragraph 10, wherein:

$R^{31}$ and $R^{32}$, and $R^{41}$ and $R^{42}$ when present, are each $-CH_3$;
$R^{33}$ and $R^{34}$ are each independently H or $-CH_3$, or $R^{33}$ and $R^{34}$ taken together form =O;
$R^{35}$ and $R^{36}$ are each independently H or $-CH_3$, or $R^{35}$ and $R^{36}$ taken together form =O, and
$R^{37}$ and $R^{38}$ are each independently H or $-CH_3$, or $R^{37}$ and $R^{38}$ taken together form =O.

16. The compound according to paragraph 10, wherein:

$R^{33}$ and $R^{34}$ taken together form =O;
$R^{35}$ and $R^{36}$ are each independently H or $C_1$-$C_4$ alkyl, and
$R^{37}$ and $R^{38}$ taken together form =O.

17. The compound according to paragraph 10, wherein:

$R^{33}$ and $R^{34}$ are each independently H or $C_1$-$C_4$ alkyl;
$R^{35}$ and $R^{36}$ taken together form =O, and
$R^{37}$ and $R^{38}$ taken together form =O.

18. The compound according to paragraph 10, wherein:

$R^{33}$ and $R^{34}$ are each independently H or $C_1$-$C_4$ alkyl;
$R^{35}$ and $R^{36}$ taken together form =O, and
$R^{37}$ and $R^{38}$ are each independently H or $C_1$-$C_4$ alkyl.

19. The compound according to paragraph 10, wherein:

$R^{33}$ and $R^{34}$ taken together form =O;
$R^{35}$ and $R^{36}$ are each independently H or $C_1$-$C_4$ alkyl, and
$R^{37}$ and $R^{38}$ arc each independently H or $C_1$-$C_4$ alkyl.

20. The compound according to paragraph 10, wherein:

$R^{33}$ and $R^{34}$ are each independently H or $C_1$-$C_4$ alkyl;
$R^{35}$ and $R^{36}$ are each independently H or $C_1$-$C_4$ alkyl, and
$R^{37}$ and $R^{38}$ taken together form =O.

21. The compound according to any of paragraphs 10 to 20, wherein:

$R^{31}$ and $R^{32}$ are each $-CH_3$.

22. The compound according to any of paragraphs 10 to 21, wherein each halo is -Cl or -Br.

23. A precursor of the biocidal compound according to any of paragraphs 1 to 22, wherein each halo substituent in each N-halamine moiety is replaced with a hydrogen substituent, and wherein halogenation of said substituent results in the biocidally activity compound.

24. The precursor according to paragraph 23, having general formula (VII):

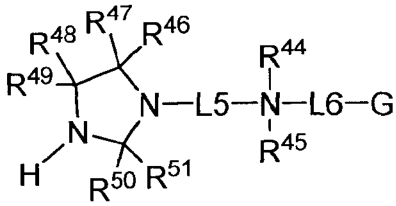

(VII)

wherein:

L5 is $C_1$-$C_6$ alkyl;
$R^{44}$ and $R^{45}$ are each independently $C_1$-$C_6$ alkyl;
L6 is absent, $C_1$-$C_6$ alkyl or

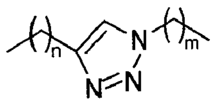

;

G is $R^{52}$, a N-halamine precursor of general formula (V) in which each halo substituent is replaced with a hydrogen substituent, or $-N^+R^{53}R^{54}R^{55}$;
$R^{52}$ is $C_1$-$C_{18}$ alkyl;
$R^{53}$ and $R^{54}$ are each independently $C_1$-$C_6$ alkyl;
$R^{55}$ is $C_1$-$C_{18}$ alkyl or $-(CH_2)_pJ$;
J is a N-halamine precursor of general formula (V) which comprises a hydrogen substituent in place of each halo substituent;
n and m are each 0-6, and
p is 1-6,
$R^{46}$ and $R^{47}$ are each independently H, $C_1$-$C_4$ alkyl, or $C_1$-$C_4$ alkoxy, or $R^{46}$ and $R^{47}$ taken together form =O;
$R^{48}$ and $R^{49}$ are each independently H, $C_1$-$C_4$ alkyl, or $C_1$-$C_4$ alkoxy, or $R^{48}$ and $R^{49}$ taken together form =O;
$R^{50}$ and $R^{51}$ are each independently H, $C_1$-$C_4$ alkyl, or $C_1$-$C_4$ alkoxy, or $R^{50}$ and $R^{51}$ taken together form =O, and

wherein

when G is $R^{52}$, L6 is absent, and
when G is a N-halamine precursor or $-N^+R^{53}R^{54}R^{55}$, L6 is $C_1$-$C_6$ alkyl or

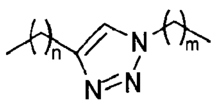

,

and wherein
when $R^{46}$ and $R^{47}$ taken together form =O, $R^{48}$ and $R^{49}$ are each independently H, $C_1$-$C_4$ alkyl, or $C_1$-$C_4$ alkoxy.

25. The compound according to paragraph 1, or the precursor according to paragraph 23, wherein the compound or precursor is selected from compounds having general formula (VIII), (IX) or (X):

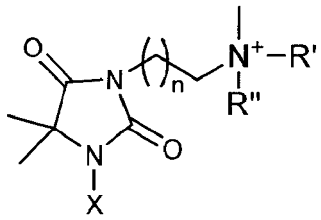

(VIII)

wherein:

X is H, Cl or Br;
n is 1 or 2;
R' is $C_1$-$C_{12}$ alkyl, and
R" is $C_1$-$C_6$ alkyl;

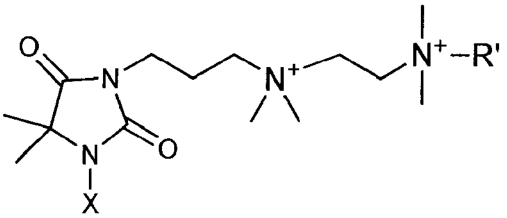

(IX)

wherein:

X is H, Cl or Br, and
R' is $C_1$-$C_{12}$ alkyl;

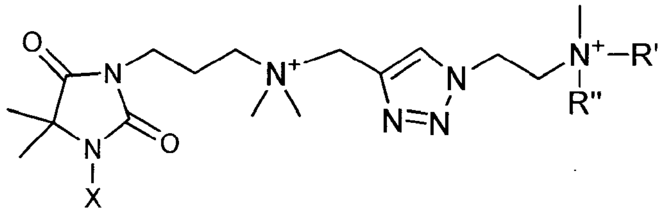

(X)

wherein:

X is H, Cl or Br;
R' is $C_1$-$C_{12}$ alkyl, and
R" is $C_1$-$C_6$ alkyl.

26. The compound according to any of paragraphs 1 to 22 and 25, or the precursor according to any of paragraphs 23 to 25, wherein the compound or precursor is derivatized to allow attachment of the compound or precursor to another compound, surface, substrate or polymer.

27. The compound or precursor according to paragraph 26, wherein the compound or precursor is derivatized to include an azide moiety or an alkynyl group to allow for attachment to another compound, surface, substrate or polymer through "click" chemistry.

28. The compound or precursor according to paragraph 27, wherein one or more of the alkyl groups attached to the quaternary ammonium centre in any of general formulae (II), (III), (IV), (V), (VI) or (VII), is derivatized to include a terminal azide or alkynyl moiety.

29. The compound according to paragraph 25, the precursor according to paragraph 25, or a derivative thereof,

wherein the compound, precursor, or derivative is selected from compounds 1 to 42:

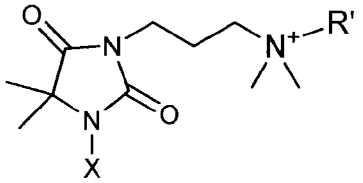

**1** X = H, R' = $CH_3$ **5** X = H, R' = $C_6H_{13}$
**2** X = Cl, R' = $CH_3$ **6** X = Cl, R' = $C_6H_{13}$
**3** X = H, R' = $C_4H_9$ **7** X = H, R' = $C_{12}H_{25}$
**4** X = Cl, R' = $C_4H_9$ **8** X = Cl, R' = $C_{12}H_{25}$

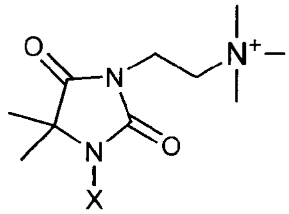

**9** X = H
**10** X = Cl

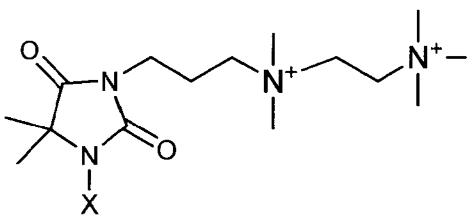

**11** X = H
**12** X = Cl

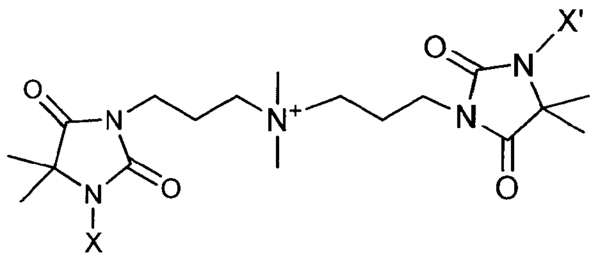

**13** X, X' = H
**14** X, X' = Cl

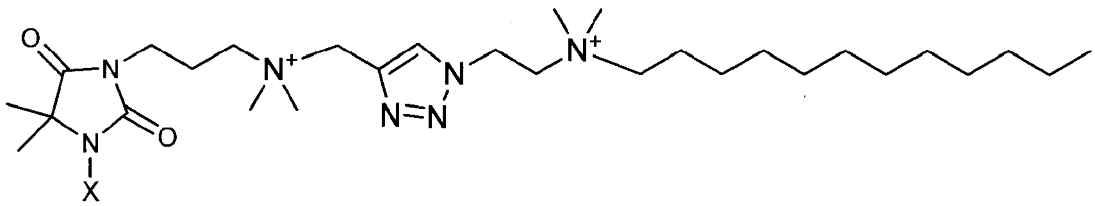

**15** X = H
**16** X = Cl

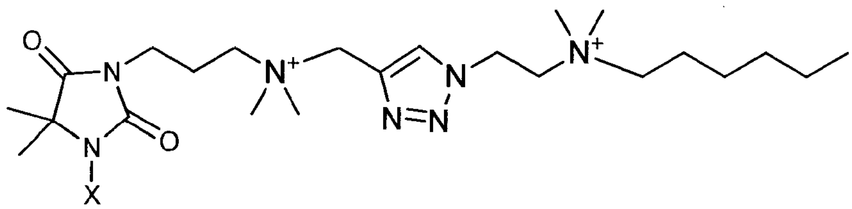

**17** X = H
**18** X = Cl

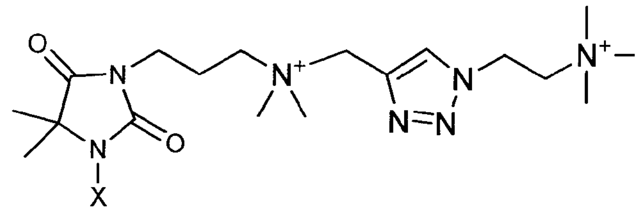

**19** X = H
**20** X = Cl

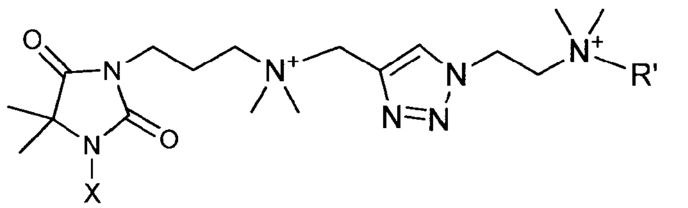

**21** X = H, R' = $C_2H_5$ **25** X = H, R' = $C_8H_{17}$
**22** X = Cl, R' = $C_2H_5$ **26** X = Cl, R' = $C_8H_{17}$
**23** X = H, R' = $C_4H_9$
**24** X = Cl, R' = $C_4H_9$
**37** X = H, R' = $C_6H_{13}$ **38** X = Cl, R' = $C_6H_{13}$

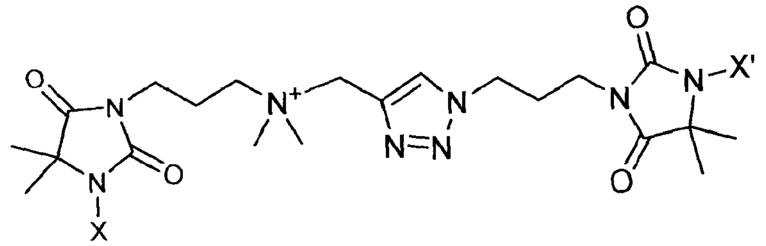

**27** X, X' = H
**28** X, X' = Cl

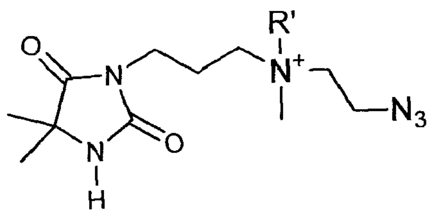

**29** R' = $CH_3$
**30** R' = $C_{12}H_{25}$

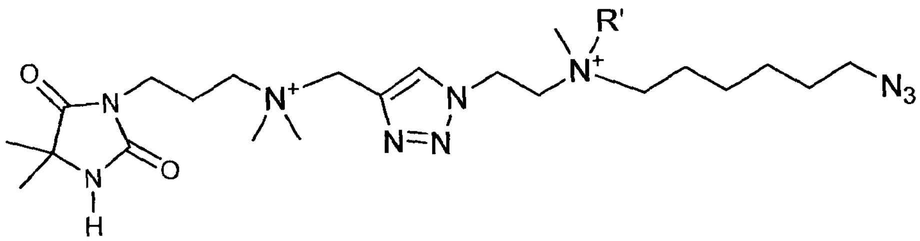

**31** R' = $CH_3$ **36** R' = $C_6H_{13}$
**32** R' = $C_2H_5$ **35** R' = $C_8H_{17}$
**33** R' = $C_4H_9$ **36** R' = $C_{12}H_{25}$

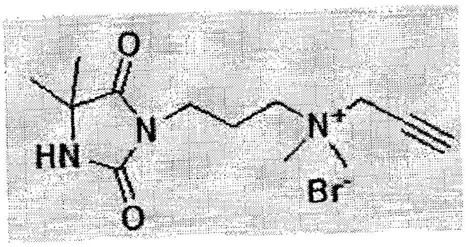

**39**

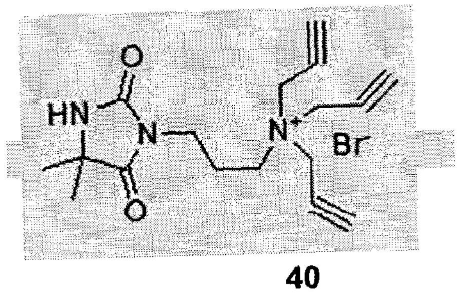

**40**

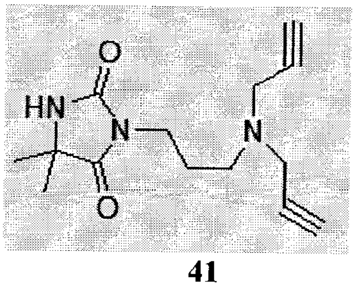

**41**

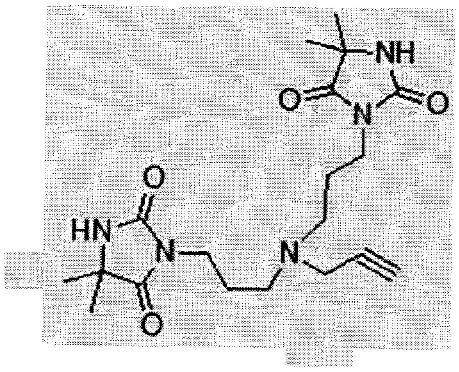

**42**

30. A composition comprising the compound of any of paragraphs 1 to 22, 25 to 28, and 29, the precursor of any of paragraphs 23 to 25, 28, and 29, or the derivative of claim 29.

31. The composition according to paragraph 30, wherein the composition is a solution.

32. The composition according to paragraph 30, wherein the composition comprises a substrate comprising one or more of said compounds, precursors, or derivatives attached to the surface of the substrate.

33. The composition according to paragraph 30, wherein the composition comprises a substrate comprising one or more of said compounds, precursors, or derivatives incorporated within the substrate.

34. The composition according to paragraph 32 or paragraph 33, wherein the substrate is a woven, knit, or nonwoven substrate.

35. The composition according to paragraph 34, wherein the substrate is a semicrystalline thermoplastic polymeric substrate.

36. The composition according toparagraph 35, wherein the substrate is PET.

37. The composition according to paragraph 34, wherein the substrate is cotton.

38. Use of the compound of any of paragraphs 1 to 22, 25 to 28, and 29, the precursor of any of paragraphs 23 to 25, 28, and 29, or the derivative of paragraph 29 as a disinfectant.

## Claims

**1.** A biocidal compound having general formula (I):

N-halamine-L-QUAT (I)

wherein:

the N-halamine is a cyclic or acyclic N-halamine;
L is $C_1$-$C_6$ alkyl, cyclic aromatic or non-aromatic ring,

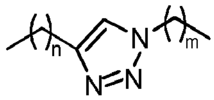

,

ether, or ketone; and
QUAT has general formula (II):

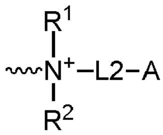

(II)

wherein:

$R^1$ and $R^2$ are each independently $C_1$-$C_6$ alkyl;
L2 is absent $C_1$-$C_6$ alkyl, or

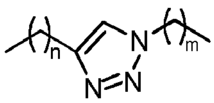

;

A is $R^3$, N-halamine or $-N^+R^4R^5R^6$;
$R^3$ is $C_1$-$C_{18}$ alkyl;
$R^4$ and $R^5$ are each independently $C_1$-$C_6$ alkyl;
$R^6$ is $C_1$-$C_{18}$ alkyl or $-(CH_2)_pB$;
B is N-halamine;
n and m are each independently 1-6, and
p is 1-6,

and wherein

when A is $R^3$, L2 is absent, and
when A is N-halamine or $-N^+R^4R^5R^6$, L2 is $C_1$-$C_6$ alkyl or

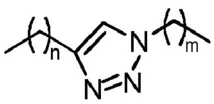

;

and wherein the biocidal compound is derivatized to allow attachment of the compound to another compound, surface, substrate or polymer.

2. A compound as claimed in claim 1, wherein the N-halamine is a cyclic N-halamine.

3. A compound as claimed in claim 1, wherein each N-halamine is independently a cyclic N-halamine having general formula (III) or general formula (IV):

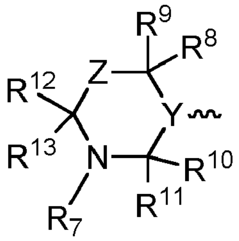
(III)

wherein:

Y is CH or N;
Z is absent, $CH_2$ or $NR^{23}$;
$R^7$ is halo;
$R^8$ and $R^9$ are each independently H, $C_1$-$C_4$ alkyl, or $C_1$-$C_4$ alkoxy, or $R^8$ and $R^9$ taken together form =O;
$R^{10}$ and $R^{11}$ are each independently H, $C_1$-$C_4$ alkyl, or $C_1$-$C_4$ alkoxy, or $R^{10}$ and $R^{11}$ taken together form =O; and
$R^{12}$ and $R^{13}$ are each independently H, $C_1$-$C_4$ alkyl, or $C_1$-$C_4$ alkoxy, or $R^{12}$ and $R^{13}$ taken together form =O, and
$R^{23}$ is H or halo,
wherein when Z is absent and $R^8$ and $R^9$ taken together form =O, $R^{12}$ and $R^{13}$ are each independently H, $C_1$-$C_4$ alkyl, or $C_1$-$C_4$ alkoxy;

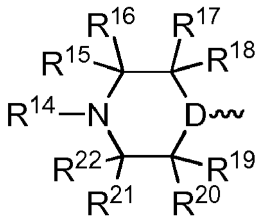
(IV)

wherein:

D is CH or N;
$R^{14}$ is halo;
$R^{15}$ and $R^{16}$ are each independently H, $C_1$-$C_4$ alkyl, or $C_1$-$C_4$ alkoxy, or $R^{15}$ and $R^{16}$ taken together form =O;
$R^{17}$ and $R^{18}$ are each independently H, $C_1$-$C_4$ alkyl, or $C_1$-$C_4$ alkoxy, or $R^{17}$ and $R^{18}$ taken together form =O;
$R^{19}$ and $R^{20}$ are each independently H, $C_1$-$C_4$ alkyl, or $C_1$-$C_4$ alkoxy, or $R^{19}$ and $R^{20}$ taken together form =O, and
$R^{21}$ and $R^{22}$ are each independently H, $C_1$-$C_4$ alkyl, or $C_1$-$C_4$ alkoxy, or $R^{21}$ and $R^{22}$ taken together form =O,
wherein when $R^{15}$ and $R^{16}$ taken together form =O, $R^{17}$ and $R^{18}$ are each independently H, $C_1$-$C_4$ alkyl, or $C_1$-$C_4$ alkoxy, and
wherein when $R^{21}$ and $R^{22}$ taken together form =O, $R^{19}$ and $R^{20}$ are each independently H, $C_1$-$C_4$ alkyl, or $C_1$-$C_4$ alkoxy.

4. A compound as claimed in claim 3, wherein each N-halamine is a cyclic N-halamine having general formula (IV).

5. A compound as claimed in claim 3, wherein each N-halamine is a cyclic N-halamine having general formula (III).

6. A compound as claimed in claim 5, wherein each cyclic N-halamine has general formula (V):

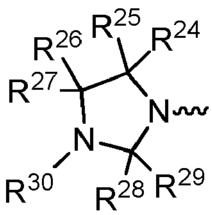

(V)

wherein:

$R^{24}$ and $R^{25}$ are each independently H, $C_1$-$C_4$ alkyl, or $C_1$-$C_4$ alkoxy, or $R^{24}$ and $R^{25}$ taken together form =O; $R^{26}$ and $R^{27}$ are each independently H, $C_1$-$C_4$ alkyl, or $C_1$-$C_4$ alkoxy, or $R^{26}$ and $R^{27}$ taken together form =O; $R^{28}$ and $R^{29}$ are each independently H, $C_1$-$C_4$ alkyl, or $C_1$-$C_4$ alkoxy, or $R^{28}$ and $R^{29}$ taken together form =O, and $R^{30}$ is halo,

and wherein:

when $R^{24}$ and $R^{25}$ taken together form =O, $R^{26}$ and $R^{27}$ are each independently H, C1-C4 alkyl, or C1-C4 alkoxy.

**7.** A precursor of the derivatized biocidal compound as claimed in any of claims 1 to 6, wherein each halo substituent in each N-halamine moiety is replaced with a hydrogen substituent, and wherein halogenation of said substituent results in the biocidally active compound.

**8.** A composition comprising a compound of any of claims 1 to 6, or a precursor of claim 7, and a substrate.

**9.** A method of functionalising a surface or material to provide biocidal activity, comprising immobilizing compounds of any of claims 1 to 8 to a surface by physical coating or by covalent chemical bonding to the surface, or by inclusion into the material as an additive.

**10.** A method as claimed in claim 9, wherein the surface is a soft surface or a polymer-based surface.

a

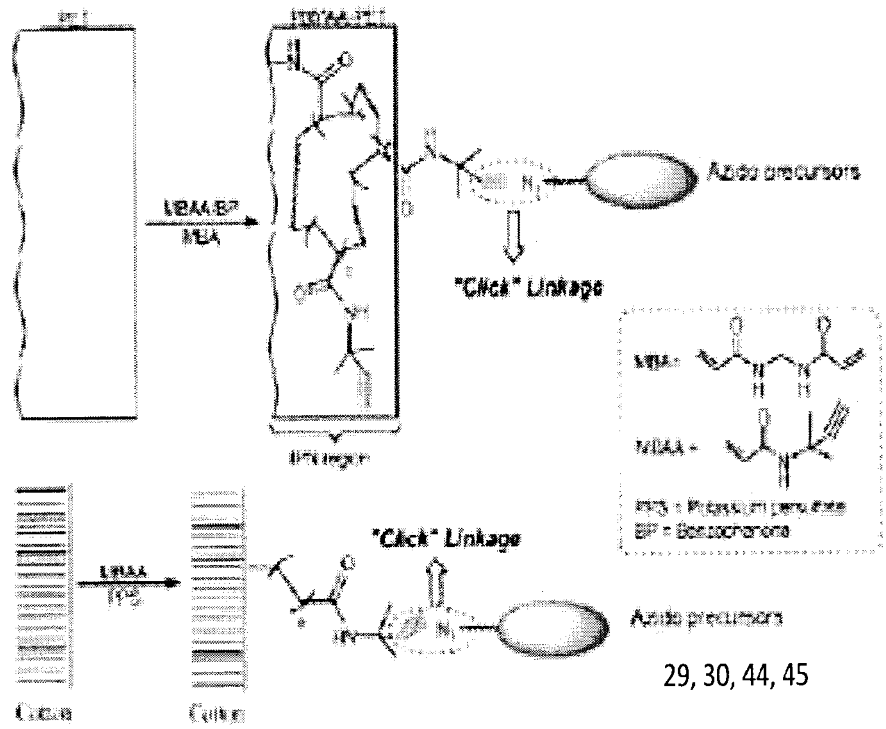


29, 30, 44, 45

b

29, 30, 44, 45

Fig. 1

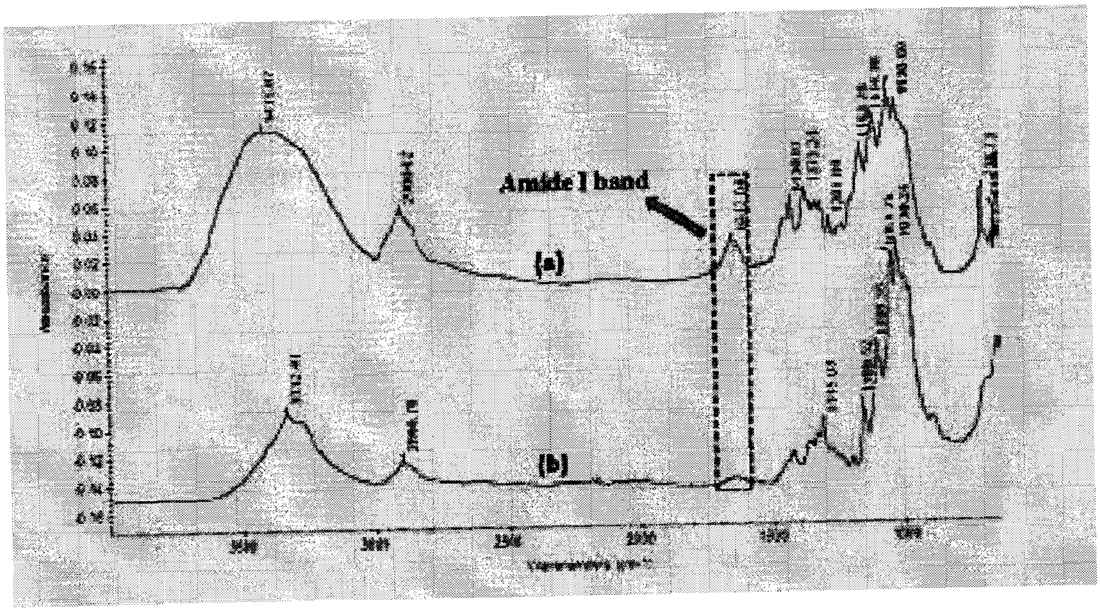


Fig. 2

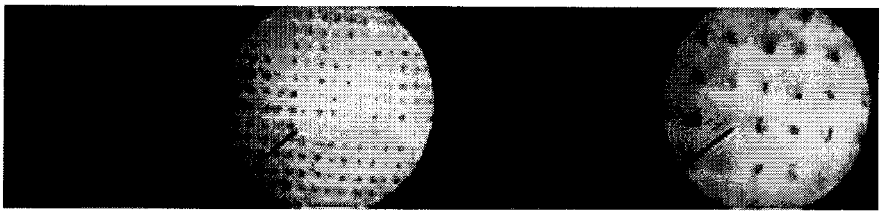

a b c d

Fig. 3

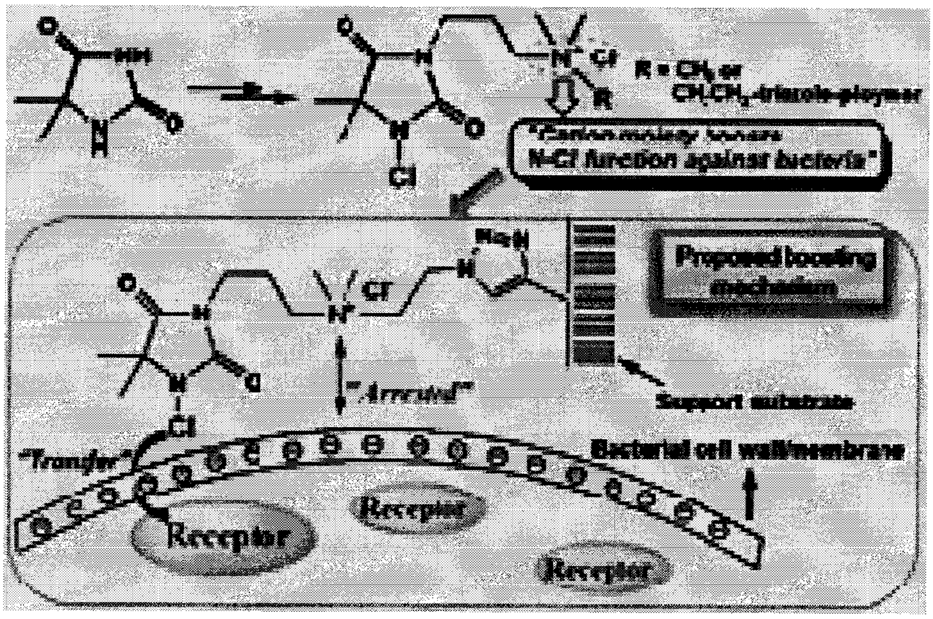
Support substrate
Bacterial cell wall/membrane
"Transfer"
Receptor
Receptor
Receptor

Fig. 4

Europäisches Patentamt
European Patent Office
Office européen des brevets

# EUROPEAN SEARCH REPORT

Application Number
EP 17 19 6453

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br>Y | CHARLES FRANCAVILLA ET AL: "Novel-chloroheterocyclic antimicrobials", BIOORGANIC & MEDICINAL CHEMISTRY LETTERS, PERGAMON, AMSTERDAM, NL, vol. 21, no. 10, 10 March 2011 (2011-03-10), pages 3029-3033, XP028208773, ISSN: 0960-894X, DOI: 10.1016/J.BMCL.2011.03.035 [retrieved on 2011-03-16]<br>* Scheme 5; compound 33 *<br>* Scheme 8; table 2; compounds 61, 62 *<br>----- | 1-8<br><br><br><br><br><br><br><br><br><br>1-10 | INV.<br>C07D403/12<br>A01N43/50<br>A01N43/647<br>A01P1/00<br>C07D233/72<br>C07D233/82<br>A01N33/12<br>A01N35/02<br>A01N59/00 |
| X<br><br><br>Y | WO 2010/054009 A1 (NOVABAY PHARMACEUTICALS INC [US]; JAIN RAKESH K [US]; LOW EDDY [US]; F) 14 May 2010 (2010-05-14)<br>* example 1; compounds 22-27 *<br>* example 5; compounds 22-04 *<br>* example 6; compounds 22-38 *<br>* example 8; compounds 22-40 *<br>* example 11; compounds 22-48 *<br>* example 12; compounds 22-49 *<br>* paragraphs [0240] - [0241] *<br>----- | 1-8<br><br><br>1-10 | TECHNICAL FIELDS SEARCHED (IPC)<br>A01N |
| X<br><br>Y | GB 1 583 905 A (GLYCO CHEMICALS INC) 4 February 1981 (1981-02-04)<br>* claims *<br>----- | 1-8<br><br>1-10 | |
| | -/-- | | |

The present search report has been drawn up for all claims

1

| Place of search | Date of completion of the search | Examiner |
|---|---|---|
| Munich | 26 June 2018 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 19 6453

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EDDY LOW ET AL: "Structure stability/activity relationships of sulfone stabilized,-dichloroamines", BIOORGANIC & MEDICINAL CHEMISTRY LETTERS, PERGAMON, AMSTERDAM, NL, vol. 21, no. 12, 19 April 2011 (2011-04-19), pages 3682-3685, XP028387826, ISSN: 0960-894X, DOI: 10.1016/J.BMCL.2011.04.084 [retrieved on 2011-04-24] | 1,7,8 | |
| Y | * table 2; compounds 4,8,12 * | 1,7-10 | |
| | ----- | | |
| X | WO 2012/054521 A2 (NOVABAY PHARMACEUTICLAS INC [US]; JAIN RAKESH K [US]; SHIAU TIMOTHY [U) 26 April 2012 (2012-04-26) | 1-8 | |
| Y | * page 143, paragraph 263; example 30 * | 1-10 | |
| | ----- | | |
| Y | WO 2008/156636 A1 (MICHIGAN MOLECULAR INST [US]; KAGANOVE STEVEN N [US]) 24 December 2008 (2008-12-24) * examples * * claims * * figures 1,2 * | 1-10 | TECHNICAL FIELDS SEARCHED (IPC) |
| | ----- | | |
| | -/-- | | |

The present search report has been drawn up for all claims

1

| Place of search | Date of completion of the search | Examiner |
|---|---|---|
| Munich | 26 June 2018 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 17 19 6453

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LINGDONG LI ET AL: "Versatile surface biofunctionalization of poly(ethylene terephthalate) by interpenetrating polymerization of a butynyl monomer followed by Click Chemistry", POLYMER, ELSEVIER SCIENCE PUBLISHERS B.V, GB, vol. 53, no. 1, 19 November 2011 (2011-11-19), pages 67-78, XP028349456, ISSN: 0032-3861, DOI: 10.1016/J.POLYMER.2011.11.036 [retrieved on 2011-11-27] * compound 2 * * abstract * * table 2 * ----- | 1-10 | |
| | | | TECHNICAL FIELDS SEARCHED (IPC) |

1

The present search report has been drawn up for all claims

| Place of search | Date of completion of the search | Examiner |
|---|---|---|
| Munich | 26 June 2018 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO.

EP 17 19 6453

This annex lists the patent family members relating to the patent documents cited in the above-mentioned European search report. The members are as contained in the European Patent Office EDP file on
The European Patent Office is in no way liable for these particulars which are merely given for the purpose of information.

26-06-2018

| Patent document cited in search report | Publication date | Patent family member(s) | Publication date |
|---|---|---|---|
| WO 2010054009 A1 | 14-05-2010 | CA 2741660 A1 | 14-05-2010 |
| | | EP 2341777 A1 | 13-07-2011 |
| | | JP 5753785 B2 | 22-07-2015 |
| | | JP 2012511504 A | 24-05-2012 |
| | | US 2010137349 A1 | 03-06-2010 |
| | | WO 2010054009 A1 | 14-05-2010 |
| GB 1583905 A | 04-02-1981 | DE 2744353 A1 | 13-04-1978 |
| | | FR 2366283 A1 | 28-04-1978 |
| | | GB 1583905 A | 04-02-1981 |
| | | JP S5346978 A | 27-04-1978 |
| WO 2012054521 A2 | 26-04-2012 | AU 2011317223 A1 | 23-05-2013 |
| | | BR 112013009279 A2 | 26-07-2016 |
| | | CA 2814378 A1 | 26-04-2012 |
| | | CN 103260402 A | 21-08-2013 |
| | | EP 2629607 A2 | 28-08-2013 |
| | | JP 2013545728 A | 26-12-2013 |
| | | KR 20130123383 A | 12-11-2013 |
| | | SG 189857 A1 | 28-06-2013 |
| | | US 2012129793 A1 | 24-05-2012 |
| | | WO 2012054521 A2 | 26-04-2012 |
| WO 2008156636 A1 | 24-12-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007120173 A


### Non-patent literature cited in the description


- **WORLEY et al.** *App Environ Microbiol,* 1988, vol. 54, 2583-5
- **LI et al.** *Polymer,* 2012, vol. 53, 67-78
- **LI et al.** *Ind. Eng. Chem. Res.,* 2009, vol. 48, 613
- **TOWNSEND et al.** *Med. J. Australia,* 1983, vol. 2, 310
- **QIAN et al.** *J. Appl. Polym. Sci.,* 2003, vol. 89, 2418
- **WILLIAMS et al.** *Appl. Environ. Microbiol.,* 1988, vol. 54, 2583
- **SONOHARA et al.** *Biophys. Chem.,* 1995, vol. 55, 273
- **OHSHIMA et al.** *J. Colloid Interface Sci.,* 1989, vol. 130, 281